# FASCICULE DE BREVET EUROPEEN

(11) **EP 0 711 280 B1**
(45) Date de publication et mention de la délivrance du brevet: **23.09.1998**
(21) Numéro de dépôt: 94923755.6
(22) Date de dépôt: 28.07.1994
(51) Int. Cl.: C07D 209/44, A61K 31/40

(54) **DERIVES DE PERHYDROISOINDOLE COMME ANTAGONISTES DE LA SUBSTANCE P**
PERHYDROISOINDOLDERIVATE ALS SUBSTANZ P ANTAGONISTEN
PERHYDROISOINDOLE DERIVATIVES AS P SUBSTANCE ANTAGONISTS

(30) Priorité: 30.07.1993 FR 9309400; 07.10.1993 FR 9311946
(43) Date de publication de la demande: 15.05.1996
(73) Titulaire: RHONE-POULENC RORER S.A., 92160 Antony (FR)
(72) Inventeur: ACHARD, Daniel, F-94320 Thiais (FR); GRISONI, Serge, F-94600 Choisy-le-Roi (FR); JAMES-SURCOUF, Evelyne, F-77150 Lésigny (FR); MALLERON, Jean-Luc, F-91460 Marcoussis (FR); MORGAT, Anne, F-94250 Gentilly (FR); PEYRONEL, Jean-François, F-91120 Palaiseau (FR); SABUCO, Jean-François, F-94320 Thiais (FR); TABART, Michel, F-75013 Paris (FR)
(86) Numéro de dépôt international: FR9400952
(87) Numéro de publication internationale: WO9504040

(56) Documents cités:
- EP-A- 0 429 366
- EP-A- 0 430 771
- EP-A- 0 514 273
- EP-A- 0 514 274
- WO-A-93/21154
- WO-A-93/21155

## Description

La présente invention concerne de nouveaux dérivés du perhydroisoindole de formule générale : 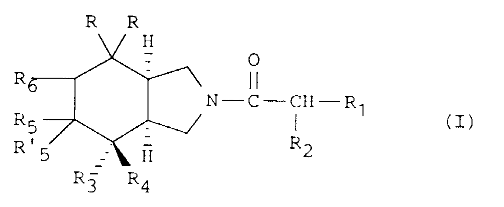 ainsi que leurs sels lorsqu'ils existent, qui antagonisent les effets de la substance P et sont de ce fait particulièrement intéressants dans les domaines thérapeutiques où cette substance est connue pour intervenir.

Dans la demande de brevet européen EP 429 366 ont été décrits des antagonistes de la substance P de structure : 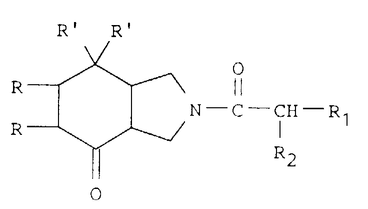 dans laquelle les symboles R sont hydrogène ou forment ensemble une liaison, les symboles R' sont des radicaux phényle éventuellement substitués et les symboles R₁ et R₂ représentent diverses substitutions. Cependant ces dérivés de perhydroisoindolone se sont montrés principalement actifs dans des tests de binding utilisant des homogénats de cerveau de rat et manifestent moins d'activité dans des tests de binding utilisant des cellules humaines lymphoblastiques en culture.

Dans le brevet américain 4 042 707 avaient été décrits des produits dérivés de l'isoindole de formule générale: 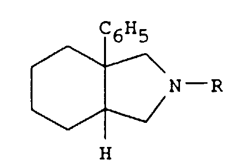 ayant une activité opiacée. Ces produits n'ont pas d'activité vis à vis de la substance P.

Dans la formule générale (I) :
- le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino), alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle, indényle ou hétérocyclyle mono ou polycyclique, saturé ou insaturé contenant 5 à 9 atomes de carbone et un ou plusieurs hétéroatomes choisis parmi l'oxygène, l'azote ou le soufre, et éventuellement substitué par un atome d'halogène ou par un radical alcoyle ou alcoyloxy,
- le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino ou acylamino,
- le symbole R₃ représente un radical phényle éventuellement substitué en position -2 par un radical alcoyle ou alcoyloxy contenant 1 ou 2 atomes de carbone ou par un atome de fluor, ou disubstitué par des radicaux trifluorométhyle et

- les symboles R₅ et R'₅ sont identiques ou différents et représentent l'un un atome d'hydrogène ou un radical hydroxy ou alcoyle et l'autre un atome d'hydrogène ou un radical alcoyle, et
- le symbole R₄ représente un radical hydroxy, ou bien
- le symbole R₄ représente un atome de fluor si les symboles R₅ et R'₅ représentent un atome d'hydrogène ou un radical alcoyle, ou bien
- le symbole R₄ forme avec R₅ une liaison et,

- le symbole R₆ représente un atome d'hydrogène ou un radical alcoyle, hydroxy ou hydroxyalcoyle, et
- les symboles R représentent l'un un atome d'hydrogène, un radical alcoyle, un radical hydroxy ou hydroxyalcoyle et l'autre un atome d'hydrogène, un radical alcoyle, phényle ou hydroxyalcoyle.

Il est entendu que les radicaux alcoyle ou acyle cités ci-dessus contiennent (sauf mention spéciale) 1 à 4 atomes de carbone en chaîne droite ou ramifiée.

Lorsque R₁ contient un atome d'halogène, ce dernier peut être choisi parmi le chlore, le brome, le fluor ou l'iode.

Lorsque R₁ représente un radical hétérocyclyle mono ou polycyclique, saturé ou insaturé, à titre d'exemple il peut être choisi parmi thiényle, furyle, pyridyle, dithiinyle, indolyle, isoindolyle, benzothiényle, thiazolyle, isothiazolyle, oxazolyle, imidazolyle, pyrrolyle, triazolyle, thiadiazolyle, quinolyle, isoquinolyle, ou naphtyridinyle.

Lorsque R₁ représente phényle substitué par une chaîne portant un hétérocycle, ce dernier peut être choisi parmi pyrrolidinyle, morpholino, pipéridinyle, tétrahydropyridinyle, pipérazinyle, thiomorpholino.

De plus, lorsque le symbole R₂ est autre que l'atome d'hydrogène, la chaîne substituée sur l'isoindole présente un centre chiral, il est entendu que les formes stéréoisomères (R) ou (S) et leurs mélanges font partie de la présente invention.

Selon l'invention les dérivés du perhydroisoindole de formule générale (I) peuvent être obtenus par action de l'acide de formule générale : 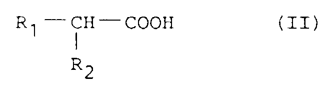 ou d'un dérivé réactif de cet acide, dans lequel R₁ et R₂ sont définis comme précédemment, sur un dérivé de l'isoindole de formule générale : 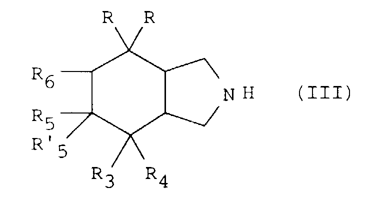 dans laquelle les symboles R, R₃, R₄, R₅, R'₅ et R₆ sont définis comme précédemment, puis éventuellement transformation du produit obtenu pour lequel R₄ est un radical hydroxy et R₅ est un atome d'hydrogène ou un radical alcoyle en un produit pour lequel R₄ est un atome de fluor et R₅ est un atome d'hydrogène ou un radical alcoyle ou en un produit pour lequel R₄ et R₅ forment ensemble une liaison.

Il est entendu que, les radicaux amino, alcoylamino ou carboxy contenus dans R₁ et/ou R₂ sont de préférence préalablement protégés. La protection s'effectue par tout groupement compatible, dont la mise en place et l'élimination n'affectent pas le reste de la molécule. Notamment, on opère selon les méthodes décrites par T.W. Greene, Protective Groups in Organic Synthesis, A. Wiley - Interscience Publication (1981), ou par Mc Omie, Protective Groups in Organic Chemistry, Plenum Press (1973).

A titre d'exemple,
- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués ;
- les groupements acides peuvent être protégés par des radicaux méthyle, éthyle, t.butyle, benzyle, benzyle substitué ou benzhydryle.

De plus, lorsque les produits de formule générale (II) ou (III) portent des radicaux hydroxy, il est préférable de protéger préalablement ce radical. La protection s'effectue selon les méthodes habituelles, par exemple par un radical acétyle, trialcoylsilyle, benzyle, sous forme d'un carbonate par un radical -COORa dans lequel Ra est un radical alcoyle ou benzyle ou sous forme de dérivé carbonylé ou carboxyle.

Lorsque l'on effectue la condensation d'un dérivé réactif de l'acide de formule generale (II), on opère avantageusement au moyen du chlorure d'acide, de l'anhydride, d'un anhydride mixte ou d'un ester réactif dans lequel le reste de l'ester est un radical succinimido, benzotriazolyl-1 éventuellement substitué, nitro-4 phényle, dinitro-2,4 phényle, pentachlorophényle ou phtalimido.

La réaction s'effectue généralement à une température comprise entre -40 et +40°C, dans un solvant organique tel qu'un solvant chloré (dichlorométhane, dichloréthane, chloroforme par exemple), un hydrocarbure (toluène par exemple), un éther (tétrahydrofuranne, dioxanne par exemple), un ester (acétate d'éthyle par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment triéthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl)-1 éthyl-3 carbodiimide), le NN'-carbonyldiimidazole ou l'éthoxy-2 éthoxycarbonyl-1 dihydro-1,2 quinoléine ou bien en milieu hydroorganique, en présence d'un agent alcalin de condensation comme le bicarbonate de sodium.

Dans l'alternative où l'on a obtenu un dérivé de perhydroisoindole de formule générale (I) pour lequel R₄ est un radical hydroxy et R₅ est un atome d'hydrogène ou un radical alcoyle et où l'on veut obtenir un dérivé de perhydroisoindole pour lequel R₄ est un atome de fluor et R₅ est un atome d'hydrogène ou un radical alcoyle, on opère par fluoration du dérivé précédemment obtenu.

La réaction s'effectue avantageusement au moyen d'un agent de fluoration comme un fluorure de soufre [trifluorure de morpholino soufre, tétrafluorure de soufre (J. Org. Chem., 40, 3808 (1975)), trifluorure de diéthylamino soufre (Tetrahedron, 44, 2875 (1988)), trifluorure de phényl soufre (J. Am. Chem. Soc., 84, 3058 (1962)], comme le tétrafluorure de sélenium (J. Am. Chem. Soc., 96,925 (1974) ou comme le tétrafluorophénylphosphorane (Tet. Let., 907 (1973), en opérant dans un solvant organique tel qu'un solvant chloré (dichlorométhane, dichloréthane par exemple) à une température comprise entre -30 et 30°C. Il est entendu que dans l'alternative où des radicaux hydroxy sont présents sur la molécule, ces derniers sont préalablement protégés.

Dans l'alternative où l'on a obtenu un dérivé de perhydroisoindole de formule générale (I) pour lequel R₄ est un radical hydroxy et R₅ est un atome d'hydrogène et où l'on veut obtenir un dérivé de perhydroisoindole pour lequel R₄ et R₅ forment ensemble une liaison, on opère par toute méthode connue de deshydratation des alcools qui n'altère pas le reste de la molécule. Notamment on effectue la deshydratation en milieu acide par exemple par action d'un acide sulfonique (acide p.toluènesulfonique...), de l'acide formique, de l'acide sulfurique, de l'acide phosphorique, du pentaoxyde de phosphore ou de l'oxyde d'aluminium ou par action d'un mélange acide chlorhydrique-acide acétique ou acide bromhydrique-acide acétique, à une température comprise entre 25°C et la température de reflux du mélange réactionnel.

Selon l'invention les dérivés de l'isoindole de formule générale (I) peuvent également être obtenus par action d'un composé organométallique de formule générale :

R₃-M (IV)

dans laquelle R₃ est défini comme précédemment, et M représente le lithium, un radical MgX ou CeX₂ pour lequel X est un atome d'halogène, sur un dérivé de perhydroisoindolone de formule générale : 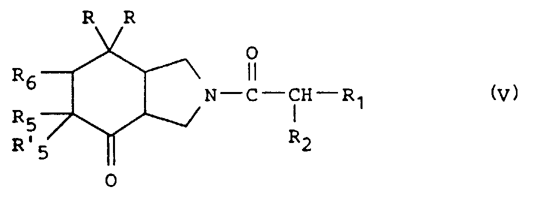 dans laquelle les symboles R, R₁, R₂, R₅, R'₅ et R₆ sont définis comme précédemment, et dont le cas échéant les radicaux hydroxy sont de préférence préalablement protégés, puis transformation le cas échéant de l'alcool obtenu de formule générale (I) en un dérivé de perhydroisoindole pour lequel R₄ est un atome de fluor et R₅ est un atome d'hydrogène ou un radical alcoyle ou en un dérivé de perhydroisoindole pour lequel R₄ et R₅ forment ensemble une liaison ou élimination le cas échéant du radical protecteur de R₅.

La réaction s'effectue en milieu anhydre, dans les conditions habituelles de réaction des composés organométalliques sur une cétone, qui n'affectent pas le reste de la molécule. Notamment on opère dans un éther (par exemple le tétrahydrofuranne ou l'éther éthylique) éventuellement en présence de chlorure de cérium anhydre à une température comprise entre -78 et 30°C.

Les opérations subséquentes de transformation en un dérivé de formule générale (I) pour lequel R₄ est un atome de fluor et R₅ est hydrogène ou pour lequel R₄ et R₅ forment ensemble une liaison, s'effectuent dans les conditions décrites précédemment.

Selon l'invention, les dérivés de perhydroisoindole de formule générale (I) pour lesquels l'un des radicaux R représente un radical hydroxy, R₄ représente un radical hydroxy et l'un au moins de R₅ et R'₅ est un atome d'hydrogène, peuvent être préparés à partir d'un dérivé de perhydroisoindolone de formule générale : 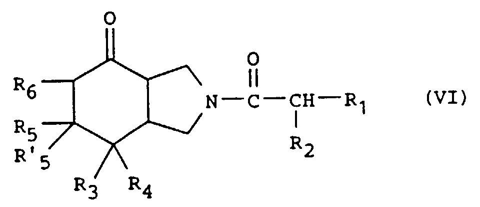 dans laquelle R₁, R₂, R₃, R₄, R₅, R'₅ et R₆ sont définis comme précédemment, par action d'un composé organométallique de formule générale :

R-M (VII)

dans laquelle R est un radical alcoyle, phényle ou hydroxyalcoyle dont la fonction hydroxy est préalablement protégée, et M est défini comme précédemment, ou par réduction dans l'alternative où l'on veut obtenir un dérivé de formule générale (I) pour lequel l'autre radical R est un atome d'hydrogène, puis le cas échéant élimination des radicaux protecteurs.

La réaction du composé organo-métallique sur le dérivé de perhydroisoindolone de formule générale (VI) s'effectue dans des conditions analogues à celles décrites pour l'action d'un composé de formule générale (IV) sur un dérivé de perhydroisoindolone de formule générale (V).

La réaction de réduction lorsque l'on veut obtenir un dérivé de perhydroisoindole pour lequel l'un des R est hydroxy et l'autre est hydrogène, s'effectue par toute méthode connue pour la réduction des cétones, qui n'altère pas le reste de la molécule; par exemple on opère au moyen de borohydrure de sodium en milieu alcoolique (méthanol par exemple) ou au moyen d'hydrure d'aluminium et de lithium dans un éther, à une température comprise entre 20 et 50°C.

Selon l'invention, les dérivés de perhydroisoindole de formule générale (I) pour lesquels les radicaux R₅ (ou R'₅) et R₆ sont simultanément des radicaux hydroxy et les symboles R sont différents de l'atome d'hydrogène, peuvent être préparés par action de tétroxyde d'osmium sur un dérivé de perhydroisoindole de formule générale : 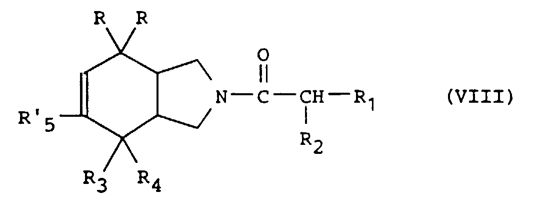 dans laquelle R₁, R₂, R₃, R₄, R et R'₅ sont définis comme précédemment.

La réaction s'effectue généralement dans la pyridine à une température comprise entre 0°C et la température de reflux du mélange réactionnel.

Les acides de formule générale (II) peuvent être préparés selon les méthodes décrites ci-après dans les exemples, selon les méthodes décrites dans la demande de brevet EP 429 366 ou par analogie avec ces méthodes.

Le dérivé de perhydroisoindole de formule générale (III) pour lequel R₄ et R₅ forment ensemble une liaison peuvent être obtenus par deshydratation du dérivé correspondant de perhydroisoindole pour lequel R₄ est un radical hydroxy et R₅ est un atome d'hydrogène.

La réaction s'effectue dans les conditions décrites précédemment pour la préparation des dérivés de formule générale (I) dans laquelle R₄ et R₅ forment ensemble une liaison à partir du dérivé correspondant de perhydroisoindole pour lequel R₄ est un radical hydroxy et R₅ est un atome d'hydrogène.

Le dérivé de l'isoindole de formule générale (III) pour lequel R₄ est un atome de fluor et R₅ et R'₅ sont des atomes d'hydrogène ou des radicaux alcoyle peuvent être préparés par fluoration d'un dérivé de l'isoindole de formule générale : 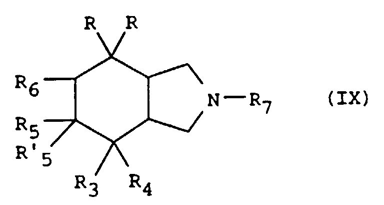 dans laquelle R, R₃ et R₆ sont définis comme précédemment, R₇ est un radical protecteur, R₄ est un radical hydroxy et R₅ et R'₅ sont des atomes d'hydrogène ou des radicaux alcoyle, puis élimination du radical protecteur R₇.

Le radical protecteur R₇ peut être tout groupement protecteur d'amino qui soit compatible avec la réaction et dont la mise en place et l'élimination n'altère pas le reste de la molécule. A titre d'exemple peuvent être cités les groupements alcoyloxycarbonyle, benzyloxycarbonyle, benzyle éventuellement substitués, formyle, chloracétyle, trichloracétyle, trifluoracétyle, vinyloxycarbonyle, phénoxycarbonyle, chloro-1 éthoxycarbonyle ou chlorocarbonyle.

La fluoration s'effectue dans les conditions décrites précédemment pour la fluoration d'un dérivé de formule générale (I) dans laquelle R₄ est hydroxy, à une température comprise entre -30 et +30°C. Il est entendu que les fonctions hydroxy présentes sur la molécule sont le cas échéant préalablement protégées.

L'élimination subséquente des radicaux protecteurs s'effectue selon les méthodes habituelles. Notamment selon les méthodes décrites par T.W. Greene, par A. Wiley ou par Mc Omie dans les références citées précédemment.

Le dérivé de perhydroisoindole de formule générale (III) ou (IX) pour lequel R₄ est un radical hydroxy et R₅ est un atome d'hydrogène ou un radical alcoyle ou hydroxy, peut être obtenu par action d'un composé organométallique de formule générale (IV) sur le dérivé correspondant de perhydroisoindolone de formule générale : 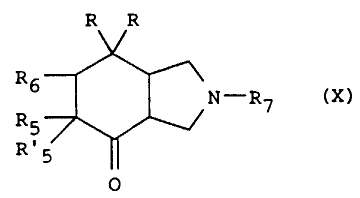 dans laquelle R, R₅, R'₅, R₆ et R₇ sont définis comme précédemment, étant entendu que les fonctions hydroxy sont le cas échéant éventuellement protégées, puis libération des radicaux protecteurs de R₅, R'₅, R₆ ou R et éventuellement élimination du radical protecteur R₇ lorsque l'on veut obtenir le dérivé de formule générale (III).

La réaction s'effectue dans des conditions analogues à celles décrites pour l'obtention du perhydroisoindole de formule générale (I) à partir de la perhydroisoindolone correspondante de formule générale (V). Il est entendu que selon la nature du radical protecteur de R₅, R'₅, R₆ ou R, ce dernier peut être éliminé simultanément à la réaction.

Il est entendu que, lorsque l'on veut obtenir un dérivé pour lequel R est un radical hydroxyméthyle, ce radical peut être protégé à l'état de radical allyloxycarbonyle sur le dérivé de perhydroisoindolone de formule générale (X). La réaction s'effectue en passant intermédiairement par un dérivé de perhydropyrano[3,4-c]pyrrolone-6 qui est transformé par réduction et le cas échéant par élimination du radical protecteur d'amino pour obtenir un dérivé de formule générale (III).

Le dérivé de la perhydroisoindolone de formule générale (X) pour laquelle R₅, R'₅ et R₆ sont des atomes d'hydrogène, des radicaux alcoyle ou des radicaux hydroxy (ou hydroxyalcoyle) préalablement protégés peuvent être préparés par analogie avec la méthode décrite dans la demande de brevet européen EP 429 366, ou comme décrit ci-après dans les exemples.

La préparation du dérivé de la perhydroisoindolone de formule générale (V) s'effectue par analogie avec la méthode décrite dans la demande de brevet européen EP 429 366 par action d'un acide de formule générale (II) ou d'un de ses dérivés réactifs sur un dérivé de perhydroisoindolone de formule générale : 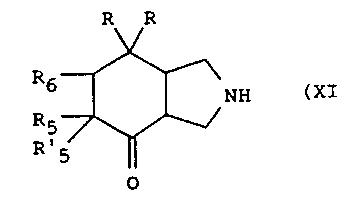 dans laquelle R, R₅, R'₅ et R₆ sont définis comme dans la formule générale (V) dans les conditions citées dans la demande européenne précitée ou dans les conditions citées pour l'action des acides de formule générale (II) sur un dérivé de perhydroisoindole de formule générale (III).

Les dérivés de formule générale (VI) peuvent être obtenus par action d'un acide de formule générale (II) sur une perhydroisoindolone de formule générale : 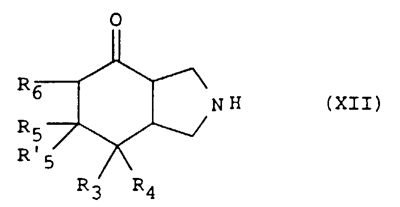 dans laquelle R₃, R₄, R₅, R'₅ et R₆ sont définis comme dans la formule générale (VI), par analogie avec la méthode décrite pour l'obtention d'un dérivé de perhydroisoindole de formule générale (I) à partir de produits de formules générales (II) et (III).

Le dérivé de perhydroisoindolone de formule générale (XII) peut être préparé par action d'un dérivé organométallique de formule générale (IV) sur un dérivé de formule générale : 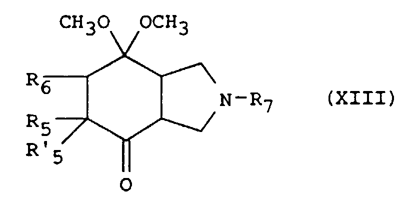 dans laquelle R₅, R'₅ et R₆ sont définis comme dans la formule générale (XII) et R₇ est un radical protecteur tel que défini précédemment, suivie de l'élimination de la protection de la fonction cétone en position -7 et de l'élimination du radical protecteur R₇.

La réaction s'effectue dans les conditions décrites précédemment pour la préparation du produit de formule générale (III) à partir d'un dérivé de perhydroisoindolone de formule générale (X). L'élimination des radicaux protecteurs s'effectue selon les méthodes habituelles citées précédemment et/ou décrites ci-après dans les exemples.

Le dérivé de perhydroisoindolone de formule générale (XIII) peut être préparé par réduction du dérivé insaturé correspondant de formule générale : 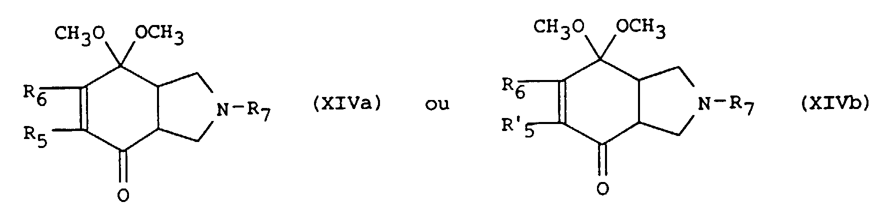 dans laquelle R₅, R'₅, R₆ et R₇ sont définis comme dans la formule générale (XIII).

La réduction s'effectue par toute méthode connue qui n'altère pas le reste de la molécule. Notamment on opère par hydrogénation catalytique en présence de nickel de Raney.

Le dérivé de perhydroisoindolone de formule générale (XIVa) ou (XIVb) peut être obtenu à partir de la cyclohexadiènone correspondante par analogie avec la méthode décrite précédemment dans la demande européenne EP 430 771.

La cyclohexadiènone de départ peut être obtenue selon la méthode décrite dans J. Org. Chem., 52, 2763 (1987).

Le dérivé de perhydroisoindole de formule générale (VIII) peut être obtenu par action d'un dérivé organométallique de formule générale (IV) sur un dérivé de perhydroisoindolone de formule générale : 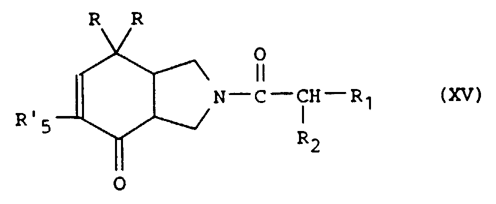 dans laquelle R₁, R₂, R'₅ et R sont définis comme précédemment pour la formule générale (VIII).

Le dérivé de perhydroisoindolone de formule générale (XV) peut être préparé par analogie avec la méthode décrite dans la demande européenne EP 430 771, ou comme décrit ci-après dans les exemples.

Les dérivés de perhydroisoindole de formules générales (III), (IX), (X) et (XI) sont des produits nouveaux qui peuvent être définis par la structure : 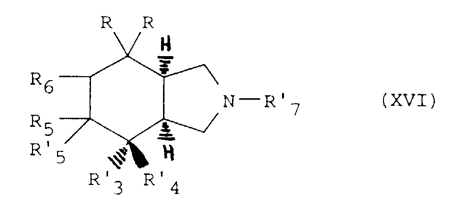 dans laquelle R, R'₅, R₅ et R₆ sont définis comme précédemment, R'₃ et R'₄ sont soit définis comme R₃ et R₄, soit forment ensemble un radical oxo et R'₇ est soit un atome d'hydrogène, soit représente un radical protecteur tel que défini précédemment pour R₇.

Il est bien entendu que le dérivé de perhydroisoindole de formule générale (XVI) présente différentes formes stéréoisomères et que sa stéréochimie est semblable à celle décrite précédemment pour les dérivés de formule générale (I).

Il est entendu que les dérivés de perhydroisoindole de formule générale (III), (V) , (IX), (X), (XI) ou (XVI) présentent plusieurs formes stéréoisomères. Lorsque l'on veut obtenir l'énantiomère de formule générale (I), la séparation est mise en oeuvre par exemple au niveau du dérivé de formule générale (I) ou au niveau d'un intermédiaire de formule générale (V), (X), (XI) ou (XVI) portant un radical oxo en position -4. Elle peut aussi être mise en oeuvre au niveau du dérivé de formule générale (III), (IX) ou (XV). La séparation s'effectue selon toute méthode connue et compatible avec la molécule.

A titre d'exemple, la séparation peut être effectuée par préparation d'un sel optiquement actif, par action de l'acide L(+) ou D(-) mandélique, ou de l'acide dibenzoyltartrique ou ditoluoyltartrique, puis séparation des isomères par cristallisation. L'isomère recherché est libéré de son sel en milieu basique.

Une autre alternative peut être aussi, le cas échéant, d'opérer directement à partir d'un énantiomère de la cyclohexénone de départ.

Les nouveaux dérivés de l'isoindole de formule générale (I) peuvent être purifiés le cas échéant par des méthodes physiques telles que la cristallisation ou la chromatographie.

Le cas échéant, les nouveaux dérivés de formule générale (I) pour lesquels les symboles R₁ et/ou R₂ contiennent des substituants amino ou alcoylamino, ou les intermédiaires de formule générales (III), (XI) ou (XVI) pour lesquels R'₇ est un atome d'hydrogène peuvent être transformés en sels d'addition avec les acides. Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les nouveaux dérivés de l'isoindole de formule générale (I) peuvent aussi, le cas échéant, lorsque R₂ représente un radical carboxy, être transformés en sels métalliques ou en sels d'addition avec une base azotée, selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhyldrylamine, quinine, choline, arginine, lysine, leucine, dibenzylamine).

Les nouveaux dérivés de l'isoindole selon la présente invention qui antagonisent les effets de la substance P peuvent trouver une application dans les domaines de l'analgésie, de l'inflammation, de l'asthme, des allergies, sur le système nerveux central, sur le système cardiovasculaire, comme antispasmodique, comme antiémétique, ou sur le système immunitaire, ainsi que dans le domaine de la stimulation des secrétions lachrymales.

En effet, les produits selon l'invention manifestent une affinité pour les récepteurs à substance P à des doses comprises entre 5 et 1000 nM selon les techniques adaptées de D.G. Payan et coll., J. of immunology, 133(6), 3260-5 (1984) : Stereospecific receptors for substance P on cultured human IM-9 lymphoblasts et de Mc Pherson et coll., J. Pharmacol. Meth., 14, 213 (1985) : Analysis of radioligand binding experiments.

Il a de plus été démontré qu'il s'agit d'un effet antagoniste de la substance P, au moyen de différents produits. Dans la technique décrite par S. Rosell et coll., Substance P, Ed. by US Von Euler and B. Pernow, Raven Press, New-York (1977), pages 83 à 88, les produits étudiés manifestent un antagonisme des contractions de l'iléon de cobaye induites par la substance P ou des contractions de l'iléon de cobaye induites par le septide, à des concentrations de 1 à 1000 nM.

La substance P est connue pour être impliquée dans un certain nombre de domaines pathologiques :
- Agonists and antagonists of substance P, A.S. Dutta Drugs of the futur, 12 (8), 782 (1987) ;
- Substance P and pain : an updating, J.L. Henry, TINS, 3(4), 97 (1980) ;
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987) ;
- Substance P in Human Essential Hypertension, J. Cardiocascular Pharmacology, 10 (suppl. 12), 5172 (1987).

L'étude de certains dérivés de l'isoindole de formule générale (I) dans la technique de A. Saria et coll., Arch. Pharmacol.,324, 212-218 (1983) adaptée au cobaye a permis de mettre en évidence un effet inhibiteur de l'augmentation de la perméabilité capillaire entraîné par le septide (agoniste de la substance P), ce qui témoigne d'une activité anti-inflammatoire :

| Produit étudié | DE₅₀ |
|---|---|
| Exemple 1 | 0,07 mg/kg i.v. |
| | 3 à 10 mg/kg p.o. |

L'injection de substance P provoque chez l'animal un bronchospasme. La bronchoconstriction induite in vivo chez le cobaye par l'injection de substance P ou d'un agoniste sélectif de la substance P : septide, est étudiée selon la technique de H. Konzett et R. Rosseler, Archiv. Exp. Path. Pharmak., 195, 71-74 (1940). Cette bronchoconstriction est inhibée par l'injection d'un produit selon l'invention, ce qui témoigne d'une activité anti-asthmatique.

Par ailleurs, les produits selon l'invention sont étudiés dans le test de douleur au formol chez le cobaye. On détermine ainsi la DE₅₀ du produit.

Enfin, les dérivés de l'isoindole selon la présente invention ne présentent pas de toxicité, ils se sont montrés atoxiques chez la souris à la dose de 40 mg/kg par voie sous-cutanée.

La présente invention concerne également l'association synergisante constituée d'au moins un antagoniste des récepteurs NK1 de formule générale (I) et d'au moins un antagoniste des récepteurs NK2.

Les effets de la neurokinine A sont médiés principalement par les récepteurs NK2. La neurokinine A est impliquée dans de nombreuses pathologies telles que la transmission de la douleur, l'arthrite, l'asthme, les phénomènes inflammatoires, les psychoses, les désordres tensionnels, les désordres vésicaux, les cystites ...

Des antagonistes des récepteurs NK2 (antagonistes des effets de la neurokinine A) sont connus et décrits notamment dans les demandes de brevet EP 428 434, EP 474 561, EP 512 901, EP 515 240, FR 2 678 267, WO 92/19254 ou WO 93/14084.

A titre non limitatif, des antagonistes des récepteurs NK2 peuvent être notamment des dérivés de la classe des arylalkylamines, la classe des polypeptides α-substitués ou de la classe des dérivés de la pipéridine ...

A titre d'exemple des antagonistes des récepteurs NK2 de la classe des arylalkylamines peuvent être des produits de formule générale : 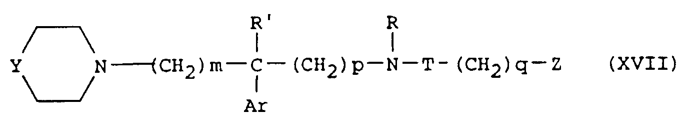 dans laquelle :
1) Y est un groupe >N-CXX'-Ar', >CH-CXX'-Ar' ou >C=CX-Ar' pour lequel X est H et X' est H ou OH ou X et X' forment ensemble un radical oxo, dialcoylaminoalcoyloxyimino pour lequel les parties alcoyle contiennent 1 à 4 atomes de carbone et la partie alcoyloxy contient 2 ou 3 atomes de carbone,
   Ar et Ar' sont indépendemment thiényle, phényle éventuellement mono ou poly substitué (par halogène, alcoyle ou alcoyloxy (1 à 3 atomes de carbone), trifluorométhyle, hydroxy ou méthylènedioxy) ou imidazolyle, ou bien Ar peut être benzothiényle ou naphtyle éventuellement substitués par halogène, biphényle, ou indolyle pouvant porter un groupe benzyle sur l'atome d'azote,
   R' est un atome d'hydrogène, un radical alcoyle (1 à 4 atomes de carbone), un radical alcoyle (2 ou 3 atomes de carbone) substitué (par pipéridino, benzyl-4 pipéridino ou dialcoylamino dont les parties alcoyle contiennent 1 à 4 atomes de carbone,
   R et T sont définis comme pour la formule générale (VI) et
   Z est H, alcoyle droit ou ramifié (1 à 6 atomes de carbone), phénylalcoyle dont la partie alcoyle contient 1 à 3 atomes de carbone, éventuellement mono ou poly substitué sur le phényle par halogène, OH, alcoyle ou alcoyloxy (1 à 4 atomes de carbone), pyridylalcoyle ou naphtylalcoyle ou pyridylthioalcoyle ou (méthyl-1)imidazolyl-2 thioalcoyle dont la partie alcoyle contient 1 à 3 atomes de carbone, styryle, oxo-1 phénylindan-3 yle-2, ou un groupe aromatique ou hétéroaromatique non substitué, mono ou polysubstitué, et
   m est un nombre entier de 1 à 3, p égale 1 et q égale 0, ou bien
2) Y est un groupe >N-Ar' pour lequel Ar' est un radical phényle pouvant être substitué 1 ou plusieurs fois (par halogène, OH, alcoyloxy ou alcoyle (1 à 4 atomes de carbone) ou trifluorométhyle), pyrimidinyle ou pyridyle,
   un groupe >N-cycloalcoyle (3 à 7 atomes de carbone), ou bien
   un groupe >CX-(CH₂)ₓ-Ar' pour lequel Ar' est défini comme ci-dessus à l'exception de représenter pyrimidinyle, ou représente thiényle, X est OH, alcoyloxy (1 à 4 atomes de carbone), hydroxyalcoyle ou acyloxy dont la partie alcoyle contient 1 à 3 atomes de carbone, phénacyloxy, carboxy, carbalcoyloxy (1 à 4 atomes de carbone), cyano, aminoalcoylène (1 à 3 atomes de carbone), amino, alcoylamino ou dialcoylamino dont les parties alcoyle contiennent 1 à 4 atomes de carbone, acylamino (2 à 7 atomes de carbone), acylaminoalcoyle dont les parties alcoyle contiennent 1 à 3 atomes de carbone, acyle, -SH, alcoylthio dont la partie alcoyle contient 1 à 4 atomes de carbone et x est 0 ou 1, ou bien
   un groupe =C-(CH₂)ₓ-Ar' dans lequel Ar' est défini comme ci-dessus, T est défini comme en 1), et Z est défini comme en 1) à l'exception de représenter (méthyl-1)imidazolyl-2 thioalcoyle ou oxo-1 phénylindan-3 yle-2, ou représente phénylalcoyle substitué par trifluorométhyle ou naphtylalcoyle dont la partie alcoyle contient 1 à 3 atomes de carbone et éventuellement substitué sur le cycle naphtyle par un atome d'halogène ou par un radical trifluorométhyle, OH, alcoyle ou alcoyloxy (1 à 4 atomes de carbone),
   Ar est thiényle, phényle éventuellement mono ou poly substitué (par halogène, alcoyle ou alcoyloxy (1 à 4 atomes de carbone) , trifluorométhyle) ou benzothiényle, naphtyle ou indolyle pouvant porter un groupe alcoyle (1 à 3 atomes de carbone) sur l'atome d'azote,
   R' est un atome d'hydrogène,
   R est un atome d'hydrogène ou un radical alcoyle (1 à 6 atomes de carbone), et
   m est un nombre entier égal à 2 ou 3, p égale 1 et q égale 0, ou bien
3) Y est un groupe >N-Ar' ou >N-CH₂-Ar' pour lequel Ar' est défini comme ci-dessus en 2), ou bien
   un groupe >CX-(CH₂)ₓ-Ar' tel que défini ci-dessus, X étant OH, alcoyloxy, acyloxy ou carbalcoyloxy (1 à 4 atomes de carbone), carboxy, cyano, amino éventuellement mono ou di substitué (par alcoyle, hydroxyalcoyle ou acyle (1 à 4 atomes de carbone)), pyrrolidino, pipéridino, ou morpholino, -SH ou alcoylthio dont la partie alcoyle contient 1 à 4 atomes de carbone et x est 0 ou 1,ou bien
   un groupe =C-(CH₂)ₓ-Ar' dans lequel Ar' est défini comme ci-dessus, Ar est défini comme ci-dessus en 2) à l'exception de représenter un radical indolyle substitué,
   R et R' forment ensemble une chaîne 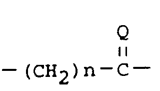 dans laquelle Q est un atome d'oxygène ou 2 atomes d'hydrogène,
   T est -CO- ou -CH₂- et
   Z est phényle ou naphtyle éventuellement mono ou polysubstitués par halogène, trifluorométhyle, OH ou alcoyle (1 à 4 carbones), ou par alcoyloxy (1 à 4 carbones) lorsque Z est phényle, pyridyle, thiényle, indolyle, quinoléyle, benzothiényle ou imidazolyle, ou un groupe aromatique ou hétéroaromatique non substitué, mono ou polysubstitué, ou lorsque T est CO, -(CH₂)q-Z peut être benzyle dont le radical méthyle est substitué par OH, alcoyle ou alcoyloxy (1 à 4 carbones), ou substitué sur le cycle par halogène, trifluorométhyle, OH, alcoyle ou alcoyloxy (1 à 4 carbones), et
   m est un nombre entier égal à 2 ou 3, p égale 1 ou 2, n égale 0 à 3 et q égale 0 à 3, étant entendu que si p=2 : n=1 et Q représente 2H; ou bien
4) Y est un groupe Ar'-X- pour lequel Ar' représente thiényle, phényle éventuellement mono ou poly substitué (par halogène, alcoyle ou alcoyloxy (1 à 3 carbones), trifluorométhyle, hydroxy ou méthylènedioxy) pyridyle ou imidazolyle éventuellement substitué par un radical alcoyle, et X est un atome d'oxygène ou de soufre ou un radical sulfonyle, sulfinyle, -NH-, >N-CO-Alk, >N-Alk, >N-Alk-NX₁X₂ pour lesquels Alk est alcoyle ou alcoylène (1 à 3 carbones) et X₁ et X₂ sont H, alcoyle (1 à 3 carbones) ou forment avec l'atome d'azote un cycle pipéridine, pyrrolidine ou morpholine,
   R' est un atome d'hydrogène, un radical alcoyle (1 à 4 carbones) ou un radical aminoalcoyle dont la partie alcoyle en chaîne droite contient 2 ou 3 atomes de carbone et le groupe amino peut être dialcoylamino dont les parties alcoyle contiennent 1 à 4 atomes de carbone, pipéridino, ou benzyl-4 pipéridino,
   Ar, R et T sont définis comme en 1),
   Z est défini comme précédemment 1) ou représente α-hydroxybenzyle ou α-alcoylbenzyle dont la partie alcoyle contient 1 à 3 atomes de carbone, et
   m, p et q sont définis comme précédemment en 2), ou bien
5) Y est un groupe >CX-(CH₂)ₓ-Ar' pour lequel Ar' est thiényle, phényle éventuellement mono ou poly substitué (par halogène, alcoyle ou alcoyloxy (1 à 4 carbones), trifluorométhyle, hydroxy) ou pyridyle, x est 0 ou 1, et X est -NH-CO-alcoyle don la partie alcoyle contient 1 à 6 carbones,
   m est 2 ou 3, p est 1 et q égale 0
   Ar, T et Z sont définis comme en 2),
   R' est H et R est H ou alcoyle;
et parmi ces produits un antagoniste spécifique des récepteurs NK2 est décrit plus particulièrement par X. Emonds-Alt et Coll., Life Science, 50, PL 100 à PL 106 (1992).

A titre d'exemple des antagonistes des récepteurs NK2 de la classe des polypeptides α-substitués peuvent être des produits de formule générale : 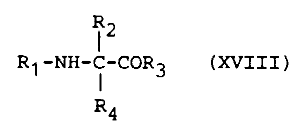 dans laquelle
R₁ est un atome d'hydrogène ou un groupe N-terminal constitué de 0 à 4 amino-acides,
R₂ est une chaine latérale d'amino-acide excepté la glycine,
R₃ est un groupe C-terminal constitué de 0 à 4 amino-acides, un radical OH ou OR dans laquelle R est un radical alcoyle droit ou ramifié ou cycloalcoyle contenant 1 à 6 atomes de carbone,
R₄ est une chaine latérale d'amino-acide excepté la glycine, ou un radical -CH=CH₂, -CH≡CH, -CH₂-CH=CH₂, -CH₂-CH≡CH, -CH₂-Ar, -CH₂-OR, -CH₂-OAr, -(CH₂)ₙCO₂R ou -CH₂-NR₅R₆, n étant un entier de 0 à 3, R étant H ou un radical alcoyle inférieur et Ar étant un carbocycle ou un hétérocycle aromatique ou hydroaromatique mono ou polycyclique non substitué ou substitué,
étant entendu que R₁ et R₃ ne peuvent pas comprendre plus de 4 résidus d'amino-acides en tout.

A titre d'exemple des antagonistes des récepteurs NK2 de la classe des dérivés de pipéridine peuvent être des produits de formule générale : 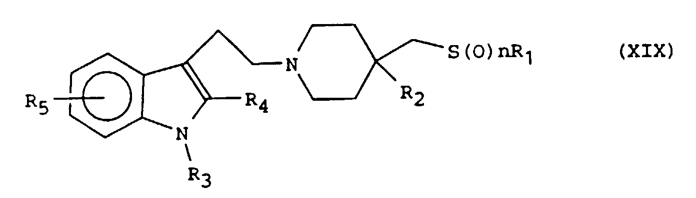 dans laquelle
R₁ est phényle éventuellement substitué par 1 ou 2 alcoyle, alcoyloxy, CF₃, ou halogène,
R₂ est H, OH ou alcoyle,
R₃ est H ou alcoyle,
R₄ est H, alcoyle ou alcoyloxy, et
R₅ est H, alcoyle, CF₃, CN ou halogène et n est 0 à 2, les radicaux alcoyle ayant 1 à 4 carbones;
et parmi ces produits plus spécialement le 1-[2-(5-fluoro-1H-indol-3-yl)éthyl]-4-[(phénylsulfinyl)méthyl]-4-pipéridinol.

D'un intérêt particulier sont les dérivés de perhydroisoindole de formule générale (I) pour lesquels le symbole R₁ représente un radical phényle éventuellement substitué par un radical hydroxy, alcoyloxy, ou dialcoylamino, ou un radical hétérocyclyle mono ou polycyclique, saturé ou insaturé contenant 5 à 9 atomes de carbone et un ou plusieurs hétéroatomes choisis parmi l'oxygène, l'azote ou le soufre, le symbole R₂ représente un atome d'hydrogène ou un radical alcoyle, le symbole R₃ représente un radical phényle éventuellement substitué en position -2 par un radical alcoyloxy contenant 1 ou 2 atomes de carbone ou par un atome de fluor, ou disubstitué par des radicaux trifluorométhyle, les symboles R₅ et R'₅ sont identiques ou différents et représentent l'un un atome d'hydrogène ou un radical hydroxy ou alcoyle et l'autre un atome d'hydrogène ou un radical alcoyle, le symbole R₄ représente un radical hydroxy et, le symbole R₆ représente un atome d'hydrogène ou un radical alcoyle, hydroxy ou hydroxyalcoyle, et les symboles R représentent l'un un atome d'hydrogène, un radical alcoyle, un radical hydroxy ou hydroxyalcoyle et l'autre un atome d'hydrogène, un radical alcoyle ou phényle.

Et parmi ces produits plus spécialement actifs sont les dérivés de perhydroisoindole suivants :
- diméthyl-7,7 (méthoxy-2 phényl)-4 [(méthoxy-2 phényl)-2 propionyl-(S)]-2 perhydroisoindolediol-4,5
- (méthoxy-2 phényl) -4 [(méthoxy-2 phényl)-2 propionyl-(S)]-2 méthyl-5 perhydroisoindolol-4
- [(hydroxy-2 phényl)-2 propionyl-(S)]-2 (méthoxy-2 phényl)-4 méthyl-6 perhydroisoindolol-4
- (hydroxyméthyl)-7 (méthoxy-2 phényl)-4 [(méthoxy-2 phényl)-2 propionyl-(S)]-2 méthyl-7 perhydroisoindolol-4
- (hydroxyméthyl)-7 (indolyl-3 acétyl)-2 (méthoxy-2 phényl)-4 méthyl-7 perhydroisoindolol-4

Les exemples suivants donnés à titre non limitatif illustrent la présente invention.

Dans les exemples qui suivent, il est entendu que, sauf mention spéciale, les spectres de RMN du proton ont été faits à 250 MHz dans le diméthylsulfoxyde ; les déplacements chimiques sont exprimés en ppm.

### Exemple 1

A une solution de 1,54 g de (méthoxy-2 phényl)-4 méthyl-6 perhydroisoindolol-4-(3aRS,4RS,6SR,7aSR) dans 150 cm³ de dichlorométhane on ajoute 1,0 cm³ de triéthylamine. Le mélange réactionnel est refroidi à 5°C et 1,08 g d'acide (méthoxy-2 phényl)-2 propionique-(S), 0,10 g d'hydroxy-1 benzotriazole monohydraté et 1,26 g de chlorhydrate de (diméthylamino-3 propyl)-1 éthyl-3 carbodiimide sont additionnés. Le mélange réactionnel est maintenu 1 heure à 5°C et 16 heures à 20°C puis lavé 2 fois avec 100 cm³ d'eau, séché sur sulfate de magnésium, filtré et concentré à sec sous pression réduite (2,7 kPa). Le résidu est chromatographié sur une colonne de gel de silice (0,060-0,200 mm, diamètre de 4 cm, hauteur de 70 cm) en éluant sous une pression de 0,7 bar par un mélange d'acétate d'éthyle et de cyclohexane [en volumes: 20/80 (2 dm³) puis 30/70 (2 dm³) puis 40/60 (2 dm³) et en recueillant des fractions de 60 cm³. Les fractions 57 à 72 sont concentrées et l'on obtient après séchage à 40°C sous 15 Pa, 0,90 g de (méthoxy-2 phényl)-4 [(méthoxy-2 phényl)-2 propionyl-(S)]-2 méthyl-6 perhydroisoindolol-4-(3aR*,4R*,6S*,7aS*) sous forme d'un solide blanc.

Spectre de RMN du proton (250 MHz, DMSO d₆ + quelques gouttes de CD₃COOD, à une température de 383°K, δ en ppm et J en Hz): 0,9 (d, J = 6,5Hz, 3H); 1,25 (d large, J = 7Hz, 3H); 2,8 (t, J = 8Hz, 1H); 3,6 (s large, 3H); 3,8 (s large, 3H); 4,1 (s large, 1H); 6,9-7,6 (m, 8H)

Spectre IR (KBr, cm⁻¹): 3410, 3070, 3000-2850, 2835, 1635, 1600, 1585, 1495, 1450, 1240, 1060, 1030, 755.

L'acide (méthoxy-2 phényl)-2 propionique-(S) peut être préparé selon la méthode décrite dans la demande de brevet EP 429 366.

Le (méthoxy-2 phényl)-4 méthyl-6 perhydroisoindolol-4-(3aRS,4RS,6SR,7aSR) peut être obtenu de la manière suivante :

A 3,5 g de benzyl-2 (méthoxy-2 phényl)-4 méthyl-6 perhydroisoindolol-4-(3aRS,4RS,65R,7aSR) sont additionnés à 20°C sous atmosphère d'azote 1,2 g de palladium sur charbon à 10% et 75 cm d'éthanol. Après chauffage à 50°C et bullage d'hydrogène pendant deux heures, le mélange réactionnel est refroidi à température ambiante et purgé à l'aide d'un courant d'azote, filtré et concentré sous pression réduite (2,7 kPa). On obtient 2,1g de (méthoxy-2 phényl)-4 méthyl-6 perhydroisoindolol-4-(3aRS,4RS,6SR,7aSR) sous la forme d'un solide blanc. PF= 164°C.

Spectre de RMN du proton (250 MHz, CDCl₃, δ en ppm et J en Hz): 0,60 (d, J = 6,5Hz, 3H); 1,8 (m, 1H); 2,25 (m, 1H); 2,6 (t, J = 9Hz, 1H); 2,8 (t, J = 9,5Hz, 1H); 3,5 (s, 3H); 6,60 (m, 2H); 6,88 (t large, J = 8Hz, 1H); 7,5 (d large, J = 8Hz, 1H)

Le benzyl-2 (méthoxy-2 phényl)-4 méthyl-6 perhydroisoindolol-4-(3aRS,4RS,6SR,7aSR) peut être obtenu de la manière suivante :

135 cm³ d'une solution 1,6 M de n.butyllithium dans l'hexane sont additionnés à 31,3 cm³ d'anisole dilué dans 400 cm³ de t.butylméthyléther et 33 cm³ de tétraméthyléthylènediamine en maintenant une température inférieure à 30°C. Après une heure à 20°C, le mélange réactionnel est refroidi à -72°C et 24 g de benzyl-2 méthyl-6 perhydroisoindolone-4-(3aRS,6SR,7aSR) dissous dans 200 cm³ de tétrahydrofurane sont additionnés à cette température en trente minutes. Après trente minutes d'agitation, 200 cm³ d'une solution aqueuse à 26% de chlorure d'ammonium sont versés en trente minutes et le mélange réactionnel est réchauffé jusqu'à température ambiante et décanté. La phase organique est lavée à l'eau (3 fois 150 cm³) puis avec une solution saturée de chlorure de sodium (150 cm³), séchée sur sulfate de magnésium, filtrée et concentré sous pression réduite (2,7 kPa). Le résidu obtenu est chromatographié sur une colonne de gel de silice (0,060-0,200 mm, diamètre de 6 cm, hauteur de 55 cm) en éluant sous une pression de 0,7 bar par un mélange d'acétate d'éthyle et de cyclohexane [en volumes, 30/70 (3 dm³) puis 50/50 (2 dm³) et 100/0 (3 dm³)] et en recueillant des fractions de 200 cm³. Les fractions 9 à 26 sont réunies, concentrées à sec sous pression réduite (2,7 kPa) et cristallisées dans l'éther isopropylique. On obtient 20,5 g de benzyl-2 (méthoxy-2 phényl)-4 méthyl-6 perhydroisoindolol-4-(3aRS,4RS,6SR, 7aSR) sous forme de cristaux blanc, PF = 120°C.

Spectre de RMN du proton (250 MHz, CDCl₃, δ en ppm et J en Hz): 0,9 (d, J = 6,5Hz, 3H); 2,15 (m, 1H); 3,58 et 3,80 (AB, J = 13Hz, 2H); 3,85 (s, 3H); 6,85 (dd, J = 7,5 et 1,5 Hz, 1H); 7,0 (ddd, J = 7,5 et 1,5Hz, 1H); 7,3 (m, 5H); 8,0 (dd, J = 7,5 et 2Hz, 1H)

Spectre IR (KBr, cm-1): 3350-3150, 3100-3000, 3000-2850, 2835, 2815, 2735, 1595, 1580, 1495, 1485, 1450, 1235, 1035.

La benzyl-2 méthyl-6 perhydroisoindolone-4-(3aRS,6SR,7aSR) peut être obtenue de la manière suivante :

A une solution de 15,9 g de méthyl-5 cyclohexèn-2-one et de 50 cm³ de N-butoxyméthyl N-triméthylsilylméthyl benzylamine dans 200 cm³ de dichlorométhane, on ajoute 10 gouttes d'acide trifluoroacétique. Le mélange réactionnel atteint le reflux après quinze minutes, puis revient lentement (2 heures) à 20°C. 10 g de carbonate de potassium sont additionnés au mélange qui est agité pendant 30 minutes, filtré et concentré sous pression réduite (2,7 kPa). Le résidu obtenu est chromatographié sur une colonne de gel de silice (0,060-0,200 mm, diamètre de 6 cm, hauteur de 50 cm) en éluant sous une pression de 0,7 bar par un mélange d'acétate d'éthyle et de cyclohexane [en volumes, 40/60 (4 dm³) puis 60/40 (4 dm³) et 100/0 (2 dm³)] et en recueillant des fractions de 250 cm³. Les fractions 9 à 42 sont réunies, concentrées à sec sous pression réduite (2,7 kPa) et on obtient 24 g de benzyl-2 méthyl-6 perhydroisoindolone-4-(3aRS,6SR, 7aSR) sous forme d'une huile.

Spectre de RMN du proton (250 MHz, CDCl₃, δ en ppm et J en Hz): 1,0 (d, J = 6,5Hz, 3H); 1,55 (ddd, J = 14, 11 et 6Hz, 1H); 1,75 (ddd, J = 14, 6 et 3Hz, 1H); 2,1 (m, 1H); 3,6 (s, 2H); 7,3 (bm, 5H)

La N-butoxyméthyl N-triméthylsilylméthyl benzylamine peut être préparée selon la méthode de Y.Terao et coll., Chem. Pharm. Bull., 33, 2762 (1985)

La méthyl-5 cyclohexèn-2-one peut être préparée selon la méthode décrite par P.L.Fuchs et A.K.Musser, J. Org. Chem., 47, 3121 (1982).

### Exemple 2

De façon identique à l'exemple 1, on utilise 0,78 g de (méthoxy-2 phényl)-4 méthyl-6 perhydroisoindolol- 4-(3aRS,4RS,6SR,7aSR) et 0,52 g d'acide (méthoxy-2 phényl) acétique. Après chromatographie sur une colonne de gel de silice (0,060-0,200 mm, diamètre de 3,5 cm, hauteur de 40 cm) en éluant sous une pression de 0,7 bar par un mélange de dichlorométhane et de méthanol [94/6 en volumes] et en recueillant des fractions de 45 cm³ les fractions 8 à 15 sont concentrées et l'on obtient après séchage à 40°C sous 15 Pa 1,0 g de (méthoxy-2 phényl)-4 [(méthoxy-2 phényl) acétyl]-2 méthyl-6 perhydroisoindolol-4-(3aRS,4RS,6SR,7aSR) sous forme d'un solide blanc, PF=186°C.

Spectre de RMN du proton (250 MHz, DMSO d₆ + quelques gouttes de CD₃COOD, à une température de 383°K, δ en ppm et J en Hz): 0,9 (d, J = 6,5Hz, 3H); 2,2 (m, 1H); 2,6 (m large, 1H); 2,9 (t, J = 7Hz, 1H); 3,75 (s, 3H); 3,85 (s, 3H); 6,90 (m, 4H); 7,2 (m, 3H); 7,6 (dd, J = 8 et 1,5Hz, 1H).

Spectre IR (KBr, cm-1): 3410, 3100-3000, 3000-2850, 2845, 2800-2300, 1635, 1595, 1495, 1460, 1235, 1115, 1025, 760.

### Exemple 3

A une solution de 0,78 g de (méthoxy-2 phényl)-4 méthyl-6 perhydroisoindolol-4-(3aRS,4RS,6SR,7aSR) dans 80 cm³ de dichlorométhane on ajoute 0,5 cm³ de triéthylamine. Le mélange réactionnel est refroidi à 5°C et 0,54 g d'acide (diméthylamino-2 phényl) acétique, 0,05 g d'hydroxy-1 benzotriazole monohydraté et 0,63 g de chlorhydrate de (diméthylamino-3 propyl)-1 éthyl-3 carbodiimide sont additionnés. Le mélange réactionnel est maintenu 1 heure à 5°C et 16 heures à 20°C puis lavé 2 fois avec 100 cm³ d'eau, séché sur sulfate de magnésium, filtré et concentré sous pression réduite (2,7 kPa). Le résidu est chromatographié sur une colonne de gel de silice (0,060-0,200 mm, diamètre de 4 cm, hauteur de 50 cm) en éluant sous une pression de 0,7 bar par un mélange de dichlorométhane et de méthanol [97,5/2,5 en volumes] et en recueillant des fractions de 65 cm³. Les fractions 17 à 23 sont concentrées et l'on obtient après séchage à 40°C sous 15 Pa 0,90 g de (méthoxy-2 phényl)-4 [(diméthylamino-2 phényl) acétyl]-2 méthyl-6 perhydroisoindolol-4-(3aRS,4RS,6SR,7aSR) sous forme d'une poudre blanche qui est dissoute dans 50 cm³ de dioxanne. On ajoute 3 cm³ d'une solution 5M d'acide chlorhydrique dans le dioxanne, après 1 heure à température ambiante la solution est concentrée sous pression réduite (2,7 kPa), triturée dans l'éther isopropylique, filtrée et séchée à 40°C sous 15 Pa. On obtient 1,0 g de chlorhydrate de (méthoxy-2 phényl)-4 [(diméthylamino-2 phényl) acétyl]-2 méthyl-6 perhydroisoindolol-4-(3aRS,4RS,6SR,7aSR).

Spectre de RMN du proton (250 MHz, DMSO d₆ + quelques gouttes de CD₃COOD, à une température de 413°K, δ en ppm et J en Hz): 0,95 (d, J = 7,5Hz, 3H); 1,40 (ddd, J = 17,5, 13,5 et 7,5, 1H); 1,7 (m, 1H); 1,85 (d, J = 17,5Hz, 1H); 2,05 (m, 1H); 2,2 (m, 1H); 2,6 (m, 1H); 3,1 (s, 6H); 3,6 (s, 2H); 3,87 (s, 3H); 6,9-7,7 (m, 8H)

Spectre IR (KBr, cm⁻¹): 3410, 3100-3000, 3000-2850, 2845, 2800-2300, 1635, 1595, 1495, 1460, 1235, 1115, 1025, 760.

L'acide diméthylamino-2 phényl acétique peut être préparé selon la méthode décrite dans la demande de brevet EP 429 366.

### Exemple 4

De façon identique à l'exemple 1, on utilise 0,78 g de (méthoxy-2 phényl)-4 méthyl-6 perhydroisoindolol-4-(3aRS,4RS,6SR,7aSR) et 0,52 g d'acide indole-3 acétique. Après chromatographie sur une colonne de gel de silice (0,060-0,200 mm, diamètre de 3,5 cm, hauteur de 40 cm) en éluant sous une pression de 0,7 bar par un mélange de dichlorométhane et de méthanol [94/6 en volumes] et en recueillant des fractions de 45 cm³, les fractions 10 à 16 sont concentrées à sec et l'on obtient après séchage à 40°C sous 15 Pa 1,0 g d'(indolyl-3 acétyl)-2 (méthoxy-2 phényl)-4 méthyl-6 perhydroisoindolol-4-(3aRS,4RS,6SR,7aSR) sous forme d'un solide blanc.

Spectre de RMN du proton (250 MHz, DMSO d₆ + quelques gouttes de CD₃COOD, à une température de 383°K, δ en ppm et J en Hz): 0,9 (d, J = 6,5Hz, 3H); 2,2 (m, 1H); 2,6 (m, 1H); 2,88 (t, J = 7Hz, 1H); 3,65 (s, 2H); 3,85 (s, 3H); 6,9-7,6 (m, 9H).

Spectre IR (KBr, cm⁻¹): 3410, 3260, 3100-3000, 3000-2850, 2835, 1625, 1585, 1415, 1450, 1235, 1105, 1025, 755, 745.

### Exemple 5

En opérant selon le mode opératoire de l'exemple 6 ci-après, à partir de 1 g de (méthoxy-2 phényl)-4 perhydroisoindolol-4-(3aRS,4RS,7aSR) et de 0,87 g d'acide (méthoxy-2 phényl)-2 propionique-(S), on obtient, après purification sur colonne de gel de silice (granulométrie 0,04-0,06 mm, diamètre 3,2 cm, hauteur 18 cm) 0,19 g de (méthoxy-2 phényl)-4 [(méthoxy-2 phényl)-2 propionyl-(S)]-2 perhydroisoindolol-4-(3aR*,4R*,7aS*), sous la forme d'une meringue blanche.

Spectre de RMN du proton (DMSO d₆) :
1,3 (d, J=7,5; 3H, CH₃) ; 2,9 (q large, J=6,5; 1H, H en 3a) ; 3,78 (bs, 3H, OCH₃) ; 3,85 (s, 3H, OCH₃) ; 4,17 (bq, J=7,5; CH-CH₃) ; 6,9 à 7,25 (m, 8H aromatiques).

Spectre IR (KBr - bandes caractéristiques en cm⁻¹) :
3425, 3070, 3000-2850, 2835, 1635, 1595, 1580, 1495, 1450, 1245, 1060, 1035, 755.

Le (méthoxy-2 phényl)-4 perhydroisoindolol-4-(3aRS, 4RS, 7aSR) peut être préparé de la manière suivante :

Un mélange de 16 g de benzyl-2 (méthoxy-2 phényl)-4 perhydroisoindolol-4-(3aRS,4RS,7aSR) et de 200 cm³ d'éthanol anhydre est chauffé à 60°C sous agitation; on ajoute 2 g d'hydroxyde de palladium sur charbon à 20% puis le mélange réactionnel est hydrogéné, sous agitation, à une température de 60°C et sous pression atmosphérique. Après 6 heures de réaction le mélange réactionnel est filtré, puis concentré à sec sous pression réduite (2,7 kPa). On obtient 5,72 g de (méthoxy-2 phényl)-4 perhydroisoindolol-4-(3aRS,4RS,7aSR) sous la forme d'une meringue crème.

Le benzyl-2 (méthoxy-2 phényl)-4 perhydroisoindolol-4-(3aRS,4RS,7aSR) peut être préparé de la manière suivante :

A une suspension de 55,63 g de bromure de méthoxy-2 phénylmagnésium dans 250 cm³ de tétrahydrofurane, on ajoute goutte à goutte, à température ambiante, sous agitation, une solution de 20,16 g de benzyl-2 perhydroisoindolone-4-(3aRS,7aSR) dans 250 cm³ de tétrahydrofurane. Le mélange réactionnel est agité à température ambiante pendant 18 heures, traité par 300 cm³ d'une solution aqueuse saturée en chlorure d'ammonium, repris par 200 cm³ d'oxyde d'éthyle et lavé par 300 cm³ d'une solution aqueuse saturée en chlorure de sodium. La phase organique est décantée, séchée sur sulfate de magnésium, filtrée et concentrée à sec sous pression réduite (2,7 kPa). Le résidu est chromatographié sur colonne de gel de silice (granulométrie 0,04-0,06 mm, diamètre 8,8 cm, hauteur 41 cm), en éluant sous une pression de 0,5 bar d'azote par un mélange de cyclohexane et d'acétate d'éthyle (80/20 en volumes) et en recueillant des fractions de 1000 cm³. Les fractions 4 à 27 sont réunies puis concentrées à sec sous pression réduite (2,5 kPa). On obtient 24,96 g de benzyl-2 (méthoxy-2 phényl)-4 perhydroisoindolol-4-(3aRS,4RS,7aSR) sous la forme de cristaux blancs fondant à 106°C.

La benzyl-2 perhydroisoindolone-4-(3aRS,7aSR) peut être préparée de la manière suivante :

A une solution de 20 g de cyclohexène-2-one et de 69 cm³ de N-butoxyméthyl N-triméthylsilylméthyl benzylamine dans 250 cm³ de dichlorométhane, on ajoute, à une température de 10°C, 5 gouttes d'acide trifluoroacétique. Le mélange réactionnel est agité à cette température pendant 3 heures, puis on ajoute du carbonate de potassium, filtre la solution sur verre fritté et concentre à sec sous pression réduite (2,7 kPa). Le résidu est chromatographié sur colonne de gel de silice (granulométrie 0,04-0,06 mm, diamètre 8,8 cm, hauteur 38 cm), en éluant sous une pression de 0,5 bar d'azote par un mélange de cyclohexane et d'acétate d'éthyle (75/25 en volumes) et en recueillant des fractions de 1000 cm³. Les fractions 4 à 26 sont réunies et concentrées à sec sous pression réduite (2,7 kPa). On obtient 28,23 g de benzyl-2 perhydroisoindolone-4-(3aRS,7aSR) sous forme d'une huile jaune.

La N-butoxyméthyl N-triméthylsilylméthyl benzylamine peut être préparée selon la méthode de Y. TARAO et coll., Chem. Pharm. Bull., 33, 2762, (1985).

### Exemple 6

A une solution de 1,5 g de (méthoxy-2 phényl)-4 perhydroisoindolol-4-(3aRS,4RS,7aSR) et de 1,27 g d'acide indolyl-3 acétique dans 40 cm³ de dichlorométhane, refroidie à 0°C, on ajoute 0,08 g d'hydroxy-1-benzotriazole et 1,4 g de chlorhydrate de (diméthylamino-3 propyl)-1 éthyl-3 carbodiimide. On agite 18 heures à température ambiante puis on lave la phase organique par 2 fois 80 cm³ d'eau, puis par 80 cm³ d'une solution aqueuse saturée en chlorure de sodium, on sèche sur sulfate de magnésium et concentre à sec sous pression réduite (2,7 kPa). Le résidu est chromatographié sur colonne de gel de silice (granulométrie 0,04-0,06 mm, diamètre 4 cm, hauteur 40 cm), en éluant sous une pression de 0,5 bar d'azote par un mélange de cyclohexane et d'acétate d'éthyle (70/30 en volumes) et en recueillant des fractions de 40 cm³. Les fractions 24 à 48 sont réunies puis concentrées à sec sous pression réduite (2,7 kPa). On obtient 1,21 g de (méthoxy-2 phényl)-4 (indolyl-3 acétyl)-2 perhydroisoindolol-4-(3aRS,4RS,7aSR) sous la forme d'une meringue blanc cassé.

Spectre de RMN du proton (DMSO d₆) :
2,9 (vb, 1H, H₃ₐ) ; 3,6 (bs, 2H, CH₂CO) ; 3,8 (s, 3H, OCH₃) ; 6,9 à 7,5 (m, 9H aromatiques).

Spectre IR (KBr - bandes caractéristiques en cm⁻¹) :
3540, 3470, 3000-2850, 2835, 1635, 1580, 1490, 1450, 1235, 1060, 1035.

### Exemple 7

En opérant selon le mode opératoire de l'exemple 8 ci-après, à partir de 0,72 g de (méthoxy-2 phényl)-4 perhydroisoindolediol-4,5-(3aRS,4RS,5RS,7aSR) et de 0,54 g d'acide (méthoxy-2 phényl)-2 propionique-(S), on obtient après purification sur colonne d'alumine (granulométrie 50-150 µm, hauteur = 30 cm, diamètre = 4 cm) en éluant par de l'acétate d'éthyle puis par un mélange de dichlcrométhane et de méthanol (99/1 en volumes), 0,17 g de (méthoxy-2 phényl)-4 [(méthoxy-2 phényl)-2 propionyl- (S)]-2 perhydroisoindolediol-4,5-(3aR*,4R*,5R*,7aS*), fondant à 107-109°C.

Le (méthoxy-2 phényl)-4 perhydroisoindolediol-4,5-(3aRS,4RS,5RS,7aSR) peut être préparé de la manière suivante :

Un mélange de 1,5 g de benzyl-2 (méthoxy-2 phényl)-4 perhydroisoindolediol-4,5 (3aRS, 4RS, 5RS, 7aSR) et de 120 cm³ d'éthanol anhydre est chauffé à 60°C sous agitation; on ajoute 0,49g d'hydroxyde de palladium sur charbon à 20% puis le mélange réactionnel est hydrogéné, sous agitation, à une température de 60°C et sous pression atmosphérique. Après 2 heures de réaction, le volume théorique d'hydrogène a été absorbé ; le mélange réactionnel est filtré, puis concentré à sec sous pression réduite (2,7 kPa). Le résidu est cristallisé dans 50 cm³ d'oxyde d'isopropyle. On obtient 1,02 g de (méthoxy-2 phényl)-4 perhydroisoindolediol-4,5-(3aRs,4Rs,5Rs,7asR) fondant à 168°C.

Le benzyl-2 (méthoxy-2 phényl) -4 perhydroisoindolediol-4,5-(3aRS,4RS,5RS,7aSR) peut être préparé de la manière suivante :

A une suspension de 27,6 g de bromure de méthoxy-2 phénylmagnésium dans 60 cm³ de tétrahydrofurane, on ajoute goutte à goutte, à température ambiante, sous agitation, une solution de 4,7 g d'acétoxy-5 benzyl-2 perhydroisoindolone-4-(3aRS,5RS,7aSR) dans 90 cm³ de tétrahydrofurane. Le mélange réactionnel est agité à température ambiante pendant 3 heures, traité par 200 cm³ d'une solution aqueuse saturée en chlorure d'ammonium, repris par 200 cm³ d'oxyde d'éthyle et 100g de glace. La phase organique est décantée, séchée sur sulfate de magnésium, filtrée et concentrée à sec sous pression réduite (2,7 kPa). Le résidu est cristallisé dans 80 cm³ d'éther de pétrole puis chromatographié sur colonne de gel de silice (granulométrie 0,04-0,06 mm, diamètre 1 cm, hauteur 20 cm), en éluant sous une pression de 0,5 bar d'azote par un mélange de dichlorométhane et de méthanol (95/5 en volumes) et en recueillant des fractions de 25 cm³. Les fractions 14 à 21 sont réunies puis concentrées à sec sous pression réduite (2,5 kPa). On obtient 1,55 g de benzyl-2 (méthoxy-2 phényl)-4 perhydroisoindolediol-4,5-(3aRS,4RS,5RS,7aSR) fondant à 140°C.

L'acétoxy-5 benzyl-2 perhydroisoindolone-4-(3aRS,5RS,7aSR) peut être préparée de la manière suivante :

A une solution de 7,5 g d'acétoxy-6 cyclohexènone-2 et de 16,83 cm³ de N-butoxyméthyl N-triméthylsilylméthyl benzylamine dans 150 cm³ de dichlorométhane, on ajoute, à une température de 10°C, 12 gouttes d'acide trifluoroacétique. Le mélange réactionnel est agité à température ambiante 7 heures puis on ajoute 2 g de carbonate de potassium, filtre la solution sur verre fritté et concentre à sec sous pression réduite (2,7 kPa). Le résidu est chromatographié sur colonne de gel de silice (granulométrie 0,04-0,06 mm, diamètre 6 cm, hauteur 35 cm), en éluant sous une pression de 0,5 bar d'azote par un mélange de dichlorométhane et de méthanol (97,5-2,5 en volumes) et en recueillant des fractions de 60 cm³. Les fractions 25 à 39 sont réunies et concentrées à sec sous pression réduite (2,7 kPa). On obtient 6,25 g d'acétoxy-5 benzyl-2 perhydroisoindolone-4-(3aRS,5RS,7aSR) sous forme d'une huile jaune.

La N-butoxyméthyl N-triméthylsilylméthyl benzylamine peut être préparée selon la méthode de Y. TARAO et coll., Chem. Pharm. Bull., 33, 2762, (1985).

L'acétoxy-6 cyclohexènone peut être préparée selon la méthode décrite par G.M. Rubottom and coll., J. Org. Chem., 1978, 43, 1599.

### Exemple 8

A une solution de 0,25 g de (méthoxy-2 phényl)-4 perhydroisoindolediol-4,5-(3aRS,4RS,5RS,7aSR) et de 0,18 g d'acide indolyl-3 acétique dans 35 cm³ de dichlorométhane, refroidie à 0°C, on ajoute 5 mg d'hydroxy-1-benzotriazole, 0,22 g de chlorhydrate de (diméthylamino-3 propyl)-1 éthyl-3 carbodiimide et 0,33 cm³ de diisopropyléthylamine. On agite 15 heures à température ambiante, ajoute 40 cm³ de dichlorométhane, lave la phase organique par 3 fois 60 cm³ d'eau, sèche sur sulfate de magnésium et concentre à sec sous pression réduite (2,7 kPa) . La meringue crême obtenue est chromatographiée sur colonne de gel de silice (granulométrie 0,04-0,06 mm, diamètre 1 cm, hauteur 30 cm), en éluant sous une pression de 0,5 bar d'azote par un mélange de dichlorométhane et de méthanol (93/7 en volumes) et en recueillant des fractions de 25 cm³. Les fractions 7 à 10 sont réunies puis concentrées à sec sous pression réduite (2,7 kPa). On obtient 0,35 g de (méthoxy-2 phényl)-4 [(indolyl-3)-acétyl]-2 perhydroisoindolediol-4,5 (3aRS,4Rs,5RS,7aSR) fondant à 166°C.

### Exemple 9

A une suspension de 2,61 g de bromure de méthoxy-2 phénylmagnésium dans 10 cm³ de tétrahydrofurane refroidie à 15°C, on ajoute goutte à goutte sous agitation, une solution de 1,3 g de diméthyl-7,7 [(méthoxy-2 phényl)-acétyl]-2 perhydroisoindolone-4- (3aRS,7aRS) dans 30 cm³ de tétrahydrofurane, puis 2 g de chlorure de cérium anhydre. Le mélange réactionnel est agité à température ambiante pendant 18 heures, traité par 80 cm³ d'une solution aqueuse saturée en chlorure d'ammonium, repris par 100 cm³ d'acétate d'éthyle et lavé par 100 cm³ d'une solution aqueuse saturée en chlorure de sodium. La phase organique est décantée, séchée sur sulfate de magnésium, filtrée et concentrée à sec sous pression réduite (2,7 kPa). Le résidu est chromatographié sur colonne de gel de silice (granulométrie 0,04-0,06 mm, diamètre 3,6 cm, hauteur 22 cm), en éluant sous une pression de 0,5 bar d'azote par un mélange de cyclohexane et d'acétate d'éthyle (70/30 en volumes) et en recueillant des fractions de 50 cm³. Les fractions 47 à 54 sont réunies puis concentrées à sec sous pression réduite (2,5 kPa). Le résidu est cristallisé dans 1 cm³ d'acétonitrile. On obtient 0,177 g de diméthyl-7,7 (méthoxy-2 phényl)-4 [(méthoxy-2 phényl)-acétyl]-2 perhydroisoindolol-4-(3aRS,4RS,7aRS) sous la forme de cristaux blancs fondant à 187°C.

La diméthyl-7,7 (méthoxy-2 phényl)-acétyl-2 perhydroisoindolone-4-(3aRS,7aRS) peut être préparée de la façon suivante :

En opérant selon le mode opératoire de l'exemple 6, à partir de 2,17 g de chlorhydrate de diméthyl-7,7 perhydroisoindolone-4-(3aRS,7aRS) et de 2,12 g d'acide méthoxy-2 phénylacétique et en ajoutant 1,37 g de diisopropyléthylamine, on obtient, après purification sur colonne de gel de silice (granulométrie 0,04-0,06 mm, diamètre 3,6 cm, hauteur 20 cm), 0,9 g de diméthyl-7,7 (méthoxy-2 phényl)-acétyl-2 perhydroisoindolone-4-(3aRS,7aRS), sous la forme de cristaux blancs fondant à 128°C.

Le chlorhydrate de diméthyl-7,7 perhydroisoindolone-4-(3aRS,7aRS) peut être préparé de la façon suivante :

Un mélange de 2,31 g de benzyl-2 diméthyl-7,7 perhydroisoindolone-4-(3aRS,7aRS) et de 9,96 cm³ d'acide chlorhydrique 1N dans 25cm3 d'éthanol anhydre est chauffé à 60°C sous agitation; on ajoute 0,11 g d'hydroxyde de palladium sur charbon à 20% puis le mélange réactionnel est hydrogéné, sous agitation, à une température de 60°C et sous pression atmosphérique. Après 6 heures de réaction le mélange réactionnel est filtré, puis concentré à sec sous pression réduite (2,7 kPa). On obtient 2,17 g de chlorhydrate de diméthyl-7,7 perhydroisoindolone-4-(3aRS,7aRS) sous la forme d'une huile rose contenant de l'éthanol.

La benzyl-2 diméthyl-7,7 perhydroisoindolone-4-(3aRS,7aRS) peut être préparée de la manière suivante :

A une solution de 5 g de diméthyl-4,4 cyclohexène-2-one et de 12,4 cm³ de N-butoxyméthyl N-triméthylsilylméthyl benzylamine dans 100 cm³ de dichlorométhane, on ajoute, à une température de 10°C, 5 gouttes d'acide trifluoroacétique. Le mélange réactionnel est agité à cette température pendant 3 heures, puis on ajoute du carbonate de potassium, filtre la solution sur verre fritté et concentre à sec sous pression réduite (2,7 kPa). Le résidu est chromatographié sur colonne de gel de silice (granulométrie 0,04-0,06 mm, diamètre 5,2 cm, hauteur 25 cm), en éluant sous une pression de 0,5 bar d'azote par un mélange de cyclohexane et d'acétate d'éthyle (85/15 en volumes) et en recueillant des fractions de 100 cm³. Les fractions 39 à 63 sont réunies et concentrées à sec sous pression réduite (2,7 kPa). On obtient 3,64 g de benzyl-2 diméthyl-7,7 perhydroisoindolone-4-(3aRS,7aRS) sous forme d'une huile jaune.

### Exemple 10

En opérant selon le mode opératoire de l'exemple 9, à partir de 0,78 g de diméthyl-7,7 [(méthoxy-2 phényl)-2 propionyl-(S)]-2 perhydroisoindolone-4 (mélange des diastéréoisomères (3aR,7aR) et (3aS,7aS)), et de 1,5 g de bromure de méthoxy-2 phénylmagnésium, on obtient, après purification sur colonne de gel de silice (granulométrie 0,04-0,06 mm, diamètre 2,8 cm, hauteur 21 cm), 0,27 g de diméthyl-7,7 (méthoxy-2 phényl)-4 [(méthoxy-2 phényl)-2 propionyl-(5)1-2 perhydroisoindolol-4-(3aR*,4R*,7aR*), sous la forme d'une meringue blanche.

Spectre de RMN du proton (DMSO d₆) :
1,1-1,15-1 et 0,9 (4s, 4 CH₃) ; 1,25 et 1,17 (2d, J=7, 2 CH₃-CH) ; 3,85-3,8-3,78 et 3,6 (4s, 4 OCH₃) ; 6,7 à 7,6 (m, 8H, aromatiques).

Spectre IR (KBr - bandes caractéristiques en cm⁻¹) :
3400, 3100-3000, 3000-2850, 2835, 1630, 1600, 1585, 1495, 1245, 1065, 1035, 755.

La diméthyl-7,7 [(méthoxy-2 phényl)-2 propionyl-(S)]-2 perhydroisoindolone-4-(mélange des diastéréoisomères (3aR,7aR) et (3aS,7aS)) peut être obtenue de la façon suivante :

En opérant selon le mode opératoire de l'exemple 6 à partir de 1 g de chlorhydrate de diméthyl-7,7 perhydroisoindolone-4-(3aRS,7aRS) et de 1,06 g d'acide (méthoxy-2 phényl)-2 propionique-(S), on obtient, après purification sur colonne de gel de silice (granulométrie 0,04-0,06 mm, diamètre 3,2 cm, hauteur 20,5 cm), 0,96 g de diméthyl-7,7 [(méthoxy-2 phényl)-2 propionyl-(S)]-2 perhydroisoindolone-4-(mélange des diastéréoisomères (3aR,7aR) et (3aS,7aS)), sous la forme d'une huile incolore.

### Exemple 11

En opérant selon le mode opératoire de l'exemple 6, à partir de 0,6 g de diméthyl-7,7 (méthoxy-2 phényl)-4 perhydroisoindolol-4-(3aRS,4RS,7aRS) et de 0,41 g d'acide diméthylamino-2 phénylacétique, on obtient, après purification sur colonne de gel de silice (granulométrie 0,04-0,06 mm, diamètre 2,8 cm, hauteur 17 cm), 0,4 g de diméthyl-7,7 (méthoxy-2 phényl)-4 [(diméthylamino-2 phényl)-acétyl]-2 perhydroisoindolol-4-(3aRS,4RS,7aRS) sous la forme d'une meringue blanche.

Spectre de RMN du proton (DMSO d₆) :
1 (s, 3H, CH₃) ; 1,2 (s, 3H, CH₃) ; 2,6 (ms, 6H, N(CH₃)₂) ; 3 (dd, J=6,5 et 6, 1H, H₇ₐ ou H₃ₐ) ; 3,1 (ms, 1H) ; 3,4 (d, J=11, 1H) ; 3,5 (mt, 4H) ; 3,85 (s, 3H, OCH₃) ; 6,9 à 7,5 (m, 8H aromatiques).

Spectre IR (KBr - bandes caractéristiques en cm⁻¹) :
3580, 3000-2850, 2835, 2790, 1630, 1595, 1580, 1495, 1455, 1240, 1065, 1035.

Le chlorhydrate de diméthyl-7,7 (méthoxy-2 phényl)-4 perhydroisoindolol-4-(3aRS,4RS,7aRS) peut être préparé de la façon suivante :

A une solution de 1,49 g de diméthyl-7,7 (méthoxy-2 phényl)-4 t.butyloxycarbonyl-2 perhydroisoindolol-4-(3aRS,4RS,7aRS) dans 21 cm³ de dioxane on ajoute 35 cm³ d'une solution 7,4 N d'acide chlorhydrique dans le dioxane. Le mélange réactionnel est agité pendant 1 heure à température ambiante, puis concentré à sec sous pression réduite (2,7 kPa). On obtient 1,47 g de chlorhydrate de diméthyl-7,7 (méthoxy-2 phényl)-4 perhydroisoindolol-4-(3aRS,4RS,7aRS) sous la forme d'une meringue blanche.

Le diméthyl-7,7 (méthoxy-2 phényl)-4 t.butyloxycarbonyl-2 perhydroisoindolol-4-(3aRS,4RS,7aRS) peut être préparé de la façon suivante :

En opérant selon le mode opératoire de l'exemple 9, à partir de 4,4 g de bromure de méthoxy-2 phénylmagnésium et de 1,86 g de diméthyl-7,7 t.butyloxycarbonyl-2 perhydroisoindolone-4-(3aRS,7aRS) on obtient 2,6 g de diméthyl-7,7 (méthoxy-2 phényl)-4 t.butyloxycarbonyl-2 perhydroisoindolol-4-(3aRS,7aRS) sous la forme de cristaux blancs fondant à 178°C.

La diméthyl-7,7 t.butyloxycarbonyl-2 perhydroisoindolone-4-(3aRS,7aRS) peut être préparée de la façon suivante :

A une solution de 3 g de chlorhydrate de diméthyl-7,7 perhydroisoindolone-4-(3aRS,7aRS) et de 1,49 g de triéthylamine dans 80 cm³ de dichlorométhane sec, on ajoute 3,56 g de dicarbonate de ditert-butyle, puis 0,18 g de diméthylamino-4 pyridine. Le mélange réactionnel est agité à température ambiante pendant 1 heure, puis lavé 2 fois par 100 cm³ d'une solution aqueuse d'acide citrique, puis par 100 cm³ d'une solution aqueuse saturée en hydrogénocarbonate de sodium, puis par 100 cm³ d'une solution aqueuse saturée en chlorure de sodium. La phase organique est séchée sur sulfate de magnésium, puis concentrée à sec sous pression réduite (2,7 kPa). Le résidu est chromatographié sur colonne gel de silice (granulométrie 0,04-0,06 mm, diamètre 2,8 cm, hauteur 15 cm), en éluant sous une pression de 0,5 bar d'azote par un mélange de cyclohexane et d'acétate d'éthyle (75/25) et en recueillant des fractions de 30 cm³. Les fractions 4 à 9 sont réunies et concentrées à sec sous pression réduite (2,7 kPa). On obtient 3,07 g de diméthyl-7,7 t.butyloxycarbonyl-2 perhydroisoindolone-4-(3aRS,7aRS) sous la forme de cristaux blancs fondant à 130°C.

### Exemple 12

En opérant selon le mode opératoire de l'exemple 6, à partir de 0,6 g de chlorhydrate de diméthyl-7,7 (méthoxy-2 phényl)-4 perhydroisoindolol-4-(3aRS,4RS,7aRS) et de 0,4 g d'acide indolyl-3 acétique, on obtient, après purification sur colonne de gel de silice (granulométrie 0,04-0,06mm, diamètre 2,4 cm, hauteur 15 cm), 0,17 g de diméthyl-7,7 (méthoxy-2 phényl)-4 [(indolyl-3)-acétyl]-2 perhydroisoindolol-4-(3aRS,4RS,7aRS), sous la forme d'une meringue blanche.

Spectre de RMN du proton (DMSO-d₆) :
1 (s, 3H, CH₃) ; 1,2 (s, 3H, CH₃) ; 2,65 (ddd, J=15-14 et 4,5, 1H, H₅) ; 3 (dd large, J=6,5 et 6, H₇ₐ ou H₃ₐ) ; 3,65 (s large, 2H, CH₂CO) ; 3,9 (s, 3H, OCH₃) ; 6,9 à 7,6 (m, 9H aromatiques).

Spectre IR (KBr - bandes caractéristiques en cm⁻¹) :
3580, 3475, 3000-2850, 2835, 1630, 1595, 1580, 1485, 1450, 1240, 1065, 1035.

### Exemple 13

En opérant selon le mode opératoire de l'exemple 8, à partir de 1,24 g de diméthyl-7,7 (méthoxy-2 phényl)-4 perhydroisoindolediol-4,5-(3aRS,4RS,5RS,7aRS) et de 0,85 g d'acide (méthoxy-2 phényl)-2 propionique-(S), on obtient après purification sur colonne de gel de silice (granulométrie 0,04-0,06 mm, hauteur 20 cm, diamètre 3 cm), 0,46 g de diméthyl-7,7 (méthoxy-2 phényl)-4 [(méthoxy-2 phényl)-2 propionyl-(S)]-2 perhydroisoindoledio1-4,5-(3aR*,4R*,5R*,7aS*), fondant à 194°C.

Le diméthyl-7,7 (méthoxy-2 phényl)-4 perhydroisoindolediol-4,5-(3aRS,4RS,5RS,7aRS) peut être préparé de la manière suivante :

Un mélange de 2,7 g de benzyl-2 diméthyl-7,7 (méthoxy-2 phényl)-4 perhydroisoindolediol-4,5-(3aRS,4RS,5RS,7aRS) et de 75 cm³ d'éthanol anhydre est chauffé à 60°C sous agitation ; on ajoute 0,8 g d'hydroxyde de palladium sur charbon à 20 % puis le mélange réactionnel est hydrogéné, sous agitation, à une température de 60°C et sous pression atmosphérique. Après 1 heure de réaction, le volume théorique d'hydrogène a été absorbé ; le mélange réactionnel est filtré, puis concentré à sec sous pression réduite (2,7 kPa). Le résidu est cristallisé dans 15 cm³ d'oxyde d'isopropyle. On obtient 1,74 g de diméthyl-7,7 (méthoxy-2 phényl)-4 perhydroisoindolediol-4,5-(3aRS,4RS,5RS,7aRS) fondant à 208°C.

Le benzyl-2 diméthyl-7,7 (méthoxy-2 phényl)-4 perhydroisoindolediol-4,5-(3aRS,4RS,5RS,7aSR) peut être préparé de la manière suivante :

A une suspension de 33,8 g de bromure de méthoxy-2 phénylmagnésium dans 75 cm³ de tétrahydrofurane, on ajoute goutte à goutte, à température ambiante, sous agitation, une solution de 6,6 g d'acétoxy-5 benzyl-2 diméthyl-7,7 perhydroisoindolone-4-(3aRS,5RS,7aRS) dans 75 cm³ de tétrahydrofurane. Le mélange réactionnel est agité à température ambiante pendant 15 heures, traité par 200 cm³ d'une solution aqueuse saturée en chlorure d'ammonium, repris par 200 cm³ d'oxyde d'éthyle et 100 g de glace. La phase organique est extraite par 3 fois 100 cm³ d'oxyde d'éthyle, séchée sur sulfate de magnésium, filtrée et concentrée à sec sous pression réduite (2,7 kPa). Le résidu est cristallisé dans 500 cm³ d'éther de pétrole. On obtient 2,8 g de benzyl-2 diméthyl-7,7 (méthoxy-2 phényl)-4 perhydroisoindolediol-4,5-(3aRS,4RS,5RS,7aRS) fondant à 190°C.

L'acétoxy-5 benzyl-2 diméthyl-7,7 perhydroisoindolone-4-(3aRS,5RS,7aRS) peut être préparée de la manière suivante :

A une solution de 23,2 g d'acétoxy-6 diméthyl-4,4 cyclohexènone-2 et de 0,8 cm³ d'acide trifluoroacétique dans 770 cm³ de dichlorométhane, on ajoute, à température ambiante, 43 cm³ de N-butoxyméthyl N-triméthylsilylméthyl benzylamine. Le mélange réactionnel est agité à température ambiante 15 heures puis on ajoute 5 g de carbonate de potassium, filtre la solution sur verre fritté et concentre à sec sous pression réduite (2,7 kPa). Le résidu est chromatographié sur colonne de gel de silice (granulométrie 0,04-0,06 mm, diamètre 8 cm, hauteur 53 cm), en éluant sous une pression de 0,5 bar d'azote par un mélange d'acétate d'éthyle et de cyclohexane (20-80 en volumes) et en recueillant des fractions de 250 cm³. Les fractions 18 à 27 sont réunies et concentrées à sec sous pression réduite (2,7 kPa). On obtient 6,6 g d'acétoxy-5 benzyl-2 diméthyl-7,7 perhydroisoindolone-4-(3aRS,5RS,7aRS) sous forme d'une huile jaune.

La N-butoxyméthyl N-triméthylsilylméthyl benzylamine peut être préparée selon la méthode de Y. TARAO et coll., Chem. Pharm. Bull., 33, 2762, (1985).

L'acétoxy-6 diméthyl-4,4 cyclohexènone peut être préparée selon la méthode décrite par D.S. WATT et coll., Tetrahedron Lett., 1984, 25, 5839.

### Exemple 14

A une solution de 0,44 g de diméthyl-7,7 (méthoxy-2 phényl)-4 perhydroisoindolediol-4,5-(3aRS, 4RS, 5RS, 7aRS) et de 0,29 g d'acide indolyl-3 acétique dans 40 cm³ de dichlorométhane, refroidie à 0°C, on ajoute 5 mg d'hydroxy-1-benzotriazole, 0,35 g de chlorhydrate de (diméthylamino-3 propyl)-1 éthyl-3 carbodiimide et 0,42 cm³ de diisopropyléthylamine. On agite 15 heures à température ambiante, ajoute 100 cm³ de dichlorométhane, lave la phase organique par 50 cm³ d'une solution aqueuse de bicarbonate de sodium et par 60 cm³ d'une solution aqueuse saturée de chlorure de sodium, séche sur sulfate de magnésium et concentre à sec sous pression réduite (2,7 kPa). La meringue crème obtenue est chromatographiée sur colonne de gel de silice (granulométrie 0,04-0,06 mm, diamètre 1 cm, hauteur 30 cm), en éluant sous une pression de 0,5 bar d'azote par un mélange de dichlorométhane et de méthanol (97/3 en volumes) et en recueillant des fractions de 30 cm³. Les fractions 5 à 7 sont réunies puis concentrées à sec sous pression réduite (2,7 kPa) ; le solide obtenu est recristallisé dans 5 cm³ d'oxyde d'isopropyle. On obtient 0,48 g de diméthyl-7,7 (méthoxy-2 phényl)-4 [(indolyl-3)-acétyl]-2 perhydroisoindolediol-4,5 (3aRS,4RS,5RS,7aRS) fondant à 160-165°C.

### Exemple 15

A une suspension de bromure de méthoxy-2 phénylmagnésium préparée à partir de 0,576 g de magnésium et de 4,5 g de bromo-2 anisole dans 25 cm³ de tétrahydrofurane, on ajoute goutte à goutte sous agitation à 30°C, une solution de 1,6 g de méthyl-7 phényl-7 [(méthoxy-2 phényl) -acétyl]-2 perhydroisoindolone-4-(3aRS,7SR,7aRS) dans 40 cm³ de tétrahydrofurane. Le mélange réactionnel est agité à température ambiante pendant 18 heures, traité par 100 cm³ d'une solution aqueuse saturée en chlorure d'ammonium, repris par 100 cm³ d'acétate d'éthyle et lavé par 100 cm³ d'eau puis par 100 cm³ d'une solution aqueuse saturée en chlorure de sodium. La phase organique est décantée, séchée sur sulfate de magnésium, filtrée et concentrée à sec sous pression réduite (2,7 kPa). Le résidu est chromatographié sur colonne de gel de silice (granulométrie 0,04-0,06 mm, diamètre 3 cm, hauteur 16 cm), en éluant sous une pression de 0,5 bar d'azote par un mélange de cyclohexane et d'acétate d'éthyle (70/30 en volumes) et en recueillant des fractions de 35 cm³. Les fractions 8 à 16 sont réunies puis concentrées à sec sous pression réduite (2,5 kPa). Le résidu est cristallisé dans 4 cm³ d'acétate d'éthyle. On obtient 0,6 g de méthyl-7 phényl-7 (méthoxy-2 phényl)-4 [(méthoxy-2 phényl)-acétyl]-2 perhydroisoindolol-4-(3aRS,4RS,7SR,7aRS) sous la forme de cristaux blancs fondant à 234°C.

La méthyl-7 phényl-7 (méthoxy-2 phényl)-acétyl-2 perhydroisoindolone-4-(3aRS,7SR,7aRS) peut être préparée de la façon suivante :

A une solution de 1,66 g d'acide méthoxy-2 phénylacétique dans 40 cm³ de dichlorométhane on ajoute à 4°C, 1,62 g de N,N' carbonyldiimidazole. Après 30 minutes d'agitation à température ambiante on additionne goutte à goutte une solution contenant 1,85 g de chlorhydrate de méthyl-7 phényl-7 perhydroisoindolone-4-(3aRS,7SR,7aRS) et 0,7 g de triéthylamine dans 30 cm³ de dichlorométhane. Après 2 heures d'agitation le mélange réactionnel est lavé deux fois avec 50 cm³ d'eau puis avec 50 cm³ d'une solution aqueuse saturée en chlorure de sodium. La phase organique est décantée, séchée sur sulfate de magnésium, filtrée et concentrée à sec sous pression réduite (2,7 kPa). Le résidu est chromatographié sur colonne de gel de silice (granulométrie 0,04-0,06 mm, diamètre 3 cm, hauteur 15 cm), en éluant sous une pression de 0,5 bar d'azote par un mélange de cyclohexane et d'acétate d'éthyle (80/20 en volumes) et en recueillant des fractions de 50 cm³. Les fractions 16 à 60 sont réunies puis concentrées à sec sous pression réduite (2,5 kPa). Le résidu est cristallisé dans 30 cm³ d'acétate d'éthyle. On obtient 1,3 g de méthyl-7 phényl-7 (méthoxy-2 phényl)-acétyl-2 perhydroisoindolone-4-(3aRS,7SR,7aRS) sous la forme de cristaux blancs fondant à 160°C.

Le chlorhydrate de méthyl-7 phényl-7 perhydroisoindolone-4-(3aRS,7SR,7aRS) peut être préparé de la façon suivante :

On dissout 4,27 g de méthyl-7 phényl-7 vinyloxycarbonyl-2 perhydroisoindolone-4-(3aRS,7SR,7aRS) dans 100 cm³ de tétrahydrofurane saturé en acide chlorhydrique. Après une heure d'agitation à température ambiante la solution est concentrée à sec sous pression réduite (2,7 kPa). Le résidu est repris par 100 cm³ d'éthanol absolu et chauffé une heure à 60°C. Après évaporation du solvant sous pression réduite (2,7 kPa) on obtient 3,71 g de chlorhydrate de méthyl-7 phényl-7 perhydroisoindolone-4-(3aRS,7SR,7aRS) sous la forme d'une huile jaune.

La méthyl-7 phényl-7 vinyloxycarbonyl-2 perhydroisoindolone-4(3aRS,7SR,7aRS) peut être préparée de la manière suivante :

A une solution de 9 g de benzyl-2 méthyl-7 phényl-7 perhydroisoindolone-4-(3aRS,7SR,7aRS) dans 100 cm³ de dichloro-1,2 éthane, on ajoute, à température ambiante, 2,84 cm³ de chloroformiate de vinyle et chauffe 2 heures à reflux. La solution est concentrée à sec sous pression réduite (2,7 kPa) et le résidu purifié par chromatographie sur colonne de gel de silice (granulométrie 0,04-0,06 mm, diamètre 4 cm, hauteur 23 cm), en éluant sous une pression de 0,5 bar d'azote par un mélange de cyclohexane et d'acétate d'éthyle (90/10 en volumes) et en recueillant des fractions de 100 cm³. Les fractions 17 à 34 sont réunies et concentrées à sec sous pression réduite (2,7 kPa). On obtient 4,27 g de méthyl-7 phényl-7 vinyloxycarbonyl-2 perhydroisoindolone-4-(3aRS,7SR,7aRS) sous la forme d'une huile jaune.

La benzyl-2 méthyl-7 phényl-7 perhydroisoindolone-4-(3aRS,7SR,7aRS) peut être préparée da la façon suivante :

A une solution de 14,5 g de méthyl-4 phényl-4 cyclohexène-2-one et de 24,45 cm³ de N-butoxyméthyl N-triméthylsilylméthyl benzylamine dans 65 cm³ de dichlorométhane, on ajoute, à une température de 25°C, 3 gouttes d'acide trifluoroacétique. Le mélange réactionnel est agité à cette température pendant 30 minutes puis chauffé à reflux pendant 2 heures. Après refroidissement on ajoute 10 g de carbonate de potassium, agite 15 minutes puis filtre la solution sur verre fritté et concentre à sec sous pression réduite (2,7 kPa). Le résidu est chromatographié sur colonne de gel de silice (granulométrie 0,04-0,06 mm, diamètre 5 cm, hauteur 39 cm), en éluant sous une pression de 0,5 bar d'azote par un mélange de cyclohexane et d'acétate d'éthyle (80/20 en volumes) et en recueillant des fractions de 100 cm³. Les fractions 8 à 30 sont réunies et concentrées à sec sous pression réduite (2,7 kPa). On obtient 9 g de benzyl-2 méthyl-7 phényl-7 perhydroisoindolone-4-(3aRS,7SR,7aRS) sous forme d'une huile jaune.

La N-butoxyméthyl N-triméthylsilylméthyl benzylamine peut être préparée selon la méthode de Y. Terao et coll., Chem. Pharm. Bull., 33, 2762 (1985).

La méthyl-4 phényl-4 cyclohexène-2-one peut être préparée selon la méthode de H.E. Zimmerman et G. Jones, J. Amer. Chem. Soc., 92(9), 2753,(1970).

### Exemple 16

A une solution de 0,52 g d'acide méthoxy-2 phénylacétique acétique dans 20 cm³ de dichlorométhane, refroidie à 0°C, on ajoute 0,5 g de N-N'-carbonyldiimidazole, on agite 45 minutes à 0°C, puis on ajoute 0,74 g de (méthoxy-2 phényl)-4 méthyl-5 perhydroisoindolol-4-(3aRS, 4RS, 5RS, 7aSR). On agite 18 heures à température ambiante puis on lave la phase organique par 2 fois 40 cm³ d'eau, puis par 40 cm³ d'une solution aqueuse saturée en chlorure de sodium, on sèche sur sulfate de magnésium et concentre à sec sous pression réduite (2,7 kPa). Le résidu est chromatographié sur colonne de gel de silice (granulométrie 0,04-0,06 mm, diamètre 2 cm, hauteur 15 cm), en éluant sous une pression de 0,5 bar d'azote par un mélange de cyclohexane et d'acétate d'éthyle (80/20 en volumes) et en recueillant des fractions de 25 cm³. Les fractions 10 à 20 sont réunies puis concentrées à sec sous pression réduite (2,7 kPa). Le résidu est cristallisé dans de l'acétonitrile. On obtient 0,5 g de (méthoxy-2 phényl)-4 (méthoxy-2 phénylacétyl)-2 méthyl-5 perhydroisoindolol-4-(3aRS,4RS, 5RS,7aSR) sous la forme de cristaux blancs fondant à 198°C.

Le (méthoxy-2 phényl)-4 méthyl-5 perhydroisoindolol-4-(3aRS,4RS, 5RS,7aSR) peut être préparé de la manière suivante :

A une solution de 1 g de benzyl-2 (méthoxy-2 phényl)-4 méthyl-5 perhydroisoindolol-4-(3aRS,4RS,5RS,7aSR) dans 15 cm³ d'éthanol anhydre, on ajoute 2 g d'hydroxyde de palladium sur charbon à 20 % ; puis le mélange réactionnel est hydrogéné, sous agitation, à une température de 60°C et sous pression atmosphérique. Après 1 heure de réaction le mélange réactionnel est filtré, puis concentré à sec sous pression réduite (2,7 kPa). On obtient 0,74 g de (méthoxy-2 phényl)-4 méthyl-5 perhydroisoindolol-4-(3aRS,4RS,5RS,7aSR) sous la forme d'une meringue jaune.

Spectre IR (KBr - bandes caractéristiques en cm⁻¹) : 3425, 3075-3025-3000, 2955, 2880, 2835, 1595-1500-1485, 1460, 1375, 1235,1025, 750.

Le benzyl-2 (méthoxy-2 phényl)-4 méthyl-5 perhydroisoindolol-4-(3aRS,4RS,5RS,7aSR) peut être préparé de la manière suivante :

A une suspension de 2,13 g de bromure de méthoxy-2 phénylmagnésium dans 15 cm³ de tétrahydrofurane, on ajoute goutte à goutte, à température ambiante, sous agitation, une solution de 0,7 g de benzyl-2 méthyl-5 perhydroisoindolone-4-(3aRS, 5RS, 7aSR) dans 15 cm³ de tétrahydrofurane. Le mélange réactionnel est agité à température ambiante pendant 48 heures, traité par 50 cm³ d'une solution aqueuse saturée en chlorure d'ammonium, repris par de l'acétate d'éthyle et lavé par une solution aqueuse saturée en chlorure de sodium. La phase organique est décantée, séchée sur sulfate de magnésium, filtrée et concentrée à sec sous pression réduite (2,7 kPa). Le résidu est chromatographié sur colonne de gel de silice Merck (granulométrie 0,04-0,06 mm, diamètre 2 cm, hauteur 15 cm), en éluant sous une pression de 0,5 bar d'azote par un mélange de cyclohexane et d'acétate d'éthyle (60/40 en volumes) et en recueillant des fractions de 25 cm³. Les fractions 10 à 20 sont réunies puis concentrées à sec sous pression réduite (2,5 kPa). On obtient 1 g de benzyl-2 (méthoxy-2 phényl)-4 méthyl-5 perhydroisoindolol-4-(3aRS,4RS,5Rs,7aSR) sous la forme d'une huile jaune.

La benzyl-2 méthyl-5 perhydroisoindolone-4-(3aRS,5RS,7aSR) peut être préparée de la manière suivante :

A une solution de 1 g de méthyl-6 cyclohexène-2-one dans 15 cm³ de dichlorométhane, on ajoute, à température ambiante, 3 gouttes d'acide trifluoroacétique, puis 3,6 cm³ de N-butoxyméthyl N-triméthylsilylméthyl benzylamine. Le mélange réactionnel est porté au reflux sous agitation pendant 3 heures. Après refroidissement, on ajoute du carbonate de potassium, filtre la solution sur verre fritté et concentre à sec sous pression réduite (2,7 kPa). Le résidu est chromatographié sur colonne de gel de silice (granulométrie 0,04-0,06 mm, diamètre 2 cm, hauteur 20 cm), en éluant sous une pression de 0,5 bar d'azote par un mélange de cyclohexane et d'acétate d'éthyle (60/40 en volumes) et en recueillant des fractions de 25 cm³. Les fractions 10 à 20 sont réunies et concentrées à sec sous pression réduite (2,7 kPa). On obtient 0,7 g de benzyl-2 méthyl-5 perhydroisoindolone-4-(3aRS,5RS,7aSR) sous forme d'une huile incolore.

La méthyl-6 cyclohexène-2-one peut être préparée selon la méthode de J.TSUJI et coll., Tet.Lett., 24, 1797 (1993).

### Exemple 17

En opérant selon le mode opératoire de l'exemple 16, à partir de 4,5 g d'acide (méthoxy-2 phényl)-2-propionique-(S) et de 5 g de (méthoxy-2 phényl) -4 méthyl-5 perhydroisoindolol-4-(3aRs,4RS,5Rs, 7aSR), on obtient, après purification sur colonne de gel de silice (granulométrie 0,04-0,06 mm, diamètre 5,4 cm, hauteur 35 cm, en éluant sous une pression de 0,5 bar d'azote par un mélange de cyclohexane et d'acétate d'éthyle (80/20 en volumes) et en recueillant des fractions de 75 cm³) 0,84g (fractions 32 à 40) de (méthoxy-2 phényl)-4 [(méthoxy-2 phényl)-2 propionyl-(S)]-2 méthyl-5 perhydroisoindolol-4-(3aR*,4R*,5R*,7aS*) (forme A) sous la forme d'une meringue blanche et 0,27 g (fractions 69 à 85) de (méthoxy-2 phényl)-4 [(méthoxy-2 phényl)-2 propionyl-(S)]-2 méthyl-5 perhydroisoindolol-4-(3aS*,4S*,5S*,7aR*) (forme B) sous la forme d'une meringue blanche.

Spectre RMN du proton de la forme A (DMSO-d₆) + CH₃COOD T=393°K) : 0,57 (d, 3H, CH₃) ; 1,3 (d, 3H, CH₃) ; 1,9-1,3 (m, 4H, 2 CH₂) ; 2,43 (m, 1H, CH) ; 2,52 (m, 1H, CH) ; 2,83 (m, 1H, CH) ; 3,6-3,1 (m, 4H, 2 CH₂) ; 3,75 (s, 3H, OCH₃) ; 3,82 (s, 3H, OCH₃) ; 4,14 (q, 1H, CH) ; 7,3-6,8 (m, 8H, aromatiques).

Spectre IR de la forme A (KBr-bandes caractéristiques en cm⁻¹) ; 3576, 3436, 3105-3068, 2959-2929-2875, 2836, 1636, 1598-1580-1491, 1457-1435, 1371, 1239, 1062, 1028, 755.

Spectre RMN du proton de la forme B (DMSO-d₆) + CH₃COOD T=393°K : 0,6 (d, 3H, CH₃) ; 1,31 (d, 3H, CH₃) ; 1,85-1,3 (m, 4H, 2 CH₂) ; 2,48 (m, 1H, CH) ; 2,5 (m, 1H, CH) ; 2,8 (t, 1H, CH) ; 3,55-2,9 (m, 4H, 2 CH₂) ; 3,75 (s, 3H, OCH₃) ; 3,85 (s, 3H, OCH₃) ; 4,18 (q, 1H, CH) ; 7,6-6,85 (m, 8H,aromatiques).

Spectre IR de la forme B (KBr-bandes caractéristiques en cm⁻¹) : 3425, 3106-3068, 2958-2930-2876, 2836, 1626, 1599-1580-1492, 1461-1436, 1378-1370, 1239, 1062, 1028, 755.

### Exemple 18

A une solution de 3 g d'hydroxyméthyl-6 (méthoxy-2 phényl)-4 perhydroisoindolol-4-(3aRS,4RS,6SR,7aSR) et de 1,8 g d'acide (méthoxy-2 phényl) acétique dans 60 cm³ de dichlorométhane, on ajoute 2,3 g de chlorhydrate de (diméthylamino-3 propyl)-1 éthyl-3 carbodiimide. Après 20 heures d'agitation à 20°C, le mélange réactionnel est lavé par 30 cm³ d'eau, séché sur sulfate de magnésium, et concentré à sec sous pression réduite (2,7 kPa). Le résidu est chromatographié sur une colonne de gel de silice (granulométrie 0,06-0,20 mm, hauteur 40 cm) dans de l'acétate d'éthyle puis dans un mélange d'acétate d'éthyle et de méthanol (95/5 en volumes). On obtient, après séchage sous pression réduite, un solide blanc qui, après recristallisation dans un mélange d'éther éthylique et d'acétonitrile, donne 2,1 g d'hydroxyméthyl-6 [(méthoxy-2)phényl]-4 [(méthoxy-2 phényl)acétyl]-2 perhydroisoindolol-4-(3aRS,4RS,6SR,7aSR), sous la forme de cristaux blancs. PF = 162°C.

Spectre de RMN du proton (DMSO d₆ + CH₃COOD, 250 MHz, T=403°K), δ (ppm): 1,42 et 1,85 (m et d, 2x1H, CH-CH₂-CH), 1,69 et 2,04 (d et t, 2xlH, C-CH₂-CH), 2,17 (m, 1H, CH-CH₂OH), 2,57 (m, 1H, CH₂-CH-CH), 2,85 (t, 1H, CH-CH-C), 3,17 et 3,40 (m et d, 2x1H, -N-CH₂-CH), 3,35 (d, 2H, O-CH₂-CH), 3,47 (s, 2H, CO-CH₂-Ph). 3,49 (m, 2H, N-CH₂-CH), 3,75 (s, 3H, OCH₃), 3,82 (s, 3H, OCH₃), 6,80-7,55 (8H, aromatiques).

Spectre infrarouge (KBr), bandes caractéristiques (cm⁻¹) : 3430, 3075, 3000-2875, 2835, 1620, 1605, 1495, 1485, 1460, 1435, 1245, 1055, 1030, 750.

L'hydroxyméthyl-6 (méthoxy-2 phényl)-4 perhydroisoindolol-4-(3aRS,4RS,6SR,7aSR) peut être obtenu de la manière suivante :

Une suspension de 3,1 g de benzyl-2 hydroxyméthyl-6 (méthoxy-2 phényl)-4 perhydroisoindolol-4-(3aRS,4RS,6SR,7aSR) et de 0,5 g d'hydroxyde de palladium à 20 % sur noir de carbone dans 75 cm³ d'éthanol, est hydrogénée à pression atmosphérique pendant 3 heures à 60°C. Le mélange réactionnel est ensuite filtré, évaporé à sec sous pression réduite (2,7 kPa). On obtient 3,0 g d'hydroxyméthyl-6 (méthoxy-2 phényl)-4 perhydroisoindolol-4-(3aRS,4RS,6SR,7aSR), sous la forme d'une meringue blanche.

Spectre de RMN du proton (DMSO d₆ + CH₃COOD, 250 MHz), δ (ppm) : 1,41 et 1,90 (m et d, 2x1H, CH-CH₂-CH), 1,60 et 2,22 (d et t, 2x1H, C-CH₂-CH), 2,10 (m, 1H, CH-CH₂OH), 2,59 (m, 1H, CH₂-CH-CH), 2,95 (m, 1H, CH-CH-C), 2,95 (m, 2H, N-CH₂-CH), 3,20 et 3,32 (m, 2H, N-CH₂-CH), 3,30 (s, 2H, O-CH₂-CH), 3,81 (s, 3H, OCH₃), 7,00 à 7,64 (m, 4H, aromatique).

Spectre infrarouge (KBr), bandes caractéristiques (cm⁻¹) : 3420, 3075, 2920, 2880, 1595, 1575, 1485, 1460, 1235, 1055, 1030, 755.

Le benzyl-2 hydroxyméthyl-6 (méthoxy-2 phényl)-4 perhydroisoindolol-4-(3aRS,4RS,6SR,7aSR) peut être préparé de la façon suivante :

A une suspension de bromure de méthoxy-2 phényl magnésium (préparée à partir de 24,9 cm³ de bromo-2 anisole et de 4,86 g de magnésium) dans 200 cm³ de tétrahydrofuranne anhydre, on ajoute une solution de 8,0 g d'acétoxyméthyl-6 benzyl-2 perhydroisoindolone-4-(3aRS,6SR,7aSR) dans 60 cm³ de tétrahydrofurane anhydre. Le mélange réactionnel est agité pendant 20 heures à 20°C puis refroidi à +5°C et traité par 120cm³ d'une solution aqueuse saturée de chlorure d'ammonium et 100 cm³ d'acétate d'éthyle. Après filtration du mélange, la phase organique est lavée par de la saumure puis séchée sur sulfate de magnésium et concentrée à sec sous pression réduite (2,7 kPa). Le résidu est chromatographié sur une colonne de gel de silice (granulométrie 0,06-0,200 mm, hauteur 40 cm) dans un mélange de dichloro-1,2 éthane et de méthanol (95/5 en volumes). On obtient, après recristallisation dans l'éther éthylique, 3,2 g de benzyl-2 hydroxyméthyl-6 (méthoxy-2 phényl)-4 perhydroisoindolol-4-(3aRS,4RS,6SR,7aSR), sous la forme d'un solide blanc fondant à 110°C.

Spectre RMN du proton (CDCl₃ + CH₃COOD, 250 MHz, T=333°K), δ (ppm) : 1,40 et 1,85 (m et d, 2x1H, CH-CH₂-CH), 1,75 et 2,05 (t et d, 2x1H, C-CH₂-CH), 2,28 (m, 1H, CH₂-CH-CH₂), 2,82 (m, 1H, CH₂-CH-CH), 3,03 (t, 1H, CH-CH-C), 3,11 et 3,50 (d, 2x1H, N-CH₂-CH), 3,38 et 3,52 (t, 2x1H, N-CH₂-CH), 3,50 (m, 2H, O-CH₂-CH), 3,86 (s, 3H, OCH₃), 4,28 et 4,40 (d, 2x1H, N-CH₂Ph), 6,85-7,45 (m, 9H, aromatiques),

Spectre infrarouge (KBr), bandes caractéristiques (cm⁻¹) : 3560, 3420, 3060, 3025, 3005, 2910, 2850, 2800, 1590, 1575, 1495, 1480, 1230, 1055, 1030, 755, 735, 700.

L'acétoxyméthyl-6 benzyl-2 perhydroisoindolone-4-(3aRS,6SR,7aSR) peut être préparée de la façon suivante :

A une solution de 6,5 g d'acétoxyméthyl-5 cyclohexène-2 one-1 [J. Am. Chem. Soc., 110, 2919 (1988)1 et de 14 g de N-butoxyméthyl N-triméthylsilylméthyl benzylamine dans 60 cm³ de dichlorométhane sec, on ajoute 2 gouttes d'acide trifluoroacétique. Le mélange réactionnel atteignant le reflux est maintenu à la même température pendant 30 minutes puis laissé à 20°C pendant une heure. Après ajout de 1,0 g de carbonate de potassium, la suspension obtenue est filtrée, le filtrat concentré à sec sous pression réduite (2,7 kPa). Le résidu huileux obtenu est chromatographié sur une colonne de gel de silice (granulométrie 0,06-0,2 mm, hauteur 42 cm) dans un mélange de cyclohexane et d'acétate d'éthyle (40/60 en volumes) puis dans l'acétate d'éthyle. On obtient 8,2 g d'acétoxyméthyl-6 benzyl-2 perhydroisoindolone-4-(3aRS,6SR,7aSR), sous la forme d'une huile jaunâtre.

Spectre RMN du proton (CDCl₃ + CH₃COOD, 250 MHz), δ (ppm) : 1,82 (m, 2H, CH-CH₂-CH), 2,03 (s, 3H, CH₃CO), 2,2 (m, 1H, CH), 2,27 et 2,42 (t et dd: 2x1H, -COCH₂-), 2,40 et 3,60 (t, 2x1H, N-CH₂-CH), 3,10 (td, 1H, CH), 3,22 (m, 1H, CH), 3,60 et 3,75 (m, 2x1H, N-CH₂-CH), 4,00 (m, 2H, O-CH₂-), 4,10 et 4,23 (d, 2x1H, N-CH₂-Ph), 7,30-7,45 (m, 5H, aromatiques),

Spectre infrarouge (CCl₄), bandes caractéristiques (cm⁻¹) : 3105, 3090, 3065, 3030, 2920, 2795, 1745, 1712, 1605, 1585, 1495, 1455, 1425, 1365, 1240, 1035, 700.

### Exemple 19

En opérant de façon identique à l'exemple 16, mais à partir de 1,46 g d'(hydroxyméthyl)-7 (méthoxy-2 phényl)-4 méthyl-7 perhydroisoindolol-4-(3aRS,4RS,7SR,7aRS) et 0,83 g d'acide (méthoxy-2 phényl) acétique. Après chromatographie sur une colonne de gel de silice (0,060-0,200 mm, diamètre de 2,5 cm, hauteur de 20 cm) en éluant sous une pression de 0,5 bar par un mélange de dichlorométhane et de méthanol [91/9 en volumes] et en recueillant des fractions de 60 cm³ les fractions 2 à 5 sont concentrées, recristallisées dans l'acétonitrile et l'on obtient après séchage à 40°C sous 15 Pa 1,35 g d'(hydroxyméthyl)-7 (méthoxy-2 phényl)-4 [(méthoxy-2 phényl) acétyl]-2 méthyl-7 perhydroisoindolol-4- (3aRS,4RS,7SR,7aRS) sous forme d'un solide blanc fondant à 205°C.

L'(hydroxyméthyl)-7 (méthoxy-2 phényl)-4 méthyl-7 perhydroisoindolol-4-(3aRS,4RS,7SR,7aRS) peut être préparé de la manière suivante :

Un mélange de 3,5 g de benzyl-2 (hydroxyméthyl)-7 (méthoxy-2 phényl)-4 méthyl-7 perhydroisoindolol-4-(3aRS,4RS,7SR,7aRS), 2,0 g d'hydroxyde de palladium à 20 % sur noir de carbone et 75 cm³ d'éthanol est chauffé à 50°C. Après bullage d'hydrogène pendant deux heures, le mélange réactionnel est refroidi à température ambiante et purgé à l'aide d'un courant d'azote, filtré et concentré sous pression réduite (2,7 kPa). On obtient 1,5 g de (hydroxyméthyl)-7 (méthoxy-2 phényl)-4 méthyl-7 perhydroisoindolol-4- (3aRS,4RS,7SR, 7aRS) sous la forme d'un solide blanc.

Spectre de RMN du proton (DMSO d₆ + CH₃COOD, 250 MHz), δ (ppm): 1,05 et 1,80 (2m, 2x1H, C-CH₂-CH₂), 1,20 (s, 3H, CH₃), 1,45 et 2,75 (d et td, 2x1H, C-CH₂-CH₂), 2,20 (m, 1H, CH-CH₂N ), 2,80 et 2,90 (t et dd, 2x1H, CH₂-N ), 3,05 (t, 1H : C-CH-CH₂N ), 3,20 (s, 2H, CH₂OH ), 3,20 et 3,40 (t et d, 2x1H, CH₂-N ), 3,84 (s, 3H, OCH₃), 6,90-7,60 (4H aromatiques).

Le benzyl-2 (hydroxyméthyl)-7 (méthoxy-2 phényl)-4 méthyl-7 perhydroisoindolol-4-(3aRS,4RS,7SR,7aRS) peut être obtenu de la manière suivante :

A une suspension de 1,52 g d'hydrure de lithium et d'aluminium dans 60 cm³ de tétrahydrofurane sont additionnés 8,7 g de benzyl-2 éthano-4,7 (méthoxy-2 phényl) -4 méthyl-7 perhydropyrano[3,4-c]pyrrolone-6 dissous dans 60 cm³ de tétrahydrofurane. Après 2 heures à température ambiante, on ajoute goutte à goutte 10 cm³ d'eau puis 200 cm³ d'acétate d'éthyle. Le mélange obtenu est séché sur sulfate de magnésium, filtré et concentré à sec sous pression réduite (2,7 kPa). On obtient 7,0 g de benzyl-2 (hydroxyméthyl)-7 (méthoxy-2 phényl)-4 méthyl-7 perhydroisoindolol4--(3aRS,4RS, 7SR,7aRS) sous la forme d'un solide blanc.

Spectre de RMN du proton (CDCl₃, 250 MHz), δ (ppm) : 1,00 et 1,80 (2m, 2x1H,: C-CH₂-CH₂), 1,20 (s, 3H, CH₃), 1,30 et 2,60 (m, 2x1H, C-CH₂-CH₂), 2,25 (m, 1H, CH-CH₂N ), 2,30 et 2,65 (m, 2x1H, CH₂-N ), 2,40 et 3,00 (m, 2x1H, CH₂-N), 3,00 (m, 1H, CH-CH₂N ), 3,30 (m, 2H, CH₂OH), 3,55 et 3,70 (d, 2x1H, N-CH₂Ph ), 3,80 (s, 3H, OCH₃), 6,80-8,00 (9H aromatiques).

Le benzyl-2 éthano-4,7 (méthoxy-2 phényl)-4 méthyl-7 perhydropyrano[3,4-c]pyrrolone-6 peut être préparé de la manière suivante :

A une suspension de bromure de méthoxy-2 phénylmagnésium dans 200 cm³ de tétrahydrofurane, préparée à partir de 18 cm³ de bromo-2 anisole et 3,8 g de magnésium, on ajoute goutte à goutte à température ambiante 31 g d'(allyloxycarbonyl)-7 benzyl-2 méthyl-7 perhydroisoindolone-4-(3aRS,7SR,7aRS) dissous dans 100 cm³ de tétrahydrofurane. Après une heure à température ambiante, 200 cm³ d'une solution aqueuse à 26 % de chlorure d'ammonium est coulée en trente minutes, le mélange réactionnel est extrait à l'acétate d'éthyle, la phase organique est lavée à l'eau (2 x 150 cm³) puis séchée sur sulfate de magnésium, filtrée et concentré sous pression réduite (2,7 kPa). Le résidu obtenu est chromatographié sur une colonne de gel de silice (0,060-0,200 mm, diamètre de 8 cm, hauteur de 60 cm) en éluant sous une pression de 0,5 bar par un mélange d'acétate d'éthyle et de cyclohexane [en volumes, 20/80 (4 dm³) puis 30/70 (4 dm³) et 40/60 (4 dm³)] et en recueillant des fractions de 500 cm³. Les fractions 14 à 22 sont réunies, concentrées à sec sous pression réduite (2,7 kPa). On obtient 13,1 g de benzyl-2 éthano-4,7 (méthoxy-2 phényl)-4 méthyl-7 perhydropyrano[3,4-c]pyrrolone-6.

Spectre de RMN du proton (CDCl₃+ CH₃COOD, 250 MHz), δ (ppm) : 1,15 (s, 3H, CH₃), 1,80, 1,95 et 2,70 (4H, CH₂-CH₂), 2,30 et 3,65 (t et dd, 2x1H, CH-CH₂-N ), 2,40 et 3,25 (t, 2H: CH-CH₂-N ), 2,90 (m, 1H, CH-CH₂N ), 3,80 (s, 3H, OCH₃), 3,90 (m, 1H, CH-CH₂N ), 3,92 et 4,13 (d, 2x1H, O-CH₂-CH), 6,80-7,60 (9H aromatiques).

L'allyloxycarbonyl-7 benzyl-2 méthyl-7 perhydroisoindolone-4-(3aRS,7SR,7aRS) peut être obtenue de la manière suivante :

A une solution de 35 g d'allyloxycarbonyl-4 méthyl-4 cyclohexèn-2-one et de 65 cm³ de N-butoxyméthyl N-triméthylsilylméthyl benzylamine dans 350 cm³ de dichlorométhane, on ajoute 10 gouttes d'acide trifluoroacétique. Le mélange réactionnel atteint le reflux après 15 minutes, puis revient lentement (3 heures) à 20°C. 10 g de carbonate de potassium sont additionnés au mélange qui est agité pendant 30 minutes, filtré et concentré sous pression réduite (2,7 kPa). Le résidu obtenu est chromatographié sur une colonne de gel de silice (0,060-0,200 mm, diamètre de 9 cm, hauteur de 60 cm) en éluant sous une pression de 0,5 bar par un mélange d'acétate d'éthyle et de cyclohexane [en volumes, 30/70 (6 dm³) puis 40/60 (6 dm³) et 50/50 (6 dm³)] et en recueillant des fractions de 1000 cm³. A partir des fractions 9 à 15 réunies et concentrées à sec sous pression réduite (2,7 kPa), on obtient 31 g de allyloxycarbonyl-7 benzyl-2 méthyl-7 perhydroisoindolone-4-(3aRS,7SR,7aRS) sous forme d'huile.

Spectre de RMN du proton (CDCl₃+ CH₃COOD, 250 MHz), δ (ppm) : 1,42 (s, 3H, CH₃), entre 2,00 et 3,80 (10H, 2 fois CH-CH₂N , N-CH₂-CH et C-CH₂-CH₂ ), 4,50 (d, 2H, O-CH₂-CH), 5,20 et 5,30 (dd, 2x1H, CH=CH₂), 5,85 (m, 1H, CH=CH₂), 7,15-7,35 (5H aromatiques).

La N-butoxyméthyl N-triméthylsilylméthyl benzylamine peut être préparée selon la méthode de Y.Terao et coll., Chem.Pharm.Bull., 33, 2762 (1985).

L'allyloxycarbonyl-4 méthyl-5 cyclohexèn-2-one peut être préparée par analogie avec la méthode décrite par P.E. Vorndam, J.Org.Chem., 55, 3693 (1990).

### Exemple 20

A une suspension refroidie à +5°C de 0,5 g d'hydroxy-7 (méthoxy-2 phényl)-7 [(méthoxy-2 phényl)acétyl]-2 perhydroisoindolone-4-(3aRS,7RS,7aSR) dans 50 cm³ de méthanol on ajoute 0,027 g de borohydrure de sodium. Après 2 heures d'agitation à 5°C, la solution réactionnelle est additionnée de 0,2 cm³ d'acide chlorhydrique 1N puis concentrée à sec sous pression réduite. Le résidu est repris par 50 cm³ d'eau et 70 cm³ de dichlorométhane. Après agitation, la suspension obtenue est filtrée, la phase organique du filtrat est lavée à l'eau puis séchée sur sulfate de magnésium et concentrée à sec sous pression réduite (2,7 kPa). On obtient 0,42 g de (méthoxy-2 phényl)-4 [(méthoxy-2 phényl)acétyl]-2 perhydroisoindoldiol-4,7-(3aRS,4RS,7SR,7aSR) sous la forme d'une meringue blanche.

Spectre RMN du proton (DMSO d₆ + CH₃COOD) : 1,60 (mt, J = 12,5, 8 et 4, 1H, H en 5); 1,70 (mt, J =14 et 8, 1H, H en 5); 2,05 (mt, J =14, 12,5 et 3, 1H, H en 6); 2,45 (mt, J = 14 et 4, 1H, H en 5); 2,7 (mt, 1H, H en 7a); 3,0 (dd large, J = 7, 1H, H en 3a); 3,20 (mf, 1H, H en 3); 3,45 (dd, J = 12 et 1,5, 1H, H en 3);3,58 (mt, 3H, H en 1 et CH₂CO); 3,75 (mf, 1H, H en 1); 3,8 (S, 3H, OCH₃); 3,9 (s, 3H, OCH₃); 4,05 (mt, 1H, H en 7); 6,95-7,60 (m 8H, aromatiques).

Spectre infrarouge (CH₂Cl₂), bandes caractéristiques (cm⁻¹) : 3600 + 3530, 2975, 2880, 2835, 1630, 1600 + 1580 + 1495, 1465, 1440, 1245, 1060, 1030.

L'hydroxy-7 (méthoxy-2 phényl)-7 [(méthoxy-2 phényl)acétyl]-2 perhydroisoindolone-4-(3aRS,7RS,7aSR) peut être préparée de la manière suivante :

A une solution de 2,8 g d'hydroxy-7 (méthoxy-2 phényl)-7 perhydroisoindolone-4-(3aRS,7RS,7aSR) et de 1,3 cm³ de triéthylamine dans 60 cm³ de dichlorométhane anhydre, on ajoute 1,55 g d'acide (méthoxy-2 phényl) acétique, 0,03 g d'hydrate d'hydroxybenzotriazole et 1,96 g de chlorhydrate de (diméthylaminopropyl-3)-1 éthylcarbodiimide-3. Après 20 heures d'agitation à 20°C, le mélange réactionnel est lavé par 100 cm³ d'eau, séché sur sulfate de magnésium, puis concentré à sec sous pression réduite (2,7 kPa). Le résidu obtenu est recristallisé dans de l'éther éthylique, les cristaux sont essorés puis séchés sous pression réduite (2,7 kPa). on obtient 3,1 g d'hydroxy-7 (méthoxy-2 phényl)-7 [(méthoxy-2 phényl)acétyl]-2 perhydroisoindolone-4-(3aRS,7RS,7aSR) sous la forme d'un solide blanc fondant à 184°C.

Le chlorhydrate d'hydroxy-7 (méthoxy-2 phényl)-7 perhydroisoindolone-4-(3aRS,7RS,7aSR) peut être préparé par hydrogénation d'une suspension de 3,3 g de benzyl-2 hydroxy-7 (méthoxy-2 phényl)-7 perhydroisoindolone-4-(3aRS,7RS,7aSR) dans 60 cm³ de méthanol et 10 cm³ d'acide chlorhydrique 1N à pression atmosphérique pendant 24 heures à 20°C en présence de 0,6 g d'hydroxyde de palladium à 10 % sur noir de carbone. Le mélange réactionnel est filtré, concentré à sec sous pression réduite (2,7 kPa). On obtient 2,8 g de chlorhydrate d'hydroxy-7 (méthoxy-2 phényl)-7 perhydroisoindolone-4-(3aRS,7RS,7aSR), sous forme de meringue.

Spectre RMN du proton (DMSO, d₆ + CH₃COOD) : 2,90 (d large, J = 8, 2H en 1); 3,35 (mt, 3H, 2H en 3 et H en 3a ou H en 7a); 3,8 (mt, J = 8 et 10, 1H, H en 3a ou H en 7a); 3,9 (S, 3H, OCH₃); 7,0-7,55 (m, 4H, aromatiques).

Spectre infrarouge (KBr), bandes caractéristiques (cm⁻¹): 3400, 3000 - 2250, 1600 + 1580 + 1490, 1455 + 1440, 1240, 1055, 1025, 795 + 760.

La benzyl-2 hydroxy-7 (méthoxy-2 phényl)-7 perhydroisoindolone-4-(3aRS,7RS,7aSR) peut être préparée de la façon suivante :

A une solution de 7 g de benzyl-2 diméthoxy-7,7 (méthoxy-2 phényl)-4 perhydroisoindolol-4-(3aRS,4RS,7aSR) dans 70 cm³ de dichlorométhane sec, on ajoute 6,5 cm³ de triéthylamine et 6,5 cm³ d'acide trifluoroacétique. Après 3 heures d'agitation à température ambiante, le mélange réactionnel, refroidi à +5°C est alcalinisé par 50 cm³ d'hydroxyde de sodium 1N. La phase organique est séparée, lavée par de l'eau, séchée sur sulfate de magnésium, puis concentré à sec sous pression réduite (2,7 kPa). L'huile obtenue est cristallisée dans de l'oxyde de diisopropyle. Le solide, lavé par de l'éther de pétrole, est ensuite essoré, seché sous pression réduite à 40°C. On obtient 3,4 g de benzyl-2 hydroxy-7 (méthoxy-2 phényl)-7 perhydroisoindolone-4-(3aRS,7RS,7aSR), sous la forme d'un solide de couleur crème. PF = 96°C.

Le benzyl-2 diméthoxy-7,7 (méthoxy-2 phényl)-4 perhydroisoindolol-4-(3aRS,4RS,7aSR) peut être préparé selon le mode opératoire suivant :

A une suspension de bromure de méthoxy-2 phényl magnésium (préparée à partir de 19,3 cm³ de bromo-2 anisole et de 3,7 g de magnésium) dans 30 cm³ d'éther éthylique sec, on ajoute en 30 minutes une solution de 18,1 g de benzyl-2 diméthoxy-7,7 perhydroisoindolone-4-(3aRS,7aSR) dans 100 cm³ d'éther éthylique. Le mélange réactionnel est ensuite porté au reflux pendant une heure, puis dilué par 50 cm³ de tétrahydrofurane. Après 3 heures de reflux et 20 heures à 20°C, le mélange est refroidi à +5°C, traité par 100 cm³ d'une solution aqueuse saturée en chlorure d'ammonium, extrait par 200 cm³ d'acétate d'éthyle.

La phase organique est lavée par de l'eau, séchée sur sulfate de magnésium et concentrée à sec sous pression réduite. Le résidu est purifié par chromatographie sur colonne de gel de silice (granulométrie 0,06 - 0,20 mm) dans un mélange de cyclohexane et d'acétate d'éthyle (80/20 en volumes). On obtient, après séchage du produit sous pression réduite (15Pa), 7 g de benzyl-2 diméthoxy-7,7 (méthoxy-2 phényl)-4 perhydroisoindolol-4-(3aRS,4RS,7aSR), sous la forme d'une huile.

Spectre RMN du proton (C₆D₆): 1,80 (mt, 2H, H en 5 et H en 6); 2,10 (dd, J = 9 et 5, 1H, H en 1); 2,30 (dd, J = 11,5 et 10, 1H, H en 3); 2,5 (mt, J = 14 et 4, 1H, H en 5); 2,7 à 3,0 (mt, 3H, H en 6 et H en 1 et H en 3a ou en 7a); 3,10 (s, 3H, OCH₃); 3,20 (s, 3H, OCH₃); 3,25 (mt, 2H, H en 3 et N-CH₂-Ph); 3,40 (mt, 2H, H en 7a et N-CH₂-Ph); 3,50 (s, 3H, OCH₃); 6,7-8,6 (m, 9H, aromatiques).

Spectre infrarouge (KBr), bandes caractéristiques (cm⁻¹): 3090 + 3070 + 3030, 2930, 2850, 2830, 2810 + 2730, 1600 + 1580 + 1485, 1370, 1465 + 1435, 1235, 1060, 1035, 700.

Le benzyl-2 diméthoxy-7,7 perhydroisoindolone-4-(3aRS,7aSR) peut être préparé par hydrogénation d'une suspension de 19 g de benzyl-2 diméthoxy-7,7 hexahydro-2,3,3a,4,7,7a-1H-isoindolone-4-(3aRS,7aSR) dans 200 cm³ d'éthanol à pression atmosphérique pendant 6 heures à 20°C en présence de Nickel de Raney (4 cm³ de la suspension aqueuse commerciale à 50 % filtrée, lavée 3 fois par 20 cm³ d'éthanol). Le mélange réactionnel est filtré, concentré à sec sous pression réduite (2,7 kPa). On obtient 18,1 g de benzyl-2 diméthoxy-7,7 perhydroisoindolone-4-(3aRS,7aSR), sous la forme d'une huile brun-clair.

Spectre RMN du proton (CDCl₃) : 3,18 (S, 3H, OCH₃); 3,2 (S, 3H, OCH₃); 3,6 (ab, J = 12,5, 2H, N-CH₂-Ph); 7,30 (mt, 5H, phényle).

Spectre infrarouge (CCl₄), bandes caractéristiques (cm⁻¹) : 3090 + 3070 + 3030, 2960, 2930, 2870, 2835, 2800 + 2730, 1740, 1715, 1495, 1470, 1455, 1245, 1030, 700.

Le benzyl-2 diméthoxy-7,7 hexahydro-2,3,3a,4,7,7a-1H-isoindolone-4-(3aRS,7aSR) peut être préparé de la façon suivante :

A une solution de 22,9 g de diméthoxy-4,4 cyclohexadiène-2,5 one-1 [J. Org. Chem., 52, 2763 (1987)] et de 46 cm³ de N-butoxyméthyl N-triméthylsilylméthyl benzylamine dans 150 cm³ de dichlorométhane sec, on ajoute goutte à goutte 3 cm³ d'acide trifluoroacétique. Le mélange réactionnel atteignant le reflux est laissé revenir à température ambiante, puis agité pendant pendant une heure. Après ajout de 5 g de carbonate de potassium, la suspension obtenue est filtrée, le filtrat concentré à sec sous pression réduite (2,7 kPa). Le résidu obtenu est purifié par chromatographie sur une colonne de gel de silice (granulométrie 0,06 - 0,20 mm), en éluant par un mélange de cyclohexane et d'acétate d'éthyle (90/10 puis 80/20 en volumes). On obtient 19 g de benzyl-2 diméthoxy-7,7 hexahydro-2,3,3a,4,7,7a-1H-isoindolone-4-(3aRS,7aSR), sous forme d'huile.

Spectre RMN du proton (DMSO, d₆): 2,8 (mt, J = 6,5 et 1,5, 1H, H en 7a ou en 3a); 3,10 (mt, 1H, H en 7a ou en 3a); 3,17 (S, 3H, OCH₃); 3,2 (S, 3H, OCH₃); 3,6 (ab, J = 13,5, 2H, NCH₂Ph); 6,18 (d, J = 9, 1H, H en 5); 6,9 (dd, J = 9 et 2,5, 1H, H en 6); 7,3 (mt, 5H, aromatiques)

Spectre infrarouge (CCl₄), bandes caractéristiques (cm⁻¹) : 3095 + 3070 + 3030, 2930, 2830, 2800 + 2740, 1690, 1645, 1495, 1455, 1380, 1235, 1045, 700.

### Exemple 21

A une suspension refroidie à -70°C, sous atmosphère d'argon, de 0,5 g d'hydroxy-7 (méthoxy-2 phényl) -7 [(méthoxy-2 phényl)acétyl]-2 perhydroisoindolone-4-(3aRS,7RS,7aSR) on ajoute lentement 4 cm³ d'une solution de méthyllithium 1,6 M dans l'ether éthylique. Après retour à température ambiante et agitation pendant 3 heures, le mélange réactionnel est refroidi à +5°C puis additionné de 25 cm³ d'une solution aqueuse saturée de chlorure d'ammonium et 20 cm³ d'acétate d'éthyle. La phase organique est séchée sur sulfate de magnésium puis concentrée à sec sous pression réduite (2,7 kPa). Le résidu est chromatographié sur colonne de gel de silice (granulométrie 0,06-0,20 mm) dans de l'acétate d'éthyle puis dans un mélange d'acétate d'éthyle et de méthanol (90/10 en volumes). On obtient, après séchage sous pression réduite et recristallisation dans de l'acétonitrile 0,1 g de (méthoxy-2 phényl)-4 [(méthoxy-2 phényl)acétyl]-2 méthyl-7 perhydroisoindoldiol-4,7-(3aRS,4RS,7aSR), sous la forme de cristaux blancs fondant à 180°C.

### Exemple 22

A une solution de 0,81 g de diméthyl-7,7 (méthoxy-2 phényl)-4 [(méthoxy-2 phényl)-acétyl]-2 hexahydro-2,3,3a,4,7,7a-(1H)-isoindolol-4-(3aRS,4RS,7aRS) dans 6 cm³ de pyridine on ajoute 0,5 g de tétroxyde d'osmium en solution dans 5 cm³ de pyridine. Le mélange réactionnel est agité à température ambiante pendant 48 heures, puis on ajoute une solution de 0,9 g de bisulfite de sodium dans 15 cm³ d'eau, puis 10 cm³ de pyridine; on décante, lave trois fois par 10 cm³ d'eau, puis par 10 cm³ d'une solution aqueuse saturée en chlorure de sodium. La phase organique est décantée, séchée sur sulfate de magnésium, filtrée et concentrée à sec sous pression réduite (2,7 kPa). Le résidu est chromatographié sur colonne de gel de silice (granulométrie 0,04-0,06 mm, diamètre 2 cm, hauteur 20 cm), en éluant sous une pression de 0,5 bar d'azote par un mélange de dichloro-1,2 éthane et de méthanol (80/20 en volumes) et en recueillant des fractions de 25 cm³. Les fractions 5 à 10 sont réunies puis concentrées à sec sous pression réduite (2,5 kPa). Le résidu est trituré dans de l'oxyde de diisopropyle On obtient 0,169 g de diméthyl-7,7 (méthoxy-2 phényl)-4 [(méthoxy-2 phényl)-acétyl]-2 perhydroisoindoletriol-4,5,6-(3aRS,4RS,7aRS) sous la forme d'une meringue blanche.

Spectre RMN du proton (DMSO d₆, deux rotamères à température ambiante :

1,2 (s,6H, 2 CH₃); 2,23 (m, 1H, CH); 3,2 à 3,7 (m, 8H, CHOH + 2 CH₂N + CH₂CO); 3,8 (s, 3H, OCH₃); 3,9 (s, 3H, OCH₃); 4,74 (d, 1H, CHOH); 6,9-7,63 (m, 8H, aromatiques).

Spectre IR (KBr - bandes caractéristiques en cm⁻¹) : 3375, 3075, 2960, 2910, 2880, 2835, 1620, 1600-1495-1485, 1465-1440, 1250, 1085-1055, 1030, 755.

A une suspension de 22,73 g de bromure de méthoxy-2 phénylmagnésium dans 70 cm³ de tétrahydrofurane refroidie à 15°C, on ajoute goutte à goutte sous agitation, une solution de 6,77 g de diméthyl-7,7 [(méthoxy-2 phényl)-acétyl]-2 hexahydro-2,3,3a,4,7,7a-1(H)-isoindolone-4-(3aRS,7aRS) dans 10 cm³ de tétrahydrofurane, puis 5,3 g de chlorure de cérium anhydre. Le mélange réactionnel est agité à température ambiante pendant 18 heures, traité par 80 cm³ d'une solution aqueuse saturée en chlorure d'ammonium, repris par 100 cm³ d'acétate d'éthyle et lavé par 100 cm³ d'une solution aqueuse saturée en chlorure de sodium. La phase organique est décantée, séchée sur sulfate de magnésium, filtrée et concentrée à sec sous pression réduite (2,7 kPa). Le résidu est chromatographié sur colonne de gel de silice (granulométrie 0,04-0,06 mm, diamètre 5,5 cm, hauteur 30 cm), en éluant sous une pression de 0,5 bar d'azote par un mélange de cyclohexane et d'acétate d'éthyle (50/50 en volumes) et en recueillant des fractions de 75 cm³. Les fractions 17 à 40 sont réunies puis concentrées à sec sous pression réduite (2,5 kPa). On obtient 4,98 g de diméthyl-7,7 (méthoxy-2 phényl)-4 [(méthoxy-2 phényl)-acétyl]-2 hexahydro-2,3,3a,4,7,7a-(1H)-isoindolol-4-(3aRs, 4RS,7aRS) sous la forme d'une huile incolore.

Spectre IR (KBr - bandes caractéristiques en cm⁻¹) : 3500, 2945, 2885, 2855, 2840, 1640, 1600-1580-1495, 1465, 1245, 1025.

La diméthyl-7,7 [(méthoxy-2 phényl)-acétyl]-2 hexahydro-2,3, 3a,4,7,7a-1(H)-isoindolone-4-(3aRS,7aRS) peut être préparée de la manière suivante :

En opérant selon le mode opératoire de l'exemple 16 à partir de 5,2 g de chlorhydrate de diméthyl-7,7 [(méthoxy-2 phényl)-acétyl]-2 hexahydro-2,3,3a,4,7,7a-1(H)-isoindolone-4-(3aRS,7aRS) et de 4,71 g d'acide méthoxy-2 phénylacétique et en ajoutant 3,62 cm³ de triéthylamine, on obtient, après purification sur colonne de gel de silice (granulométrie 0,04-0,06 mm, diamètre 6 cm, hauteur 30 cm), 5,96 g de diméthyl-7,7 [(méthoxy-2 phényl)-acétyl]-2 hexahydro-2,3,3a,4,7,7a-1(H)-isoindolone-4-(3aRS,7aRS) sous la forme d'une huile incolore.

Le chlorhydrate de diméthyl-7,7 hexahydro-2,3,3a,4,7,7a-1(H)-isoindolone-4-(3aRS,7aRS) peut être préparé de la manière suivante :

Une solution de 5,83 g de diméthyl-7,7 (vinyloxycarbonyl)-2 hexahydro-2,3,3a,4,7,7a-1(H)-isoindolone-4-(3aRS, 7aRS) dans 80 cm³ de dioxanne saturé en acide chlorhydrique est agitée à température ambiante pendant 1 heure puis concentrée à sec sous pression réduite (2,5 kPa). Le résidu est dissous dans 150 cm³ d'éthanol, on porte au reflux pendant 1 heure 30 minutes, puis on concentre à sec sous pression réduite (2,5 kPa). On obtient 5,2 g de chlorhydrate de diméthyl-7,7 hexahydro-2,3,3a,4,7,7a-1(H)-isoindolone-4-(3aRS,7aRS) sous la forme d'une meringue brune.

Spectre IR (KBr - bandes caractéristiques en cm⁻¹) : 2965, 2900, 2710-2490-2435, 1675,1590,1460, 1380-1370.

La diméthyl-7,7 (vinyloxycarbonyl)-2 hexahydro-2,3,3a,4,7,7a-1(H)-isoindolone-4-(3aRS,7aRS) peut être obtenue de la manière suivante :

A une solution de 14,7 g de benzyl-2 diméthyl-7,7 hexahydro-2,3,3a,4,7,7a-1(H)-isoindolone-4-(3aRS,7aRS) dans 100 cm³ de dichloro-1,2 éthane on ajoute à température ambiante 7,9 cm³ de chloroformiate de vinyle. Le mélange réactionnel est porté au reflux 1 heure 30 minutes, puis concentré à sec sous pression réduite (2,7 kPa). Le résidu est chromatographié sur colonne de gel de silice Merck (granulométrie 0,04-0,06 mm, diamètre 6,6 cm, hauteur 30 cm), en éluant sous une pression de 0,5 bar d'azote par un mélange de cyclohexane et d'acétate d'éthyle (80/20 en volumes) et en recueillant des fractions de 75 cm³. Les fractions 56 à 70 sont réunies puis concentrées à sec sous pression réduite (2,5 kPa). On obtient 5,83 g de diméthyl-7,7 (vinyloxycarbonyl)-2 hexahydro-2,3,3a,4,7,7a-1(H)-isoindolone-4-(3aRS,7aRS) sous la forme d'une huile jaune.

La benzyl-2 diméthyl-7,7 hexahydro-2,3,3a,4,7,7a-1(H)-isoindolone-4-(3aRS,7aRS) peut être préparée de la manière suivante :

A une solution de 15,96 g de diméthyl-4,4 cyclohexadiène-2,5-one et de 46 cm³ de N-butoxyméthyl N-triméthylsilylméthyl benzylamine dans 200 cm³ de dichlorométhane, on ajoute, à une température de 10°C, 8 gouttes d'acide trifluoroacétique. Le mélange réactionnel est porté au reflux pendant 2 heures, puis on ajoute du carbonate de potassium, filtre la solution sur verre fritté et concentre à sec sous pression réduite (2,7 kPa). Le résidu est chromatographié sur colonne de gel de silice (granulométrie 0,04-0,06 mm, diamètre 8,5 cm, hauteur 30 cm), en éluant sous une pression de 0,5 bar d'azote par un mélange de cyclohexane et d'acétate d'éthyle (80/20 en volumes) et en recueillant des fractions de 100 cm³. Les fractions 38 à 56 sont réunies et concentrées à sec sous pression réduite (2,7 kPa). On obtient 14,7 g de benzyl-2 diméthyl-7,7 hexahydro-2,3,3a,4,7,7a-1(H)-isoindolone-4-(3aRS,7aRS) sous la forme d'une huile jaune.

La diméthyl-4,4 cyclohexadiène-2,5-one est préparée selon la méthode de Zimmerman, J. Am. Chem. Soc., 93, 3653 (1971).

### Exemple 23

En opérant selon le mode opératoire de l'exemple 22, à partir de 2 g de méthyl-7 phényl-7 (méthoxy-2 phényl)-4 [(méthoxy-2 phényl)-acétyl]-2 hexahydro-2,3,3a,4,7,7a-(1H)-isoindolol-4-(3aRS,4RS,7aRS) et de 1 g de tétroxyde d'osmium, on obtient après purification sur colonne de gel de silice 0,6 g de méthyl-7 phényl-7 (méthoxy-2 phényl)-4 [(méthoxy-2 phényl)-acétyl]-2 perhydroisoindoletriol-4,5,6-(3aRS,4RS,7SR,7aRS), sous forme d'une meringue beige.

Spectre de RMN du proton (DMSO d₆ + CH₃COOD, 250 MHz, T=393°K), δ (ppm) :
Diastéréoisomère A : 1,44 (s, 3H, CH₃), 2,70-3,80 (8H, CO-CH₂Ph et C-CH-CH₂N et N-CH₂-CH), 3,70 (s, 3H, OCH₃), 3,90 (s, 3H, OCH₃), 4,53 (d, 1H, CH-OH), 4,95 (d, 1H, CH-OH), 6,75-7,75 (13H aromatiques).
Diastéréoisomère B : 1,70 (s, 3H, CH₃), 2,70-3,80 (8H, CO-CH₂Ph et C-CH-CH₂N et N-CH₂-CH), 3,70 (s, 3H, OCH₃), 3,95 (s, 3H, OCH₃), 4,39 (d, 1H, CH-OH), 4,90 (d, 1H, CH-OH), 6,75-7,75 (13H aromatiques).

Spectre IR (CHCl₃, bandes caractéristiques en cm⁻¹) : 3450, 3105-3065, 2940, 2875, 2835, 1735, 1625, 1605-1495, 1465-1440, 1250-1240, 1030-1050, 700.

Le méthyl-7 phényl-7 (méthoxy-2 phényl)-4 [(méthoxy-2 phényl)-acétyl]-2 hexahydro-2,3,3a,4, 7,7a-(1H)-isoindolol-4-(3aRS,4RS,7S,7aRS) peut être préparé de la manière suivante :

En opérant selon le mode opératoire de l'exemple 22 à partir de 3,75 g de méthyl-7 phényl-7 [(méthoxy-2 phényl)-acétyl]-2 hexahydro-2,3,3a,4,7,7a-1(H)-isoindolone-4- (3aRS,7SR, 7aRS) on obtient, après cristallisation dans un mélange cyclohexane/acétate d'éthyle (90/10 en volume) 2,1 g de méthyl-7 phényl-7 (méthoxy-2 phényl)-4 [(méthoxy-2 phényl)-acétyl]-2 hexahydro-2,3,3a,4,7,7a-(1H)-isoindolol-4-(3aRS,4RS,7S,7aRS) sous la forme de cristaux blancs fondant à 188°C.

La méthyl-7 phényl-7 [(méthoxy-2 phényl)-acétyl]-2 hexahydro-2,3,3a,4,7,7a-1(H)-isoindolone-4-(3aRS,7SR,7aRS) peut être préparée de la manière suivante :

En opérant selon le mode opératoire de l'exemple 16 à partir de 7 g de chlorhydrate de méthyl-7 phényl-7 [(méthoxy-2 phényl)-acétyl]-2 hexahydro-2,3,3a,4,7,7a-1(H)-isoindolone-4-(3aRS,7SR,7aRS) et de 5,38 g d'acide méthoxy-2-phénylacétique on obtient, après purification sur colonne de gel de silice, 7,25 g de méthyl-7 phényl-7 [(méthoxy-2 phényl)-acétyl]-2 hexahydro-2,3,3a,4,7,7a-1(H)-isoindolone-4-(3aRS,7SR,7aRS) sous la forme d'une huile jaune.

Spectre IR (CCl₄, bandes caractéristiques en cm⁻¹) : 3450, 3090-3065-3030, 2975, 2940, 2875, 2835, 1680, 1650, 1605-1495, 1460, 1440, 1245, 1050-1030, 700.

Le chlorhydrate de méthyl-7 phényl-7 [(méthoxy-2 phényl)-acétyl]-2 hexahydro-2,3,3a,4,7,7a-1 (H)-isoindolone-4-(3aRS,7SR,7aRS) peut être préparé de la manière suivante :

En opérant suivant le mode opératoire de l'exemple 22 à partir de 8 g de méthyl-7 phényl-7 (vinyloxycarbonyl)-2 hexahydro-2,3,3a,4,7,7a-1(H)-isoindolone-4-(3aRS,7SR,7aRS) on obtient 7 g de chlorhydrate de méthyl-7 phényl-7 [(méthoxy-2 phényl)-acétyll-2 hexahydro-2,3,3a,4,7,7a-1(H)-isoindolone-4-(3aRS,7SR,7aRS) sous la forme d'une meringue rose utilisée directement à l'étape suivante.

La méthyl-7 phényl-7 (vinyloxycarbonyl)-2 hexahydro-2,3,3a,4,7,7a-1(H)-isoindolone-4-(3aRS,7SR,7aRS) peut être obtenue de la manière suivante :

A une solution de 11,7 g de benzyl-2 méthyl-7 phényl-7 hexahydro-2,3,3a,4,7,7a-1(H)-isoindolone-4-(3aRS,7SR,7aRS) dans 150 cm³ de dichloro-1,2 éthane on ajoute à température ambiante 5,2 cm³ de chloroformiate de vinyle. Le mélange réactionnel est porté au reflux 2 heures, puis concentré à sec sous pression réduite (2,7 kPa). Le résidu est chromatographié sur colonne de gel de silice (granulométrie 0,04-0,06 mm, diamètre 4,5 cm, hauteur 25 cm), en éluant sous une pression de 0,5 bar d'azote par un mélange de cyclohexane et d'acétate d'éthyle (80/20 en volumes) et en recueillant des fractions de 90 cm³. Les fractions 10 à 21 sont réunies puis concentrées à sec sous pression réduite (2,5 kPa). On obtient 8,1 g de méthyl-7 phényl-7 (vinyloxycarbonyl)-2 hexahydro-2,3,3a,4,7,7a-1(H)-isoindolone-4-(3aRS,7SR,7aRS) sous la forme d'une huile jaune clair utilisée directement pour l'étape suivante.

Spectre de RMN du proton (DMSO d₆, 250 MHz), δ (ppm) : 1,67 (s, 3H, CH₃), 2,60 (m, 2H, N-CH₂-CH), 3,10 (m, 1H, CH₂-CH-C), 3,30 (m, 2H, N-CH₂-CH), 3,40 (m, 1H, CH₂-CH-C), 4,00 (d, 1H, CO-CH=CH₂), 4,40 et 4,7 (2d, 2x1H, -CH=CH₂), 6,11 (d, 1H, CH=CH-C), 6,95 (q, 1H, CO-CH-CH₂), 7,2-7,5 (m, 5H, aromatiques)

La benzyl-2 méthyl-7 phényl-7 hexahydro-2,3,3a,4,7,7a-1(H)-isoindolone-4-(3aRS,7SR,7aRS) peut être obtenue de la manière suivante :

En opérant selon le mode opératoire de l'exemple 22 à partir de 11,8 g de phényl-4 méthyl-4 cyclohexadiène-2,5-one-1-(4RS) et de 28 cm³ de N-butoxyméthyl N-triméthylsilylméthyl benzylamine, on obtient après chromatographie sur colonne de gel de silice et cristallisation dans l'oxyde d'isopropyle, 6 g de benzyl-2 méthyl-7 phényl-7 hexahydro-2,3,3a,4,7,7a-1(H)-isoindolone-4-(3aRS,7SR,7aRS) sous la forme d'un solide jaune fondant à 110°C.

La phényl-4 méthyl-4 cyclohexadiène-2,5-one peut être préparée selon la méthode de H.E Zimmerman et G.Jones, J. Amer. Chem. Soc., 92, 9, 2753 (1970).

### Exemple 24

A une solution de 1,0 g de (méthoxy-2 phényl)-4 méthyl-6 perhydroisoindolol-4-(3aS,4S,6R,7aR) dans 100 cm³ de dichlorométhane on ajoute 1,5 cm³ de triéthylamine. Le mélange réactionnel est refroidi à 5°C et 0,60 g d'acide (diméthylamino-2 phényl) acétique, 0,10 g d'hydroxy-1 benzotriazole monohydrate et 0,70 g de chlorhydrate de (diméthylamino-3 propyl)-1 éthyl-3 carbodiimide sont additionnés. Le mélange réactionnel est maintenu 1 heure à 5°C et 16 heures à 20°C puis est lavé 2 fois avec 100 cm d'eau, séché sur sulfate de magnésium, filtré et concentré sous pression réduite (2,7 kPa). Le résidu est chromatographié sur une colonne de gel de silice (0,060-0,200 mm, diamètre de 4 cm, hauteur de 50 cm) en éluant sous une pression de 0,7 bar par un mélange de dichlorométhane et de méthanol [97/3 en volumes] et en recueillant des fractions de 50 cm³. Les fractions 14 à 19 sont concentrées et l'on obtient après séchage à 40°C sous 15 Pa 1,0 g de (méthoxy-2 phényl)-4 [(diméthylamino-2 phényl) acétyl]-2 méthyl-6 perhydroisoindolol-4-(3aS,4S,6R,7aR) sous forme d'une poudre blanche qui est dissoute dans 50 cm³ de dioxanne. On ajoute 3 cm³ d'une solution 5M d'acide chlorhydrique dans le dioxanne, après 1 heure à température ambiante la solution est concentrée sous pression réduite (2,7 kPa), agitée dans l'éther isopropylique, filtrée et séchée à 40°C sous 15 Pa. On obtient 0,94 g de chlorhydrate de (méthoxy-2 phényl)-4 [(diméthylamino-2 phényl) acétyl]-2 méthyl-6 perhydroisoindolol-4-(3aS,4S,6R,7aR).

Spectre de R.M.N. ¹ H (250 MHz, (CD₃)₂SO d6 avec ajout de quelques gouttes de CD₃COOD d4, δ en ppm): à température ambiante, on observe le mélange des rotamères. 0.91 et 0.92 (2d, J= 7 Hz, 3H en totalité : CH₃ en 5); de 1.25 à 1.90 (mt, 3H: CH₂ 7 et 1H du CH₂ 5); de 2.00 à 2.50 (mt, 2H: l'autre H du CH₂ 5 et H 6); de 2.50 à 2.80 (mt, 1H: H 7a); 2.83 et 3.00 (2t larges, J= 8 Hz, 1H en totalité: H 3a); 3.00 à 4.20 (mt, 6H: NCH₂ 1 - NCH₂ 3 et CH₂Ar); 3.24 (s, 6H: N(CH₃)₂); 3.80 et 3.83 (2s, 3H en totalité: OCH₃); de 6.85 à 7.95 (mt, 8H: H Aromatiques).

IR (KBr, cm⁻¹): 3420, 2950-2920, 2790-2100, 1640, 1600, 1500, 1435, 1465, 1375, 1235, 1025, 760.

[α]^{D}₂₀ = -19,5° (C = 5 g/l, méthanol).

L'acide diméthylamino-2 phényl acétique peut être préparé selon la méthode décrite dans le brevet EP 429,366.

Le (méthoxy-2 phényl)-4 méthyl-6 perhydroisoindolol-4-(3aS,4S,6R,7aR) peut être préparé comme à l'exemple 1 en partant de méthyl-5 cyclohexèn-2-one-(R) dont la préparation est décrite par W.Oppolzer et M.Petrzilka, Helv.Chim.Acta, 61(8) 2755 (1978).

### Exemple 25

A une solution de 1,0 g de (méthoxy-2 phényl)-4 méthyl-6 perhydroisoindolol-4-(3aS,4S,6R,7aR) dans 70 cm de dichlorométhane on ajoute 1,0 cm ³ de triéthylamine. Le mélange réactionnel est refroidi à 5°C et 0,69 g d'acide (benzoxy-2 phényl)-2 propionique-(S), 0,045 g d'hydroxy-1 benzotriazole monohydrate et 0,56 g de chlorhydrate de (diméthylamino-3 propyl)-1 éthyl-3 carbodiimide sont additionnés. Le mélange réactionnel est maintenu 1 heure à 5°C et 16 heures à 20°C puis est lavé 2 fois avec 50 cm d'eau, séché sur sulfate de magnésium, filtré et concentré sous pression réduite (2,7 kPa). Le résidu est chromatographié sur une colonne de gel de silice (0,060-0,200 mm, diamètre de 4 cm, hauteur de 35 cm) en éluant sous une pression de 0,7 bar par un mélange de dichlorométhane et de méthanol [97,5/2,5 en volumes] et en recueillant des fractions de 25 cm³. Les fractions 8 à 14 sont concentrées et l'on obtient après séchage à 40°C sous 15 Pa 1,0 g de (méthoxy-2 phényl)-4 [(benzoxy-2 phényl)-2 propionyl-(S)]-2 méthyl-6 perhydroisoindolol-4-(3aS,4S,6R,7aR) sous forme d'un solide blanc.

Spectre de R.M.N. 1 H (250 MHz, (CD3)2SO d6 avec ajout de quelques gouttes de CD3COOD d4, à une température de 393 K, δ en ppm): 0.91 (d, J= 7 Hz, 3H: CH3 en 5); de 1.25 à 1.40 et 1.72 (2 mts, 1H chacun: CH2 7); 1.30 (d, J= 7 Hz, 3H: CH3 5); 1.62 et 2.05 (respectivement d large et t, J= 12.5 Hz, 1H chacun: CH2 5); 2.17 (mt, 1H: H 6); 2.37 (mt, 1H: H 7a); 2.77 (t large, J= 7.5 Hz, 1H: H 3a); de 3.05 à 3.60 (mt, 4H: NCH2 1 et NCH2 3); 3.83 (s, 3H: OCH3); 419 (q, J= 7 Hz, 1H: CHAr); de 4.95 à 5.25 (mt, 2H: OCH2Ar); de 6.85 à 7.55 (mt, 13H: H Aromatiques).

IR (KBr, cm⁻¹): 3425, 3075, 3035, 2950, 2925, 2870, 2850, 1635, 1600, 1585, 1490, 1455, 1435, 1235, 1030, 755, 700.

A une solution de 0,85 g de [(benzoxy-2 phényl)-2 propionyl-(S)]-2 (méthoxy-2 phényl)-4 méthyl-6 perhydroisoindolol-4-(3aS,4S,6R,7aR) dans 12 cm³ d'éthanol absolu, on ajoute 0,2 g d'hydroxyde de palladium à 20 % et on fait buller de l'hydrogène dans le mélange réactionnel à 40°C pendant 3 heures. Après retour à température ambiante et purge à l'argon, le mélange est filtré sur célite et concentré à sec sous pression réduite (2,7 kPa). Le résidu est recristallisé dans l'éthanol et l'on obtient après filtration et séchage 0,5 g de [(hydroxy-2 phényl)-2 propionyl-(S)]-2 (méthoxy-2 phényl)-4 méthyl-6 perhydroisoindolol-4-(3as,45,6R,7aR) sous la forme de cristaux blancs fondant à 280°C [α]_{D} = -26,5° (C=5,3 g/l, CHCl₃).

L'acide (benzoxy-2 phényl)-2 propionique-(S) peut être préparé selon la méthode décrite dans la demande de brevet WO 93/21155.

### Exemple 26

En opérant de façon identique à l'exemple 1, on utilise 1,5 g de (hydroxyméthyl)-7 (méthoxy-2 phényl)-4 méthyl-7 perhydroisoindolol-4-(3aRS,4RS,7SR,7aRS) et 0,9 g d'acide (N,N-diméthylamino-2 phényl) acétique. Après concentration, le résidu brut est recristallisé dans l'acétonitrile et l'on obtient après séchage à 40°C sous 15 Pa 1,0 g de [(N,N-diméthylamino-2 phényl) acétyl]-2 (hydroxyméthyl)-7 (méthoxy-2 phényl-4 méthyl-7 perhydroisoindolol-4-(3aRS,4RS,7SR,7aRS) sous forme d'un solide blanc fondant à 138-142°C.

### Exemple 27

En opérant de façon identique à l'exemple 1, on utilise 3,0 g de (hydroxyméthyl)-7 (méthoxy-2 phényl)-4 méthyl-7 perhydroisoindolol-4-(3aRS,4RS,7SR,7aRS) et 1,74 g d'acide (méthoxy-2 phényl)-2 propionique- (S) . Après recristallisation du résidu brut dans l'acétonitrile, on effectue une séparation des 2 diastéréoisomères par Chromatographie Liquide Haute Performance (C.L.H.P.) sur une colonne de gel de silice (diamètre de 4 cm, hauteur de 10 cm) en éluant par un mélange d'acétate d'éthyle, de cyclohexane et de méthanol [64/35/1 en volumes]. Les fractions recueillies sont concentrées et le solide recristallisé dans l'acétonitrile, on obtient après séchage à 40°C sous 15 Pa 0,10 g d'(hydroxyméthyl)-7 (méthoxy-2 phényl)-4 [(méthoxy-2 phényl)-2 propionyl-(S)]-2 méthyl-7 perhydroisoindolol-4-(3aS,4S,7R,7aS) sous forme de cristaux blancs fondant à 215°C.

### Exemple 28

En opérant comme à l'exemple 1, on obtient après séparation par CLHP le [(N,N-diméthylamino-2 phényl)-2 propionyl- (S)] -2 (méthoxy-2 phényl)-4 méthyl-6 perhydroisoindolol-4-(3aS,4S,6R,7aR) sous forme d'un solide blanc fondant à 83°C. [α]²⁰_{D} = +32,7° dans le méthanol à 20°C (C=5,08 g/l).

Spectre de R.M.N. 1 H (250 MHz, (CD3)2SO d6 avec ajout de quelques gouttes de CD3COOD d4, à une température de 403 K, δ en ppm): 0.92 (d, J= 7 Hz, 3H: CH3 en 5); de 1.25 à 1.45 et 1.80 (respectivement mt et d large (J= 14 Hz), 1H chacun: CH2 7); 1.36 (d, J= 7 Hz, 3H: CH3); 1.63 et 1.97 (respectivement d large et t, J= 12.5 Hz, 1H chacun: CH2 5); 2.19 (mt, 1H: H 6); 2.48 (mt, 1H: H 7a); 2.58 (s, 6H: N(CH3)2); 2.82 (t large, J= 8 Hz, 1H: H 3a); de 3.10 à 3.60 (mt, 4H: NCH2 1 et NCH2 3); 3.83 (s, 3H: OCH3); 4.46 (q, J= 7 Hz, 1H: CHAr); de 6.85 à 7.40 (mt, 8H: H Aromatiques).

IR (KBr, cm⁻¹): 3420, 2925, 2870, 2780, 1625, 1600, 1585, 1490, 1435, 1455, 1370, 1235, 1035, 755.

### Exemple 29

A une solution de 2,77 g d'hydroxyméthyl-6 (méthoxy-2 phényl)-4 perhydroisoindolol-4-(3aRS,4RS,6SR,7aSR), de 1,80 g d'acide (méthoxy-2 phényl)-2-propionique-(S) et de 30 mg d'hydrate d'hydroxybenzotriazole dans 80 cm³ de dichlorométhane, on ajoute 2,1 g de (diméthylamino-3 propyl)-1 éthyl-3 carbodiimide. Après 20 heures d'agitation à 20°C, le mélange réactionnel est lavé par 40 cm³ d'eau, séché sur sulfate de magnésium, et concentré à sec sous pression réduite (2,7 KPa). Le résidu est chromatographié sur une colonne de gel de silice (granulométrie 0,06-0,20 mm, diamètre 5 cm, hauteur 40 cm) dans un mélange de cyclohéxane et d'acétate d'éthyle (30/70 en volumes) puis dans un mélange d'acétate d'éthyle et de méthanol (90/10 en volumes) On obtient 3,6 g d'hydroxyméthyl-6 (méthoxy-2 phényl)-4 [(méthoxy-2 phényl)-2 propionyl-(S)]-2 perhydroisoindolol-4-(mélange des 2 diastéréoisomères 3aR,4R,6S,7aS,S et 3aS,4S,6R, 7aR,S) après évaporation de l'éluant et séchage sous pression réduite (2,7 KPa), sous la forme d'un solide blanc. Pf_{K} = 178°C.

RMN du proton (DMSO d_{6,} avec ajout de quelques gouttes de CD₃COOD, à une température de 393°K, δ ppm): on observe le mélange de 2 diastéréoisomères. 1,20 à 2,10(mt, 4H, CH₂ en 7 et CH₂ en 5); 1,29 et 1,33 (2d, j = 7, 3H, CH₃) ; 2,05 à 2,30 (mt, 1H: H 6); 2,40 à 2,60 (mt, 1H: H 7a); 2,75 à 2,90 (mt, 1H, H: 3a); 2,90 à 3,60 (mt, 4H: NCH₂); 3,35 (d, j = 5,5, 2H: OCH₂); 3,76 - 3,78 - 3,83 et 3,85 (4s, 6H: OCH₃); 4,05 à 4,25 (mt, 1H: ArCH); 6,85 à 7,65 (mt, 8H: H Aromatiques).

Spectre infrarouge (bandes caractéristiques en cm⁻¹): 3425, 2930, 2885, 2840, 1625, 1600 + 1490 + 1435, 1460, 1375, 1240, 1030, 755

### Exemple 30

En opérant selon le mode opératoire de l'exemple 16, à partir de 1,21 g d'acide indolyl-3 acétique et de 1,39 g de (méthoxy-2 phényl)-4 méthyl-5 perhydroisoindolol-4-(3aRS,4RS,5RS,7aSR), on obtient, après purification sur colonne de gel de silice (granulométrie 0,04-0,06 mm, diamètre 2,8 cm, hauteur 25 cm), en éluant sous une pression de 0,5 bar d'azote par un mélange de cyclohexane et d'acétate d'éthyle (40/60 en volumes) 0,88 g de (méthoxy-2 phényl)-4 (indolyl-3 acétyl)-2 méthyl-5 perhydroisoindolol -4-(3aRS,4RS,SRS,7aSR) sous la forme d'une meringue blanche.

Spectre RMN du proton (DMSO d₆, 250 MHz à une température de 393 K, d en ppm): 0.60 (d, J= 7 Hz, 3H: CH₃); 1.36 et de 1.70 à 1.95 (2 mts, respectivement 1H et 3H: CH₂ en 6 et CH₂ en 7); de 2.45 à 2.65 (mt, 2H: H 7a et H 5); 2.90 (t, J= 7 Hz, 1H: H 3a); de 3.10 à 3.60 (mt, 4H: NCH₂ 3 et NCH₂ 1); 3.65 (s large, 2H: ArCH₂); 3.75 (s large, 1H: OH); 3.85 (s, 3H: OCH₃); de 6.90 à 7.65 (mt, 9H: H Aromatiques); 10.35 (mf, 1H: NH).

### Exemple 31

En opérant comme à l'exemple 33, mais à partir de 2,5 g de (fluoro-2 phényl)-4 méthyl-6 perhydroisoindolol-4-(3aS,4S,6R,7aR) et de 2,17 g d'acide (méthoxy-2 phényl)-2 propionique-(S), on obtient, après purification sur colonne de gel de silice (granulométrie 0,04-0,06 mm, diamètre 4 cm, hauteur 25 cm), en éluant sous une pression de 0,5 bar d'azote par un mélange de cyclohexane et d'acétate d'éthyle (90/10 en volumes), 3,87 g d'un solide qui est recristallisé dans de l'acétonitrile pour fournir 2 g de (fluoro-2 phényl)-4 [(méthoxy-2 phényl)-2 propionyl-(S)]-2 méthyl-6 perhydroisoindolol-4- (3aS,4S,6R, 7aR) sous la forme d'un solide blanc fondant à 170°C.

### Exemple 32

En opérant de façon identique à l'exemple 1, on utilise 1,5 g de (hydroxyméthyl)-7 (méthoxy-2 phényl)-4 méthyl-7 perhydroisoindolol-4-(3aRS,4RS,7SR,7aRS) et 0,88 g d'acide indole-3 acétique. Après concentration, le résidu brut est recristallisé dans l'acétonitrile et l'on obtient après séchage à 40°C sous 15 Pa, 0,7 g d'(hydroxyméthyl)-7 (indolyl-3 acétyl)-2 (méthoxy-2 phényl)-4 méthyl-7 perhydroisoindolol-4-(3aRS,4RS,7SR,7aRS) sous forme d'un solide blanc fondant à 170-180°C (fusion pâteuse).

Spectre de R.M.N. 1 H (250 MHz, (CD₃)₂SO d6 avec ajout de quelques gouttes de CD3COOD d4, à une température de 393 K, δ en ppm): 1.12 et 1.48 (2d larges, J= 13 Hz, 1H chacun: 1H du CH2 6 et 1H du CH2 7); 1.23 (s, 3H: CH3); 1.91 et 2.71 (2 dts larges, J= 13 et 4 Hz, 1H chacun: l'autre H du CH2 6 et l'autre H du CH2 7); 2.18 (mt, 1H: H 7a); 3.02 (mt, 1H: H 3a); 3.25 et 3.33 (2d, J= 10.5 Hz, 1H chacun: OCH2); de 3.20 à 3.80 (mt, 4H: NCH2 3 et NCH2 1); 3.63 (s large, 2H: ArCH2); 3.86 (s, 3H: OCH3); de 6.90 à 7.65 (mt, 9H: H Aromatiques).

IR (KBr, cm⁻¹): 3420, 2925, 2875, 2830, 1610, 1485, 1435, 1460, 1230, 1025, 760, 740.

### Exemple 33

A une solution de 0,52 g de (bis-trifluorométhyl-3,5 phényl)-4 perhydroisoindolol-4-(3aRS,4RS,7aSR) et de 0,34 g d'acide (méthoxy-2 phényl)-2 propionique-(S) dans 10 cm³ de dichlorométhane, refroidie à 0°C, on ajoute 0,02 g d'hydroxy-1-benzotriazole et 0,37 g de chlorhydrate de (diméthylamino-3 propyl)-1 éthyl-3 carbodiimide. On agite 18 heures à température ambiante puis on lave la phase organique par 2 fois 20 cm³ d'eau, puis par 20 cm³ d'une solution aqueuse saturée en chlorure de sodium, on sèche sur sulfate de magnésium et concentre à sec sous pression réduite (2,7 kPa). Le résidu est chromatographié sur colonne de gel de silice (granulométrie 0,04-0,06 mm, diamètre 2,6 cm, hauteur 20 cm), en éluant sous une pression de 0,5 bar d'azote par un mélange de cyclohexane et d'acétate d'éthyle (80/20 en volumes) et en recueillant des fractions de 20 cm³. Les fractions 14 à 35 sont réunies puis concentrées à sec sous pression réduite (2,7 kPa). On obtient 0,53 g de [(méthoxy-2 phényl)-2 propionyl-(S)]-2 bis-trifluorométhyl-3,5 phényl-4 perhydroisoindolol-4- (3aR,4R,7aS) et (3aS,4S,7aR) (mélange 50/50 des deux diastéréoisomères) sous la forme d'une meringue blanc cassé.

Spectre RMN du proton (DMSO d₆, 250MHz, à une température de 393 K, on observe le mélange de deux diastéréoisomères): de 1.2 à 2.0 (mt, 6H, CH₂); de 1.25 à 1.4 (mt, 3H, CH₃); de 2.3 à 2.7 (mt, 1H, H7a); de 2.6 à 2.9 (mt, 1H, H3a); de 3.1 à 3.65 (mt, 4H, NCH₂); 3.8 (s trés large, 3H, OCH₃); de 4.1 à 4.25 (mt, 1H, ArCH); de 4.8 à 5.2 (mf étalé, 1H, OH); de 6.95 à 7.4 (mt, 4H, H de l'aromatique disubstitué); de 7.9 à 8.3 (mt, 3H, H de l'aromatique trisubstitué).

Spectre infrarouge (bandes caractéristiques en cm⁻¹): 3340, 2935, 2875, 2860, 1625, 1495, 1460, 1450, 1440, 1175, 1130, 845.

Le (bis-trifluorométhyl-3,5 phényl)-4 perhydroisoindolol-4-(3aRS,4RS,7aSR) peut être obtenu de la façon suivante :

A une solution de 0,82 g de benzyl-2 (bis-trifluorométhyl-3,5 phényl)-4 perhydroisoindolol-4-(3aRS,4RS,7aSR) dans 15 cm³ d'éthanol absolu, on ajoute 0,25 g d'hydroxyde de palladium sur charbon à 10 %, puis le mélange réactionnel est hydrogéné sous agitation au reflux. Après 1 heures de réaction, le mélange réactionnel est filtré, puis concentré à sec sous pression réduite (2,7 kPa). On obtient 0,52 g de (bis-trifluorométhyl-3,5 phényl)-4 perhydroisoindolol-4-(3aRS,4RS, 7aSR) sous la forme d'une pâte noire.

Le benzyl-2 (bis-trifluorométhyl-3,5 phényl)-4 perhydroisoindolol-4-(3aRS,4RS,7aSR) peut être obtenu de la façon suivante :

A une solution de 3,06 cm³ de bis-trifluorométhyl-3,5 bromobenzène dans 15 cm³ de tétrahydrofurane sec, on ajoute, à -78°C, une solution de 10,9 cm³ de butyllithium 1,6 M dans l'hexane. Cette solution est agitée 30 minutes à -78°C, puis on coule goutte à goutte une solution de 1 g de benzyl-2 perhydroisoindolone-4-(3aRS,7aSR) dans 5 cm³ de tétrahydrofurane. Le mélange réactionnel est ensuite agité à température ambiante pendant 18 heures, traité par 20 cm³ d'eau puis extrait par 20 cm³ d'acétate d'éthyle. La phase organique est décantée, lavée par 2 fois 20 cm³ d'eau, puis par 20 cm³ d'une solution saturée en chlorure de sodium, séchée sur sulfate de magnésium et concentrée à sec sous pression réduite (2,7 kPa). Le résidu est chromatographié sur colonne de gel de silice (granulométrie 0,04-0,06 mm, diamètre 2 cm, hauteur 20 cm), en éluant sous une pression de 0,5 bar d'azote par un mélange de cyclohexane et d'acétate d'éthyle (90/10 en volumes) et en recueillant des fractions de 20 cm³. Les fractions 26 à 37 sont réunies puis concentrées à sec sous pression réduite (2,7 kPa). On obtient 0,82 g de benzyl-2 (bis-trifluorométhyl-3,5 phényl)-4 perhydroisoindolol-4- (3aRS,4RS,7aSR) sous la forme d'une huile.

### Exemple 34

A une solution de 0,66 g d'acide méthoxy-2 phénylacétique dans 30 cm³ de dichlorométhane on ajoute à 5°C, 0,65 g de N,N' carbonyl-diimidazole. Après 4 heures d'agitation à température ambiante on additionne goutte à goutte une solution contenant 1 g de méthyl-7 phényl-7 (méthoxy-2 phényl)-4 perhydroisoindol-4,5-diol (3aRS,4RS, 5RS,7SR,7aRS) dans 20 cm³ de dichlorométhane. Après 48 heures d'agitation le milieu réactionnel est lavé avec 50 cm³ d'eau puis avec 50 cm³ d'une solution aqueuse saturée en chlorure de sodium. La phase organique est décantée, séchée sur sulfate de magnésium, filtrée et concentrée à sec sous pression réduite (2,7 kPa). Le résidu est chromatographié sur colonne de gel de silice Merck (granulométrie 0,04-0,06 mm, diamètre 2,2 cm, hauteur 26 cm), en éluant sous une pression de 0,5 bar d'azote par un mélange de cyclohexane et d'acétate d'éthyle (50/50 en volumes) et en recueillant des fractions de 35 cm³. Les fractions 11 à 25 sont réunies puis concentrées à sec sous pression réduite (2,5 kPa) .On obtient 1 g de méthyl-7 phényl-7 (méthoxy-2 phényl)-4 [(méthoxy-2 phényl)-acétyl]-2 perhydroisoindol-4,5-diol (3aRS,4RS,5RS,7SR,7aRS) sous la forme d'une meringue blanche.

RMN ¹H (DMSO d₆ + AcOD, 250 MHz, T=413K), d(ppm): 1,60 (s, 3H: -CH₃), 1,85 et 2,45 (dd et t, 2x1H, CH-CH₂-C), 2,73 (m, 1H: C-CH-CH₂N), 2,90 et 3,20 (2t, 2x1H, N-CH₂-CH), 3,20 et 3,50 (2m, 2x1H, N-CH₂-CH), 3,22 (m, 1H: C-CH-CH₂-N), 3,38 (s, 2H, CO-CH₂-Ph), 3,68 (s, 3H, -OCH₃), 3,82 (s, 3H: OCH₃), 4,93 (dd, 1H: C-CH-OH), 6,80-7,65 (m, 13H aromatiques).

Le méthyl-7 phényl-7 (méthoxy-2 phényl)-4 perhydroisoindol-4,5-diol (3aRS,4RS,5RS,7SR,7aRS) peut être préparé de la façon suivante :

A une solution de 1,6 g de benzyl-2 méthyl-7 phényl-7 (méthoxy-2 phényl)-4 perhydroisoindol-4,5-diol (3aRS,4RS, 5RS,7SR,7aRS) dans 50 cm³ d'éthanol anhydre on ajoute 0,3 g d'hydroxyde de palladium sur charbon à 20 % ; puis le mélange réactionnel est hydrogéné, sous agitation, à une température de 60°C et sous pression atmosphérique. Après 2 heures de réaction le mélange réactionnel est filtré, puis concentré à sec sous pression réduite (2,7 kPa). On obtient 1 g de méthyl-7 phényl-7 (méthoxy-2 phényl)-4 perhydroisoindol-4,5-diol (3aRS,4RS,5RS,7SR,7aRS) sous la forme d'une meringue crème utilisée directement pour l'étape suivante.

Le benzyl-2 méthyl-7 phényl-7 (méthoxy-2 phényl)-4 perhydroisoindol-4,5-diol (3aRS,4RS,5RS,7SR,7aRS) peut être préparé de la façon suivante :

On additionne 37 cm³ d'une solution 1,38 molaire de bromure de méthoxy-2 phénylmagnésium dans le tétrahydrofurane à une solution contenant 2,4 g d'acétoxy-5 benzyl-2 méthyl-7 phényl-7 perhydroisoindol-4-one (3aRS,5RS,7SR,7aRS) dans 50 cm³ de tétrahydrofurane à 4°C. Le mélange réactionnel est agité à température ambiante pendant 18 heures, traité par 150 cm³ d'une solution aqueuse saturée en chlorure d'ammonium, repris par 100 cm³ d'acétate d'éthyle et lavé par 100 cm³ d'eau puis par 100 cm³ d'une solution aqueuse saturée en chlorure de sodium. La phase organique est décantée, séchée sur sulfate de magnésium, filtrée et concentrée à sec sous pression réduite (2,7 kPa). Le résidu est chromatographié sur colonne de gel de silice Merck (granulométrie 0,04-0,06 mm, diamètre 4 cm, hauteur 27 cm), en éluant sous une pression de 0,5 bar d'azote par un mélange de cyclohexane et d'acétate d'éthyle (60/40 en volumes) et en recueillant des fractions de 35 cm³. Les fractions 8 à 15 sont réunies puis concentrées à sec sous pression réduite (2,5 kPa). On obtient 1,65 g de benzyl-2 méthyl-7 phényl-7 (méthoxy-2 phényl)-4 perhydroisoindol-4,5-diol (3aRS,4RS,5RS,7SR,7aRS) sous la forme d'une meringue jaune.

En opérant selon le mode opératoire de l'exemple 15 à partir de 16,22 g d'acétoxy-6 méthyl-4 phényl-4 cyclohexènone-2 (mélange des formes A et B) et de 22,25 g de N-butoxyméthyl N-triméthylsilylméthyl benzylamine on obtient après purification sur colonne de gel de silice 2 g d'acétoxy-5 benzyl-2 méthyl-7 phényl-7 (méthoxy-2 phényl)-4 perhydroisoindol-4-one (3aRS,5RS,7SR,7aRS) sous forme d'une huile jaune.

RMN ¹H (CDCl₃, 250 MHz), δ (ppm): 1,75 (s, 3H: -CH₃), 2,07 et 2,30 (t et dd, 2x1H, N-CH₂-CH), 2,25 (s, 3H: -CH₃), 2,44 et 2,60 (m et t, 2x1H, C-CH₂-CH), 2,77 et 3,48 (dd, 2H, N-CH₂-CH), 3,08 (t, 1H: CO-CH-CH), 3,19 (m, 1H: C-CH-CH), 3,53 et 3,63 (d, 2H, N-CH₂-Ph), 5,64 (dd, 1H: O-CH-CO), 7,15-7,40 (m, 5H, aromatiques).

L'acétoxy-6 méthyl-4 phényl-4 cyclohexène-2-one (mélange 6/4 des formes A et B) peut être préparée à partir de la méthyl-4 phényl-4 cyclohexène-2-one selon la méthode décrite par G.M. Rubottom at coll., J. Org. Chem., 43,1599,(1978).

La méthyl-4 phényl-4 cyclohexène-2-one peut être préparée selon la méthode de H.E Zimmerman et G.Jones, J. Am. Chem. Soc., 92, 9, 2753,(1970).

La présente invention concerne également les compositions pharmaceutiques constituées par un produit de formule générale (I) ou un sel lorsqu'ils existent, éventuellement en association avec tout autre produit pharmaceutiquement compatible, pouvant être inerte ou physiologiquement actif. Les compositions selon l'invention peuvent être utilisées par voie parentérale, orale, sublinguale, rectale, topique, oculaire, intranasale ou en aérosols à visée pulmonaire.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont des suppositoires ou des capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

Les compositions pour administration oculaires peuvent être des instillations.

Les compositions pour administration intranasale peuvent être des solutions ou des poudres pharmaceutiquement acceptables destinées à des gouttes ou à des pulvérisations.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, du sérum ou tout autre véhicule pharmaceutiquement acceptable. Pour l'usage sous forme d'aérosols secs destinés à être directement inhalés, le principe actif est est finement divisé et associé à un diluant ou véhicule solide hydrosoluble d'une granulométrie de 30 à 80 µm, par exemple le dextrane, le mannitol ou le lactose.

En thérapeutique humaine, les produits selon l'invention peuvent être particulièrement utiles dans le traitement des douleurs d'origine traumatique, post-chirurgicale, menstruelle, céphaliques, dans les algies vasculaires de la face (cluster headache) et dans les traitements des migraines. Les nouveaux dérivés de l'isoindole sont également utiles dans le traitement de l'inflammation en rhumathologie, dans le traitement de l'arthrite rhumatoïde et dans les troubles dus au dérèglement du système immunitaire, dans les traitements des inflammations en dermatologie tels que le psoriasis, l'herpès, les urticaires, les eczémas, les photodermatoses, les brûlures et dans les troubles inflammatoires dentaires ou oculaires et dans le domaine des sécretions lachrymales ; ils sont également utiles dans les traitements des manifestations spasmodiques, douloureuses et inflammatoires des voies digestives (colites ulcéreuses, syndrome du colon irritable, maladie de Crohn), des voies urinaires (hyperréflexies urinaires, cystites) et des voies respiratoires (asthme, hypersécretion bronchique, bronchite chronique, rhinites) ainsi que dans les traitements anti-émétiques. Les produits selon l'invention peuvent également trouver une application dans les traitements de maladies neurologiques, maladie de Parkinson, maladie d'Alzheimer, dans les traitements des maladies inflammatoires et/ou autoimmunes et/ou démyélinisantes du système nerveux central et/ou périphérique (sclérose en plaque, syndrome de Guillain-Barré, encéphalopathies d'origine virale ...), dans les syndromes neurologiques en relation avec une extravasation plasmatique (oedème de la moelle épinière, oedème cérébral...), en relation avec une atteinte de la barrière hématoencéphalique ou dans tout syndrome neurologique spastique (traitements myorelaxants). Les produits selon l'invention peuvent également être utiles dans les traitements de l'anxiété, des psychoses, de la schizophrénie, ou encore dans les traitements des troubles cardiovasculaires tels de l'hypotension. Une autre application peut être également le traitement des troubles gynécologiques, le traitement des troubles liés à une mauvaise régulation de la croissance (nanisme, hypothrophies secondaires à des maladies chroniques de l'enfant, ostéoporose, développement de greffes).

Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

### Exemple

On prépare, selon la technique habituelle, des comprimés de produit actif ayant la composition suivante :
- (méthoxy-2 phényl)-4 (méthoxy-2 phénylacétyl)-2 méthyl-5 perhydroisoindolol-4-(3aRS,4RS,SRS,7aSR) 25 mg
- amidon 83 mg
- silice 30 mg
- stéarate de magnésium 3 mg

## Revendications

1. Un dérivé de perhydroisoindole caractérisé en ce qu'il répond à la formule générale : 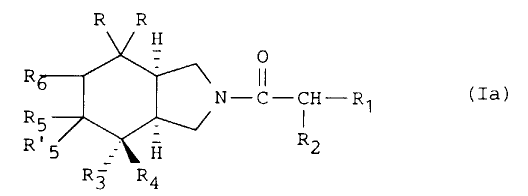 dans laquelle
- le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino), alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle, indényle ou hétérocyclyle mono ou polycyclique, saturé ou insaturé contenant 5 à 9 atomes de carbone et un ou plusieurs hétéroatomes choisis parmi l'oxygène, l'azote ou le soufre, et éventuellement substitué par un atome d'halogène ou par un radical alcoyle ou alcoyloxy,
- le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino ou acylamino,
- le symbole R₃ représente un radical phényle éventuellement substitué en position -2 par un radical alcoyle ou alcoyloxy contenant 1 ou 2 atomes de carbone ou par un atome de fluor, ou disubstitué par des radicaux trifluorométhyle et
- les symboles R₅ et R'₅ sont identiques ou différents et représentent l'un un atome d'hydrogène ou un radical hydroxy ou alcoyle et l'autre un atome d'hydrogène ou un radical alcoyle, et
- le symbole R₄ représente un radical hydroxy, ou bien
- le symbole R₄ représente un atome de fluor si les symboles R₅ et R'₅ représentent un atome d'hydrogène ou un radical alcoyle, ou bien
- le symbole R₄ forme avec R₅ une liaison et,
- le symbole R₆ représente un atome d'hydrogène ou un radical alcoyle, hydroxy ou hydroxyalcoyle, et
- les symboles R représentent l'un un atome d'hydrogène, un radical alcoyle, un radical hydroxy ou hydroxyalcoyle et l'autre un atome d'hydrogène, un radical alcoyle, phényle ou hydroxyalcoyle,
étant entendu que les radicaux alcoyle et acyle cités ci-dessus sont (sauf mention spéciale) droits ou ramifiés et contiennent 1 à 4 atomes de carbone,
et étant entendu que la chaîne -CHR₁R₂ peut exister sous ses formes (R) et (S) ou leurs mélanges,
ainsi que la forme racémique du dérivé (Ia) et les mélanges avec la forme racémique,
ainsi que ses sels lorsqu'ils existent.

2. Un dérivé de perhydroisoindole selon la revendication 1, caractérisé en ce que le symbole R₁ est un radical hétérocyclyle mono ou polycyclique, saturé ou insaturé choisi parmi thiényle, furyle, pyridyle, dithiinyle, indolyle, isoindolyle, benzothiényle, thiazolyle, isothiazolyle, oxazolyle, imidazolyle, pyrrolyle, triazolyle, thiadiazolyle, quinolyle, isoquinolyle, ou naphtyridinyle.

3. Un dérivé de perhydroisoindole selon la revendication 1, caractérisé en ce que le symbole R₁ représente un radical phényle éventuellement substitué par un radical hydroxy, alcoyloxy, ou dialcoylamino, ou un radical hétérocyclyle mono ou polycyclique, saturé ou insaturé contenant 5 à 9 atomes de carbone et un ou plusieurs hétéroatomes choisis parmi l'oxygène, l'azote ou le soufre, le symbole R₂ représente un atome d'hydrogène ou un radical alcoyle, le symbole R₃ représente un radical phényle éventuellement substitué en position -2 par un radical alcoyloxy contenant 1 ou 2 atomes de carbone ou par un atome de fluor, ou disubstitué par des radicaux trifluorométhyle, les symboles R₅ et R'₅ sont identiques ou différents et représentent l'un un atome d'hydrogène ou un radical hydroxy ou alcoyle et l'autre un atome d'hydrogène ou un radical alcoyle, le symbole R₄ représente un radical hydroxy et, le symbole R₆ représente un atome d'hydrogène ou un radical alcoyle, hydroxy ou hydroxyalcoyle, et les symboles R représentent l'un un atome d'hydrogène, un radical alcoyle, un radical hydroxy ou hydroxyalcoyle et l'autre un atome d'hydrogène, un radical alcoyle ou phényle.

4. Un dérivé de perhydroisoindole selon la revendication 1, caractérisé en ce qu'il s'agit du diméthyl-7,7 (méthoxy-2 phényl)-4 [(méthoxy-2 phényl)-2 propionyl-(S)]-2 perhydroisoindolediol-4,5.

5. Un dérivé de perhydroisoindole selon la revendication 1, caractérisé en ce qu'il s'agit du (méthoxy-2 phényl)-4 [(méthoxy-2 phényl)-2 propionyl-(S)]-2 méthyl-5 perhydroisoindolol-4.

6. Un dérivé de perhydroisoindole selon la revendication 1, caractérisé en ce qu'il s'agit du [(hydroxy-2 phényl)-2 propionyl-(S)]-2 (méthoxy-2 phényl)-4 méthyl-6 perhydroisoindolol-4.

7. Un dérivé de perhydroisoindole selon la revendication 1, caractérisé en ce qu'il s'agit du (hydroxyméthyl)-7 (méthoxy-2 phényl)-4 [(méthoxy-2 phényl)-2 propionyl-(S)]-2 méthyl-7 perhydroisoindolol-4.

8. Un dérivé de perhydroisoindole selon la revendication 1, caractérisé en ce qu'il s'agit du (hydroxyméthyl)-7 (indolyl-3 acétyl)-2 (méthoxy-2 phényl)-4 méthyl-7 perhydroisoindolol-4.

9. Procédé de préparation d'un dérivé de perhydroisoindole selon la revendication 1, caractérisé en ce que l'on fait agir un acide ou un dérivé réactif de l'acide de formule générale : 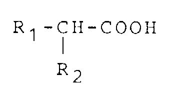 dans laquelle R₁ et R₂ sont définis comme dans la revendication 1, sur un dérivé de l'isoindole de formule générale : 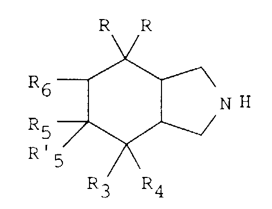 dans laquelle les symboles R, R₃, R_{4,} R₅, R'₅ et R₆ sont définis comme dans la revendication 1, puis transforme éventuellement le produit obtenu pour lequel R₄ est un radical hydroxy et R₅ est un atome d'hydrogène ou un radical alcoyle en un produit pour lequel R₄ est un atome de fluor et R₅ est un atome d'hydrogène ou un radical alcoyle ou pour lequel R₄ et R₅ forment ensemble une liaison, et/ou sépare les formes isomères obtenues, et transforme éventuellement le produit obtenu en un sel lorsqu'ils existent.

10. Procédé de préparation d'un dérivé de perhydroisoindole selon la revendication 1, caractérisé en ce que l'on fait agir un composé organométallique de formule générale :
R₃-M
dans laquelle R₃ est défini comme précédemment, et M représente le lithium, un radical MgX ou CeX₂ pour lequel X est un atome d'halogène, sur un dérivé de perhydroisoindolone de formule générale : 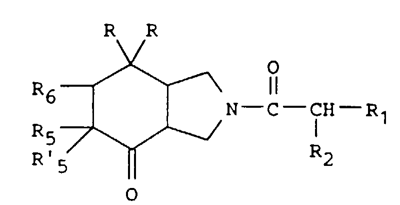 dans laquelle R₁, R₂, R₅, R'₅, R₆ et R sont définis comme dans la revendication 1, et dont de préférence les radicaux hydroxy sont préalablement protégés, puis transforme le cas échéant l'alcool obtenu en un dérivé de perhydroisoindole pour lequel R₄ est un atome de fluor et R₅ est un atome d'hydrogène ou un radical alcoyle ou en un dérivé de perhydroisoindole pour lequel R₄ et R₅ forment ensemble une liaison, ou élimine le cas échéant le radical protecteur de R₅, et sépare éventuellement les isomères, et/ou transforme le produit obtenu en un sel, lorsqu'ils existent.

11. Procédé de préparation d'un dérivé de perhydroisoindole selon la revendication 1, pour lequel l'un des radicaux R est un radical hydroxy, R₄ représente un radical hydroxy et l'un au moins de R₅ et R'₅ est un atome d'hydrogène, caractérisé en ce que l'on opère à partir d'un dérivé de perhydroisoindolone de formule générale : 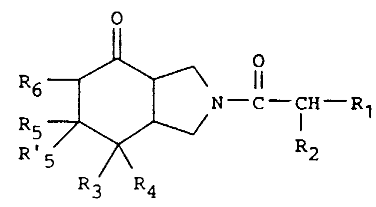 dans laquelle R₁, R₂, R₃ et R₆ sont définis comme dans la revendication 1 et R₄, R₅ et R'₅ sont définis comme ci-dessus, par action d'un composé organométallique de formule générale :
R-M
dans laquelle R est un radical alcoyle, phényle ou hydroxyalcoyle dont la fonction hydroxy est préalablement protégée, et M est défini comme dans la revendication 10, ou par réduction dans l'alternative où l'on veut obtenir un dérivé selon la revendication 1 pour lequel l'autre radical R est un atome d'hydrogène, et/ou le cas échéant séparation des formes isomères obtenues, puis le cas échéant élimination des radicaux protecteurs.

12. Procédé de préparation d'un dérivé de perhydroisoindole selon la revendication 1, pour lequel R₅ (ou R'₅) et R₆ sont simultanément des radicaux hydroxy et les symboles R sont différents de l'atome d'hydrogène, caractérisé en ce que l'on fait agir le tétroxyde d'osmium sur un dérivé de perhydroisoindole de formule générale : 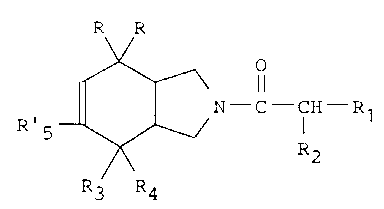 dans laquelle R₁, R₂, R₃, R₄, R et R'₅ sont définis comme dans la revendication 1, et/ou le cas échéant sépare les formes isomères obtenues.

13. Un dérivé de perhydroisoindole caractérisé en ce qu'il répond à la formule générale : 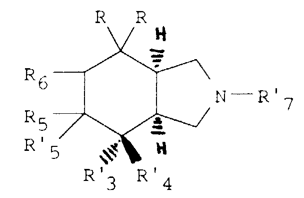 dans laquelle R, R₅, R'₅ et R₆ sont définis comme précédemment dans la revendication 1, R'₃ et R'₄ sont soit définis comme R₃ et R₄ dans la revendication 1, soit forment ensemble un radical oxo et R'₇ est soit un atome d'hydrogène, soit est choisi parmi les groupements protecteurs d'amino : alcoyloxycarbonyle, benzyloxycarbonyle, benzyle éventuellement substitués, formyle, chloracétyle, trichloracétyle, trifluoracétyle, vinyloxycarbonyle, phénoxycarbonyle, chloro-1 éthoxycarbonyle ou chlorocarbonyle, sous forme racémique, sous ses formes stéréoisomères, ou à l'état de mélange de plusieurs de ces formes, ainsi que ses sels lorsqu'ils existent.

14. Composition pharmaceutique caractérisée en ce qu'elle contient au moins un produit selon la revendication 1, à l'état pur ou sous forme d'association avec un ou plusieurs adjuvants ou diluants compatibles et pharmaceutiquement acceptables.

15. Association d'au moins un dérivé de perhydroisoindole selon la revendication 1 avec au moins un antagoniste des récepteurs NK2.

## Patentansprüche

1. Perhydroisoindol-Derivat, dadurch gekennzeichnet, daß es der allgemeinen Formel (Ia) 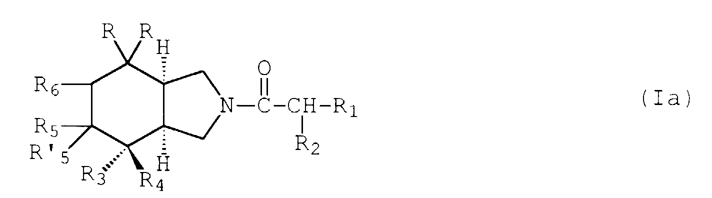 entspricht, in der
- das Symbol R₁ einen Rest Phenyl darstellt, gegebenenfalls substituiert durch ein oder mehrere Halogenatome oder Reste Hydroxy, Alkyl, das gegebenenfalls substituiert sein kann (durch Halogenatome oder Reste Amino, Alkylamino oder Dialkylamino), Alkyloxy oder Alkylthio, die gegebenenfalls substituiert sein können [durch Reste Hydroxy, Amino, Alkylamino oder Dialkylamino, gegebenenfalls substituiert (durch Reste Phenyl, Hydroxy oder Amino), oder Dialkylamino, dessen Alkylteile zusammen mit dem Stickstoffatom, an das sie gebunden sind, einen Heterocyclus mit 5 bis 6 Ringgliedern bilden, der ein anderes Heteroatom enthalten kann, ausgewählt unter Sauerstoff, Schwefel und Stickstoff, gegebenenfalls substituiert durch einen Rest Alkyl, Hydroxy, Hydroxyalkyl], oder substituiert durch Reste Amino, Alkylamino, Dialkylamino, dessen Alkylteile zusammen mit dem Stickstoffatom, an das sie gebunden sind, einen wie oben definierten Heterocyclus bilden können, oder einen Rest Cyclohexadienyl, Naphthyl, Indenyl oder einen gesättigten oder ungesättigten, mono- oder polycyclischen Rest eines Heterocyclus bedeutet, der 5 bis 9 Kohlenstoffatome und ein oder mehrere Heteroatome enthält, ausgewählt unter Sauerstoff, Stickstoff und Schwefel und gegebenenfalls substituiert durch ein Halogenatom oder durch einen Rest Alkyl oder Alkyloxy,
- das Symbol R₂ ein Wasserstoffatom oder ein Halogenatom oder einen Rest Hydroxy, Alkyl, Aminoalkyl, Alkylaminoalkyl, Dialkylaminoalkyl, Alkyloxy, Alkylthio, Acyloxy, Carboxy, Alkyloxycarbonyl, Dialkylaminoalkyloxycarbonyl, Benzyloxycarbonyl, Amino oder Acylamino darstellt,
- das Symbol R₃ einen Rest Phenyl darstellt, gegebenenfalls substituiert in Position -2 durch einen Rest Alkyl oder Alkyloxy mit 1 bis 2 Kohlenstoffatomen oder durch ein Fluoratom, oder disubstituiert durch Reste Trifluormethyl, und
- die Symbole R₅ und R'₅ gleich oder verschieden sind und das eine ein Wasserstoffatom oder einen Rest Hydroxy oder Alkyl und das andere ein Wasserstoffatom oder einen Rest Alkyl bedeuten, und
- das Symbol R₄ ein Rest Hydroxy ist, oder
- das Symbol R₄ ein Fluoratom ist, wenn die Symbole R₅ und R'₅ ein Wasserstoffatom oder einen Rest Alkyl darstellen, oder
- das Symbol R₄ zusammen mit R₅ eine Bindung bildet, und
- das Symbol R₆ ein Wasserstoffatom oder einen Rest Alkyl, Hydroxy oder Hydroxyalkyl bedeutet, und
- bei den Symbolen R das eine ein Wasserstoffatom, einen Rest Alkyl, einen Rest Hydroxy oder Hydroxyalkyl und das andere ein Wasserstoffatom, einen Rest Alkyl, Phenyl oder Hydroxyalkyl darstellen,
mit der Maßgabe, daß die oben genannten Reste Alkyl und Acyl (außer bei spezieller Erwähnung) gerade oder verzweigt sind und 1 bis 4 Kohlenstoffatome enthalten,
und mit der Maßgabe, daß die Kette -CHR₁R₂ in ihren Formen (R) und (S) oder ihren Mischungen existieren kann,
sowie die racemische Form der Derivate (Ia) und die Mischungen mit der racemischen Form,
sowie ihre Salze, wenn sie existieren.

2. Perhydroisoindol-Derivat nach Anspruch 1, dadurch gekennzeichnet, daß das Symbol R₁ ein gesättigter oder ungesättigter, mono- oder polycyclischer Rest eines Heterocyclus ist, ausgewählt unter Thienyl, Furyl, Pyridyl, Dithiinyl, Indolyl, Isoindolyl, Benzothienyl, Thiazolyl, Isothiazolyl, Oxazolyl, Imidazolyl, Pyrrolyl, Triazolyl, Thiadiazolyl, Chinolyl, Isochinolyl oder Naphthyridinyl.

3. Perhydroisoindol-Derivat nach Anspruch 1, dadurch gekennzeichnet, daß das Symbol R₁ einen Rest Phenyl, gegebenenfalls substituiert durch einen Rest Hydroxy, Alkyloxy, oder Dialkylamino, oder einen gesättigten oder ungesättigten, mono- oder polycyclischen Rest eines Heterocyclus mit 5 bis 9 Kohlenstoffatomen und einem oder mehreren Heteroatomen darstellt, ausgewählt unter Sauerstoff, Stickstoff und Schwefel, das Symbol R₂ ein Wasserstoffatom oder einen Rest Alkyl bedeutet, das Symbol R₃ ein Rest Phenyl ist, gegebenenfalls substituiert in Position -2 durch einen Rest Alkyloxy mit 1 oder 2 Kohlenstoffatomen oder durch ein Fluoratom oder disubstituiert durch Reste Trifluormethyl, die Symbole R₅ und R'₅ gleich oder verschieden sind und das eine ein Wasserstoffatom oder einen Rest Hydroxy oder Alkyl und das andere ein Wasserstoffatom oder einen Rest Alkyl darstellen, das Symbol R₄ ein Rest Hydroxy ist und das Symbol R₆ ein Wasserstoffatom oder einen Rest Alkyl, Hydroxy oder Hydroxyalkyl bedeutet, und bei den Symbolen R das eine ein Wasserstoffatom, einen Rest Alkyl, einen Rest Hydroxy oder Hydroxyalkyl und das andere ein Wasserstoffatom, einen Rest Alkyl oder Phenyl darstellen.

4. Perhydroisoindol-Derivat nach Anspruch 1, dadurch gekennzeichnet, daß es sich um 7,7-Dimethyl-4-(2-methoxy-phenyl)-2-[2-(2-methoxy-phenyl)-propionyl-(S)]-4,5-perhydroisoindoldiol handelt.

5. Perhydroisoindol-Derivat nach Anspruch 1, dadurch gekennzeichnet, daß es sich um 4-(2-Methoxy-phenyl)-2-[2-(2-methoxy-phenyl)-propionyl-(S)]-5-methyl-4-perhydroisoindolol handelt.

6. Perhydroisoindol-Derivat nach Anspruch 1, dadurch gekennzeichnet, daß es sich um 4-(2-Methoxy-phenyl)-2-[2-(2-hydroxy-phenyl)-propionyl-(S)]-6-methyl-4-perhydroisoindolol handelt.

7. Perhydroisoindol-Derivat nach Anspruch 1, dadurch gekennzeichnet, daß es sich um 7-(Hydroxymethyl)-4-(2-methoxy-phenyl)-2-[2-(2-methoxy-phenyl)-propionyl-(S)]-7-methyl-4-perhydroisoindolol handelt.

8. Perhydroisoindol-Derivat nach Anspruch 1, dadurch gekennzeichnet, daß es sich um 7-(Hydroxymethyl)-4-(2-methoxy-phenyl)-2-(3-indolyl-acetyl)-7-methyl-4-perhydroisoindolol handelt.

9. Verfahren zur Herstellung eines Perhydroisoindol-Derivates nach Anspruch 1, dadurch gekennzeichnet, daß man eine Säure oder ein reaktives Derivat der Säure der allgemeinen Formel 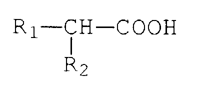 in der R₁ und R₂ wie in Anspruch 1 definiert sind, mit einem Isoindol-Derivat der allgemeinen Formel 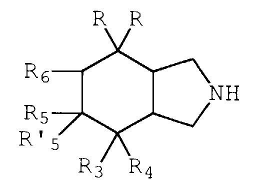 in der die Symbole R, R₃, R₄, R₅, R'₅ und R₆ wie in Anspruch 1 definiert sind, zur Reaktion bringt, anschließend gegebenenfalls das erhaltene Produkt, worin R₄ einen Rest Hydroxy darstellt und R₅ ein Wasserstoffatom oder ein Rest Alkyl ist, in ein Produkt umwandelt, worin R₄ ein Fluoratom darstellt und R₅ ein Wasserstoffatom oder ein Rest Alkyl ist, oder worin R₄ und R₅ zusammen eine Bindung bilden, und/oder die erhaltenen isomeren Formen trennt und gegebenenfalls das erhaltenen Produkt in ein Salz umwandelt, wenn diese existieren.

10. Verfahren zur Herstellung eines Perhydroisoindol-Derivates nach Anspruch 1, dadurch gekennzeichnet, daß man eine organometallische Verbindung der allgemeinen Formel
R₃-M
in der R₃ wie oben definiert ist und M Lithium, einen Rest MgX oder CeX₂ darstellt, worin X ein Halogenatom ist, mit einem Perhydroisoindolon-Derivat der allgemeinen Formel 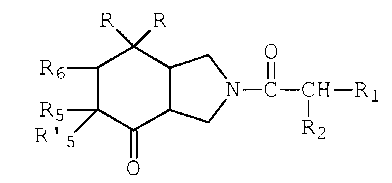 in der R₁, R₂, R₅, R'₅, R₆ und R wie in Anspruch 1 definiert sind und worin vorzugsweise die Reste Hydroxy zuvor geschützt wurden, zur Reaktion bringt, anschließend gegebenenfalls den erhaltenen Alkohol in ein Perhydroisoindol-Derivat, worin R₄ ein Fluoratom ist und R₅ ein Wasserstoffatom oder einen Rest Alkyl darstellt, oder in ein Perhydroisoindol-Derivat umwandelt, worin R₄ und R₅ zusammen eine Bindung bilden, oder gegebenenfalls die Schutzgruppe von R₅ entfernt und gegebenenfalls die Isomeren trennt und/oder das erhaltene Produkt in ein Salz umwandelt, wenn diese existieren.

11. Verfahren zur Herstellung eines Perhydroisoindol-Derivates nach Anspruch 1, worin einer der Reste R ein Rest Hydroxy ist, R₄ ein Rest Hydroxy ist und mindestens eines von R₅ und R'₅ ein Wasserstoffatom darstellt, dadurch gekennzeichnet, daß man von einem Perhydroisoindolon-Derivat der allgemeinen Formel 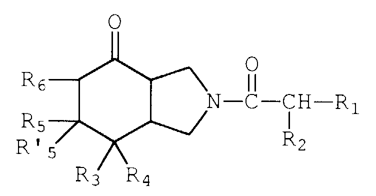 ausgeht, in der R₁, R₂, R₃ und R₆ wie in Anspruch 1 und R₄, R₅ und R'₅ wie oben definiert sind, und dieses der Einwirkung einer organometallischen Verbindung der allgemeinen Formel
R-M
unterzieht, worin R einen Rest Alkyl, Phenyl oder Hydroxyalkyl bedeutet, dessen Hydroxyfunktion zuvor geschützt wurde, und M wie in Anspruch 10 definiert ist, oder alternativ, wenn man ein Derivat gemäß Anspruch 1 erhalten will, worin der andere Rest R ein Wasserstoffatom ist, die Reduktion durchführt, und/oder gegebenenfalls die Trennung der erhaltenen isomeren Formen vornimmt sowie anschließend gegebenenfalls die Schutzgruppen entfernt.

12. Verfahren zur Herstellung eines Perhydroisoindol-Derivates nach Anspruch 1, worin R₅ (oder R'₅) und R₆ gleichzeitig Reste Hydroxy darstellen und die Symbole R von einem Wasserstoffatom verschieden sind, dadurch gekennzeichnet, daß man Osmiumtetroxid mit einem Perhydroisoindol-Derivat der allgemeinen Formel 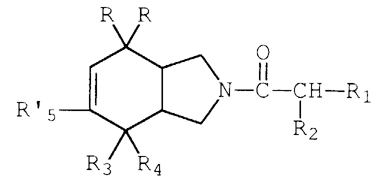 in der R₁, R₂, R₃, R₄, R und R'₅ wie in Anspruch 1 definiert sind, zur Reaktion bringt und/oder gegebenenfalls die erhaltenen isomeren Formen trennt.

13. Perhydroisoindol-Derivat, dadurch gekennzeichnet, daß es der allgemeinen Formel 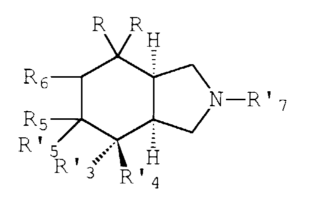 entspricht, in der R, R₅, R'₅ und R₆ wie in Anspruch 1 definiert sind, R'₃ und R'₄ entweder wie R₃ und R₄ in Anspruch 1 definiert sind oder zusammen einen Rest Oxo bilden und R'₇ entweder ein Wasserstoffatom ist oder unter den Schutzgruppen für Amino ausgewählt wird: Alkyloxycarbonyl, Benzyloxycarbonyl, Benzyl, gegebenenfalls substituiert, Formyl, Chloracetyl, Trichloracetyl, Trifluoracetyl, Vinyloxycarbonyl, Phenoxycarbonyl, 1-Chlor-ethoxycarbonyl oder Chlorcarbonyl,
in racemischer Form, in Form seiner Stereoisomeren oder im Zustand einer Mischung von mehreren dieser Formen, sowie seiner Salze, wenn diese existieren.

14. Pharmazeutische Zusammensetzung, dadurch gekennzeichnet, daß sie mindestens ein Produkt gemäß Anspruch 1 in reinem Zustand oder in Form einer Assoziation mit einem oder mehreren kompatiblen und pharmazeutisch akzeptablen Zusatzstoffen oder Verdünnungsmitteln enthält.

15. Assoziation von mindestens einem Perhydroisoindol-Derivat gemäß Anspruch 1 mit mindestens einem Antagonisten der Rezeptoren NK2.

## Claims

1. A perhydroisoindole derivative, characterized in that it corresponds to the general formula: 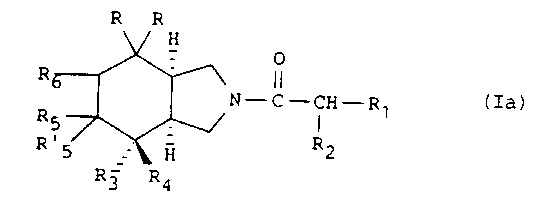 in which
- the symbol R₁ represents a phenyl radical which is optionally substituted with one or more halogen atoms or hydroxyl radicals, alkyl radicals which may be optionally substituted (with halogen atoms or amino, alkylamino or dialkylamino radicals), alkyloxy or alkylthio radicals which may be optionally substituted [with hydroxyl, amino, alkylamino or dialkylamino radicals which are optionally substituted (with phenyl, hydroxyl or amino radicals), or dialkylamino radicals in which the alkyl parts form, with the nitrogen atom to which they are attached, a 5- to 6-membered heterocycle which may contain another hetero atom chosen from oxygen, sulphur or nitrogen, optionally substituted with an alkyl, hydroxyl or hydroxyalkyl radical)], or substituted with amino, alkylamino or dialkylamino radicals in which the alkyl parts may form, with the nitrogen atom to which they are attached, a heterocycle as defined above, or represents a cyclohexadienyl, naphthyl or indenyl radical or a saturated or unsaturated mono- or polycyclic heterocyclic radical containing 5 to 9 carbon atoms and one or more hetero atoms chosen from oxygen, nitrogen or sulphur, and optionally substituted with a halogen atom or with an alkyl or alkyloxy radical,
- the symbol R₂ represents a hydrogen or halogen atom or a hydroxyl, alkyl, aminoalkyl, alkylaminoalkyl, dialkylaminoalkyl, alkyloxy, alkylthio, acyloxy, carboxyl, alkyloxycarbonyl, dialkylaminoalkyloxycarbonyl, benzyloxycarbonyl, amino or acylamino radical,
- the symbol R₃ represents a phenyl radical which is optionally substituted in the 2-position with an alkyl or alkyloxy radical containing 1 or 2 carbon atoms or with a fluorine atom, or disubstituted with trifluoromethyl radicals, and
- the symbols R₅ and R'₅ are identical or different and one represents a hydrogen atom or a hydroxyl or alkyl radical and the other represents a hydrogen atom or an alkyl radical, and
- the symbol R₄ represents a hydroxyl radical, or alternatively
- the symbol R₄ represents a fluorine atom if the symbols R₅ and R'₅ represent a hydrogen atom or an alkyl radical, or alternatively
- the symbol R₄ forms with R₅ a bond, and
- the symbol R₆ represents a hydrogen atom or an alkyl, hydroxyl or hydroxyalkyl radical, and
- one of the symbols R represents a hydrogen atom, an alkyl radical, a hydroxyl or hydroxyalkyl radical and the other represents a hydrogen atom or an alkyl, phenyl or hydroxyalkyl radical,
it being understood that the abovementioned alkyl and acyl radicals are (except where especially mentioned) straight or branched and contain 1 to 4 carbon atoms,
and it being understood that the chain -CHR₁R₂ can exist in its (R) and (S) forms or their mixtures, as well as the racemic form of the derivative (Ia) and mixtures with the racemic form, as well as its salts when they exist.

2. A perhydroisoindole derivative according to claim 1, characterized in that the symbol R₁ is a saturated or unsaturated mono- or polycyclic heterocyclic radical chosen from thienyl, furyl, pyridyl, dithiinyl, indolyl, isoindolyl, benzothienyl, thiazolyl, isothiazolyl, oxazolyl, imidazolyl, pyrrolyl, triazolyl, thiadiazolyl, quinolyl, isoquinolyl or naphthyridinyl.

3. A perhydroisoindole derivative according to claim 1, characterized in that the symbol R₁ represents a phenyl radical which is optionally substituted with a hydroxyl, alkyloxy or dialkylamino radical, or a saturated or unsaturated mono- or polycyclic heterocyclic radical containing 5 to 9 carbon atoms and one or more hetero atoms chosen from oxygen, nitrogen or sulphur, the symbol R₂ represents a hydrogen atom or an alkyl radical, the symbol R₃ represents a phenyl radical which is optionally substituted in the 2-position with an alkyloxy radical containing 1 or 2 carbon atoms or with a fluorine atom, or is disubstituted with trifluoromethyl radicals, the symbols R₅ and R'₅ are identical or different and one represents a hydrogen atom or a hydroxyl or alkyl radical and the other represents a hydrogen atom or an alkyl radical, the symbol R₄ represents a hydroxyl radical and the symbol R₆ represents a hydrogen atom or an alkyl, hydroxyl or hydroxyalkyl radical, and one of the symbols R represents a hydrogen atom, an alkyl radical, a hydroxyl or hydroxyalkyl radical and the other represents a hydrogen atom or an alkyl or phenyl radical.

4. A perhydroisoindole derivative according to claim 1, characterized in that it is 7,7-dimethyl-4-(2-methoxyphenyl)-2-[2-(S)-(2-methoxyphenyl)propionyl]-4,5-perhydroisoindolediol.

5. A perhydroisoindole derivative according to claim 1, characterized in that it is 4-(2-methoxyphenyl)-2-[2-(S)-(2-methoxyphenyl)propionyl]-5-methyl-4-perhydroisoindolol.

6. A perhydroisoindole derivative according to claim 1, characterized in that it is 2-[2-(S)-(2-hydroxyphenyl)propionyl]-4-(2-methoxyphenyl)-6-methyl indolol.

7. A perhydroisoindole derivative according to claim 1, characterized in that it is 7-(hydroxymethyl)-4-(2-methoxyphenyl)-2-[2-(S)-(2-methoxyphenyl)propionyl]-7-methyl-4-perhydroisoindolol.

8. A perhydroisoindole derivative according to claim 1, characterized in that it is 7-(hydroxymethyl)-2-(3-indolylacetyl)-4-(2-methoxyphenyl)-7-methyl-4-perhydroisoindolol.

9. Process for the preparation of a perhydroisoindole derivative according to claim 1, characterized in that an acid or a reactive derivative of the acid of general formula: 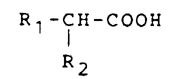 in which R₁ and R₂ are defined as in claim 1, is reacted with an isoindole derivative of general formula: 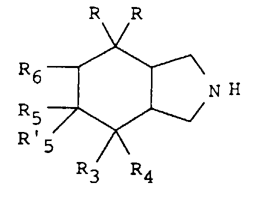 in which the symbols R, R₃, R₄, R₅, R'₅ and R₆ are defined as in claim 1, optionally followed by conversion of the product obtained for which R₄ is a hydroxyl radical and R₅ is a hydrogen atom or an alkyl radical to a product for which R₄ is a fluorine atom and R₅ is a hydrogen atom or an alkyl radical or for which R₄ and R₅ together form a bond, and/or separation of the isomeric forms obtained, and optional conversion of the product obtained to a salt when they exist.

10. Process for the preparation of a perhydroisoindole derivative according to claim 1, characterized in that an organometallic compound of general formula:
R₃-M
in which R₃ is defined as above, and M represents lithium, a radical MgX or CeX₂ for which X is a halogen atom, is reacted with a perhydroisoindolone derivative of general formula: 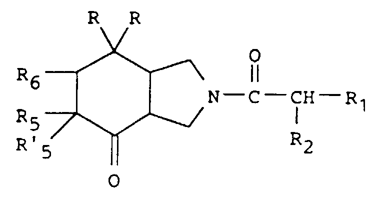 in which R₁, R₂, R₅, R'₅, R₆ and R are defined as in claim 1, and for which the hydroxyl radicals are preferably protected beforehand, followed by conversion, where appropriate, of the alcohol obtained to a perhydroisoindole derivative for which R₄ is a fluorine atom and R₅ is a hydrogen atom or an alkyl radical or to a perhydroisoindole derivative for which R₄ and R₅ together form a bond, or removal, where appropriate, of the radical protecting R₅, and optional separation of the isomers and/or conversion of the product obtained to a salt, when they exist.

11. Process for the preparation of a perhydroisoindole derivative according to claim 1 for which one of the radicals R is a hydroxyl radical, R₄ represents a hydroxyl radical and at least one of R₅ or R'₅ is a hydrogen atom, characterized in that the procedure is performed from a perhydroisoindolone derivative of general formula: 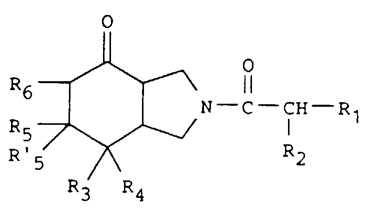 in which R₁, R₂, R₃ and R₆ are defined as in claim 1 and R₄, R₅ and R'₅ are defined as above, by the action of an organometallic compound of general formula:
R-M
in which R is an alkyl or phenyl radical or a hydroxyalkyl radical in which the hydroxyl function is protected beforehand, and M is defined as in claim 10, or by reduction in the alternative in which it is desired to obtain a derivative according to claim 1 for which the other radical R is a hydrogen atom, and/or, where appropriate, separation of the isomeric forms obtained, followed, where appropriate, by removal of the protecting radicals.

12. Process for the preparation of a perhydroisoindole derivative according to claim 1 for which R₅ (or R'₅) and R₆ are simultaneously hydroxyl radicals and the symbols R are other than a hydrogen atom, characterized in that osmium tetroxide is reacted with a perhydroisoindole derivative of general formula: 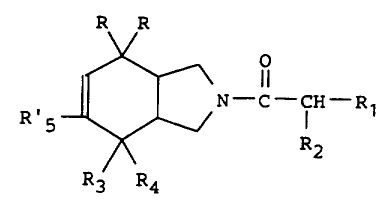 in which R₁, R₂, R₃, R₄, R and R'₅ are defined as in claim 1, and/or, where appropriate, the isomeric forms obtained are separated.

13. A perhydroisoindole derivative, characterized in that it corresponds to the general formula: 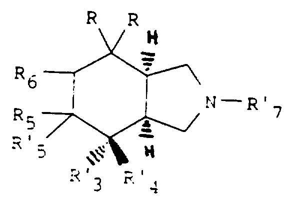 in which R, R₅, R'₅ and R₆ are defined as above in claim 1, R'₃ and R'₄ are either defined as R₃ and R₄ in claim 1 or together form an oxo radical and R'₇ is either a hydrogen atom or is chosen from the amino protecting groups: alkyloxycarbonyl, benzyloxycarbonyl, benzyl which is optionally substituted, formyl, chloroacetyl, trichloroacetyl, trifluoroacetyl, vinyloxycarbonyl, phenoxycarbonyl, 1-chloroethoxycarbonyl or chlorocarbonyl, in racemic form, in its stereoisomeric forms or in the state of a mixture of several of these forms, as well as its salts when they exist.

14. Pharmaceutical composition, characterized in that it contains at least one product according to claim 1, in the pure state or in the form of a combination with one or more pharmaceutically acceptable and compatible diluents or adjuvants.

15. Combination of at least one perhydroisoindole derivative according to claim 1 with at least one NK2-receptor antagonist.
